# EUROPEAN PATENT APPLICATION

(11) **EP 4 339 195 A1**
(43) Date of publication of application: **20.03.2024**
(21) Application number: 22904460.7
(22) Date of filing: 28.10.2022
(51) Int. Cl.: C07D 409/14, C07D 403/04, C07D 405/14, C07D 403/10, H10K 99/00, H10K 50/00, C09K 11/06

(54) **HETEROCYCLIC COMPOUND, ORGANIC LIGHT-EMITTING ELEMENT COMPRISING SAME, AND COMPOSITION FOR ORGANIC LAYER OF ORGANIC LIGHT-EMITTING ELEMENT**

(30) Priority: 10.12.2021 KR 20210176360
(71) Applicant: LT Materials Co., Ltd., Yongin-si, Gyeonggi-do 17118 (KR)
(72) Inventor: PARK, Seong-Jong, Yongin-si, Gyeonggi-do 17118 (KR); JANG, Hyung-Keun, Yongin-si, Gyeonggi-do 17118 (KR); NO, Young-Seok, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2022/016716
(87) International publication number: WO 2023/106626

(57) **Abstract**

The present specification provides a heterocyclic compound, an organic light emitting device including the same and a composition for an organic material layer of the organic light emitting device.

## Description

### [Technical Field]

This application claims priority to and the benefit of Korean Patent Application No. 10-2021-0176360 filed in the Korean Intellectual Property Office on December 10, 2021, the entire contents of which are incorporated herein by reference.

The present invention relates to a heterocyclic compound, an organic light emitting device including the same and a composition for an organic material layer of the organic light emitting device.

### [Background Art]

An electroluminescence device is a kind of self-emitting type display device, and has an advantage in that the viewing angle is wide, the contrast is excellent, and the response speed is fast.

An organic light emitting device is composed of a structure in which an organic thin film is disposed between two electrodes. When a voltage is applied to an organic light emitting device having such a structure, electrons and holes injected from the two electrodes are combined with each other in the organic thin film to make a pair, and then, the paired electrons and holes emit light while being annihilated. The organic thin film may be composed of a single layer or multiple layers, if necessary.

A material for the organic thin film may have a light emitting function, if necessary. For example, as the material for the organic thin film, it is also possible to use a compound, which may itself constitute a light emitting layer alone, or it is also possible to use a compound, which may serve as a host or a dopant of a host-dopant-based light emitting layer. In addition, as a material for the organic thin film, it is also possible to use a compound, which may perform a function such as hole injection, hole transport, electron blocking, hole blocking, electron transport or electron injection.

In order to improve the performance, efficiency and service life of the organic light emitting device, there is a continuous need for developing a material for an organic thin film.

### [Related Art Documents]

### [Patent Documents]

(Patent Document 1) US Patent No. 4,356,429

### [Disclosure]

### [Technical Problem]

The present invention has been made in an effort to provide a heterocyclic compound, an organic light emitting device including the same and a composition for an organic material layer of the organic light emitting device.

### [Technical Solution]

In an exemplary embodiment of the present application, provided is a heterocyclic compound represented by the following Chemical Formula 1.

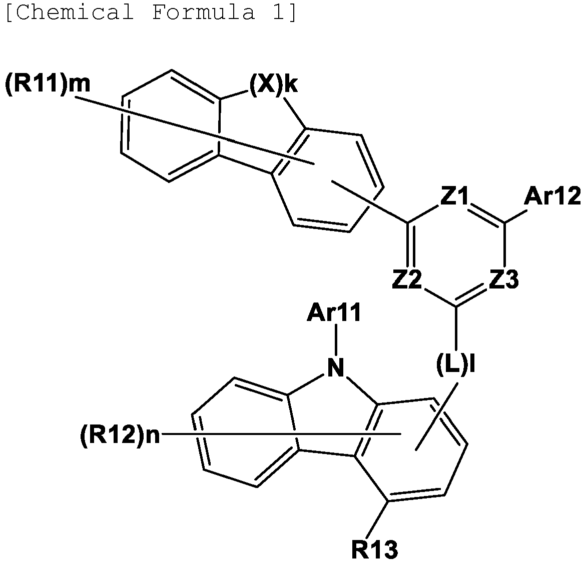

In Chemical Formula 1,
Z1 to Z3 are the same as or different from each other, and are each independently N; or CR, and at least one is N, R is hydrogen; deuterium; a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group,
L is a direct bond; or a substituted or unsubstituted C6 to C60 arylene group,
1 is an integer from 0 to 3, and when 1 is 2 or higher, L's are the same as or different from each other,
X is O; or S,
R11 to R13 are the same as or different from each other, and are each independently hydrogen; deuterium; a halogen group; a cyano group; a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group,
k is 0 or 1,
when k is 0, it means that there is no bond rather than a direct bond,
when k is 0, m is an integer from 0 to 9, and when k is 1, m is an integer from 0 to 7, and when m is 2 or higher, R11's are the same as or different from each other,
n is an integer from 0 to 6, and when n is 2 or higher, R12's are the same as or different from each other,
Ar11 and Ar12 are the same as or different from each other, and are each independently a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group,
the deuterium content of 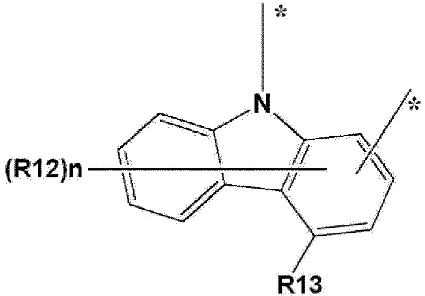 in the structure of Chemical Formula 1 is more than 0% and 100% or less, and
the 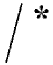 is a position bonded to Chemical Formula 1. Further, in an exemplary embodiment of the present application, provided is an organic light emitting device including: a first electrode; a second electrode provided to face the first electrode; and an organic material layer having one or more layers provided between the first electrode and the second electrode, in which one or more layers of the organic material layer include the heterocyclic compound represented by Chemical Formula 1.

Further, in an exemplary embodiment of the present application, provided is an organic light emitting device in which the organic material layer further includes a heterocyclic compound represented by the following Chemical Formula 2.

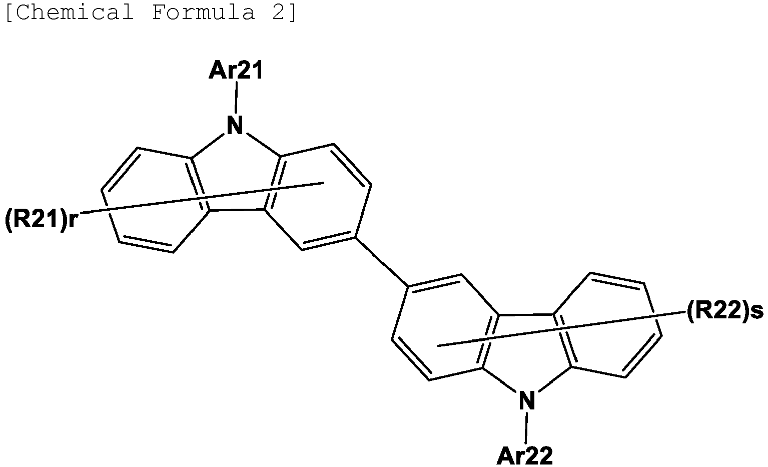

In Chemical Formula 2,
R21 and R22 are the same as or different from each other, and are each independently selected from the group consisting of hydrogen; deuterium; a halogen group; a cayno group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiR'R"R"'; -P(=O)R'R"; and an amine group which is unsubstituted or substituted with a substituted or unsubstituted C1 to C60 alkyl group, a substituted or unsubstituted C6 to C60 aryl group or a substituted or unsubstituted C2 to C60 heteroaryl group, or two or more adjacent groups are bonded to each other to form a substituted or unsubstituted C6 to C60 aliphatic or aromatic hydrocarbon ring or a substituted or unsubstituted C2 to C60 aliphatic or aromatic hetero ring,
R', R" and R"' are the same as or different from each other, and are each independently hydrogen; deuterium; a cyano group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
r and s are an integer from 0 to 7, and when r and s are 2 or higher, substituents in the parenthesis are the same as or different from each other, and
Ar21 and Ar22 are the same as or different from each other, and are each independently a substituted or unsubstituted C6 to C60 aryl group; or a substituted or unsubstituted C2 to C60 heteroaryl group.

Finally, in an exemplary embodiment of the present application, provided is a composition for an organic material layer of an organic light emitting device, which includes the heterocyclic compound represented by Chemical Formula 1; and the heterocyclic compound represented by Chemical Formula 2.

### [Advantageous Effects]

The heterocyclic compound described in the present specification may be used as a material for the organic material layer of the organic light emitting device. That is, the heterocyclic compound can serve as a light emitting material, a hole injection material, a hole transport material, an electron transport material, an electron injection material and the like in the organic light emitting device. In particular, the heterocyclic compound can be used as a material for a light emitting layer of an organic light emitting device.

Specifically, one or two or more of the heterocyclic compounds represented by Chemical Formula 1 can be used, and can be used as materials for the light emitting layer. In particular, the heterocyclic compound can be used as an n-host material for the light emitting layer of an organic light-emitting device by introducing various substituents to adjust the bandgap. In particular, when the heterocyclic compound is substituted with deuterium, the driving voltage of the organic light emitting device can be lowered, the light efficiency can be improved, and the service life characteristics of the device can be improved.

In addition, the heterocyclic compound represented by Chemical Formula 1 can be applied to the organic material layer of the organic light emitting device as a combination of two types by further including the heterocyclic compound represented by Chemical Formula 2 as a p-host material of the light emitting layer.

### [Description of Drawings]

FIGS. 1 to 3 each schematically illustrate a stacking structure of an organic light emitting device according to an exemplary embodiment of the present application.
FIG. 4 shows the results of measuring the recombination zone of a reference compound Q, FIG. 5 shows the results of measuring the recombination zone of a reference compound U, FIG. 6 shows the results of measuring the recombination zone of a reference compound W, and FIG. 7 shows the results of measuring the recombination zone of Compound 101.
FIG. 8 shows the results of measuring the hole mobility of Compound 101 and reference compounds W, Q and U, FIG. 9 shows the results of measuring the electron mobility of Compound 101 and reference compounds W, Q and U, and FIG. 10 shows the results of measuring the hole mobility of Compound 101 and reference compounds S, Q and R.
FIG. 11 illustrates the HOMO distribution map of Compound 101, and FIG. 12 illustrates the LUMO distribution map of Compound 101.

### [Mode for Invention]

Hereinafter, the present specification will be described in more detail.

When one part "includes" one constituent element in the present specification, unless otherwise specifically described, this does not mean that another constituent element is excluded, but means that another constituent element may be further included.

In the present specification, 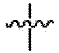 of a chemical formula means a position to which a constituent element is bonded.

The term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more are substituted, the two or more substituents may be the same as or different from each other.

In the present specification, "substituted or unsubstituted" means being unsubstituted or substituted with one or more substituents selected from the group consisting of deuterium; a halogen group; -CN; a C1 to C60 alkyl group; a C2 to C60 alkenyl group; a C2 to C60 alkynyl group; a C1 to C60 haloalkyl group; a C1 to C60 alkoxy group; a C6 to C60 aryloxy group; a C1 to C60 alkylthio group; a C6 to C60 arylthio group; a C1 to C60 alkylsulfoxy group; a C6 to C60 arylsulfoxy group; a C3 to C60 cycloalkyl group; a C2 to C60 heterocycloalkyl group; a C6 to C60 aryl group; a C2 to C60 heteroaryl group; -SiRR'R"; -P(=O)RR'; and -NRR', or a substituent to which two or more substituents selected among the exemplified substituents are linked, and R, R' and R" are each independently a substituent composed of at least one of hydrogen; deuterium; a halogen group; an alkyl group; an alkenyl group; an alkoxy group; a cycloalkyl group; a heterocycloalkyl group; an aryl group; and a heteroaryl group.

In the present specification, "when a substituent is not indicated in the structure of a chemical formula or compound" means that a hydrogen atom is bonded to a carbon atom. However, since deuterium (²H) is an isotope of hydrogen, some hydrogen atoms may be deuterium.

In an exemplary embodiment of the present application, "when a substituent is not indicated in the structure of a chemical formula or compound" may mean that all the positions that may be reached by the substituent are hydrogen or deuterium. That is, deuterium is an isotope of hydrogen, and some hydrogen atoms may be deuterium which is an isotope, and in this case, the content of deuterium may be 0% to 100%.

In an exemplary embodiment of the present application, in "the case where a substituent is not indicated in the structure of a chemical formula or compound", when the content of deuterium is 0%, the content of hydrogen is 100%, and all the substituents do not explicitly exclude deuterium, hydrogen and deuterium may be mixed and used in the compound.

In an exemplary embodiment of the present application, deuterium is one of the isotopes of hydrogen, is an element that has a deuteron composed of one proton and one neutron as a nucleus, and may be represented by hydrogen-2, and the element symbol may also be expressed as D or ²H.

In an exemplary embodiment of the present application, the isotope means an atom with the same atomic number (Z), but different mass numbers (A), and the isotope may be interpreted as an element which has the same number of protons, but different number of neutrons.

In an exemplary embodiment of the present application, when the total number of substituents of a basic compound is defined as T1 and the number of specific substituents among the substituents is defined as T2, the content T% of the specific substituent may be defined as T2/T1×100 = T%.

That is, in an example, the deuterium content of 20% in a phenyl group represented by 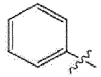 may be represented by 20% when the total number of substituents that the phenyl group can have is 5 (T1 in the formula) and the number of deuteriums among the substituents is 1 (T2 in the formula). That is, a deuterium content of 20% in the phenyl group may be represented by the following structural formula.

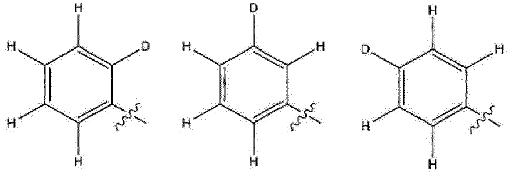

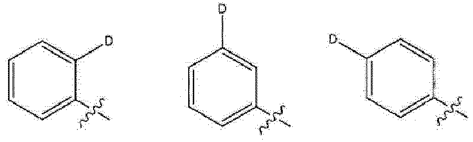

Further, in an exemplary embodiment of the present application, "a phenyl group having a deuterium content of 0%" may mean a phenyl group that does not include a deuterium atom, that is, has five hydrogen atoms.

In the present specification, the halogen may be fluorine, chlorine, bromine or iodine.

In the present specification, an alkyl group includes a straight-chain or branched-chain having 1 to 60 carbon atoms, and may be additionally substituted with another substituent. The number of carbon atoms of the alkyl group may be 1 to 60, specifically 1 to 40, and more specifically 1 to 20. Specific examples thereof include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group, and the like, but are not limited thereto.

In the present specification, an alkenyl group includes a straight-chain or branched-chain having 2 to 60 carbon atoms, and may be additionally substituted with another substituent. The number of carbon atoms of the alkenyl group may be 2 to 60, specifically 2 to 40, and more specifically 2 to 20. Specific examples thereof include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group, and the like, but are not limited thereto.

In the present specification, an alkynyl group includes a straight-chain or branched-chain having 2 to 60 carbon atoms, and may be additionally substituted with another substituent. The number of carbon atoms of the alkynyl group may be 2 to 60, specifically 2 to 40, and more specifically 2 to 20.

In the present specification, a haloalkyl group means an alkyl group substituted with a halogen group, and specific examples thereof include -CF₃, -CF₂CF₃, and the like, but are not limited thereto.

In the present specification, an alkoxy group is represented by -O(R101), and the above-described examples of the alkyl group may be applied to R101.

In the present specification, an aryloxy group is represented by -O(R102), and the above-described examples of the aryl group may be applied to R102.

In the present specification, an alkylthio group is represented by -S(R103), and the above-described examples of the alkyl group may be applied to R103.

In the present specification, an arylthio group is represented by -S(R104), and the above-described examples of the aryl group may be applied to R104.

In the present specification, an alkylsulfoxy group is represented by -S(=0)₂(R105), and the above-described examples of the alkyl group may be applied to R105.

In the present specification, an arylsulfoxy group is represented by -S(=0)₂(R106), and the above-described examples of the aryl group may be applied to R106.

In the present specification, a cycloalkyl group includes a monocycle or polycycle having 3 to 60 carbon atoms, and may be additionally substituted with another substituent. Here, the polycycle means a group in which a cycloalkyl group is directly linked to or fused with another cyclic group. Here, another cyclic group may also be a cycloalkyl group, but may also be another kind of cyclic group, for example, a heterocycloalkyl group, an aryl group, a heteroaryl group, and the like. The number of carbon atoms of the cycloalkyl group may be 3 to 60, specifically 3 to 40, and more specifically 5 to 20. Specific examples thereof include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group, and the like, but are not limited thereto.

In the present specification, a heterocycloalkyl group includes O, S, Se, N, or Si as a heteroatom, includes a monocycle or polycycle having 2 to 60 carbon atoms, and may be additionally substituted with another substituent. Here, the polycycle means a group in which a heterocycloalkyl group is directly linked to or fused with another cyclic group. Here, another cyclic group may also be a heterocycloalkyl group, but may also be another kind of cyclic group, for example, a cycloalkyl group, an aryl group, a heteroaryl group, and the like. The number of carbon atoms of the heterocycloalkyl group may be 2 to 60, specifically 2 to 40, and more specifically 3 to 20.

In the present specification, an aryl group includes a monocycle or polycycle having 6 to 60 carbon atoms, and may be additionally substituted with another substituent. Here, the polycycle means a group in which an aryl group is directly linked to or fused with another cyclic group. Here, another cyclic group may also be an aryl group, but may also be another kind of cyclic group, for example, a cycloalkyl group, a heterocycloalkyl group, a heteroaryl group, and the like. The aryl group includes a spiro group. The number of carbon atoms of the aryl group may be 6 to 60, specifically 6 to 40, and more specifically 6 to 25. Specific examples of the aryl group include a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused cyclic group thereof, and the like, but are not limited thereto.

In the present specification, the terphenyl group may be selected from the following structures.

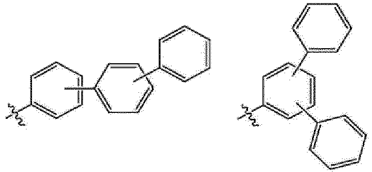

In the present specification, the fluorenyl group may be substituted, and adjacent substituents may be bonded to each other to form a ring.

When the fluorenyl group is substituted, the substituent may be 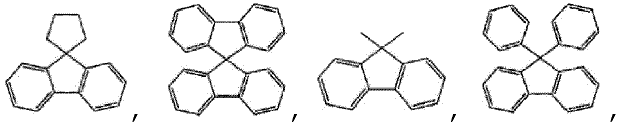 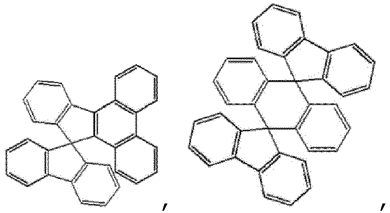 and the like, but is not limited thereto.

In the present specification, a heteroaryl group includes S, O, Se, N, or Si as a heteroatom, includes a monocycle or polycycle having 2 to 60 carbon atoms, and may be additionally substituted with another substituent. Here, the polycycle means a group in which a heteroaryl group is directly linked to or fused with another cyclic group. Here, another cyclic group may also be a heteroaryl group, but may also be another kind of cyclic group, for example, a cycloalkyl group, a heterocycloalkyl group, an aryl group, and the like. The number of carbon atoms of the heteroaryl group may be 2 to 60, specifically 2 to 40, and more specifically 3 to 25. Specific examples of the heteroaryl group include a pyridine group, a pyrrole group, a pyrimidine group, a pyridazine group, a furan group, a thiophene group, an imidazole group, a pyrazole group, an oxazole group, an isoxazole group, a thiazole group, an isothiazole group, a triazole group, a furazan group, an oxadiazole group, a thiadiazole group, a dithiazole group, a tetrazolyl group, a pyran group, a thiopyran group, a diazine group, an oxazine group, a thiazine group, a dioxin group, a triazine group, a tetrazine group, a quinoline group, an isoquinoline group, a quinazoline group, an isoquinazoline group, a quinozoline group, a naphthyridine group, an acridine group, a phenanthridine group, an imidazopyridine group, a diazanaphthalene group, a triazaindene group, an indole group, an indolizine group, a benzothiazole group, a benzoxazole group, a benzimidazole group, a benzothiophene group, a benzofuran group, a dibenzothiophene group, a dibenzofuran group, a carbazole group, a benzocarbazole group, a dibenzocarbazole group, a phenazine group, a dibenzosilole group, spirobi(dibenzosilole), a dihydrophenazine group, a phenoxazine group, a phenanthridine group, a thienyl group, an indolo[2,3-a]carbazole group, an indolo[2,3-b]carbazole group, an indoline group, a 10,11-dihydro-dibenzo[b,f]azepine group, a 9,10-dihydroacridine group, a phenanthrazine group, a phenothiathiazine group, a phthalazine group, a phenanthroline group, a naphthobenzofuran group, a naphthobenzothiophene group, a benzo[c][1,2,5]thiadiazole group, a 2,3-dihydrobenzo[b]thiophene group, a 2,3-dihydrobenzofuran group, a 5,10-dihydrodibenzo[b,e][1,4]azasiline group, a pyrazolo[1,5-c]quinazoline group, a pyrido[1,2-b]indazole group, a pyrido[1,2-a]imidazo[1,2-e]indoline group, a 5,11-dihydroindeno[1,2-b]carbazole group, and the like, but are not limited thereto.

In the present specification, when the substituent is a carbazole group, it means being bonded to nitrogen or carbon of carbazole.

In the present specification, when a carbazole group is substituted, an additional substituent may be substituted with the nitrogen or carbon of the carbazole.

In the present specification, a benzocarbazole group may be any one of the following structures.

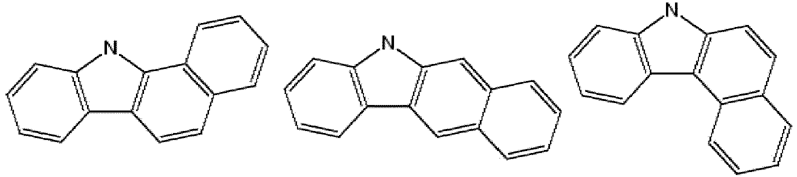

In the present specification, a dibenzocarbazole group may be any one of the following structures.

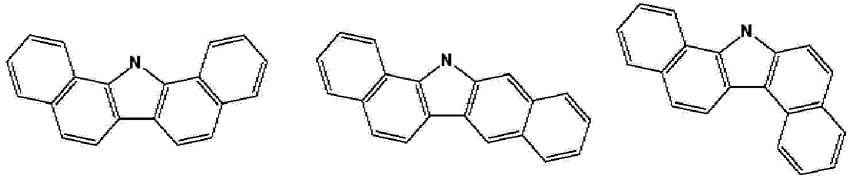

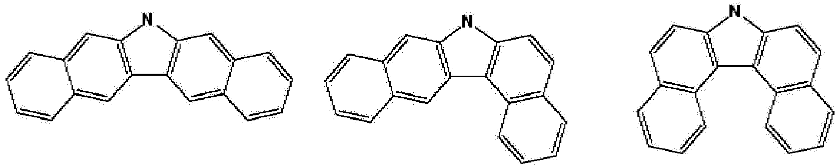

In the present specification, a naphthobenzofuran group may be any one of the following structures.

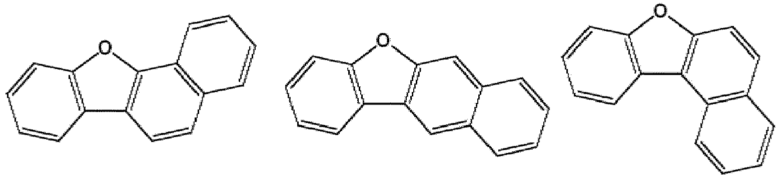

In the present specification, a naphthobenzothiophene group may be any one of the following structures.

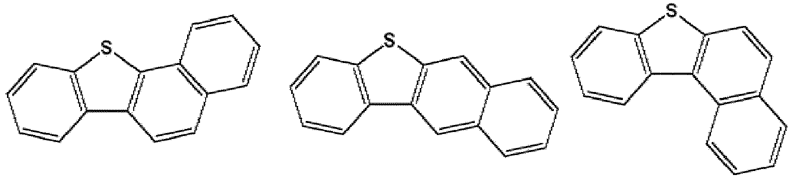

In the present specification, a silyl group includes Si and is a substituent to which the Si atom is directly linked as a radical, and is represented by - Si(R107)(R108)(R109), and R107 to R109 are the same as or different from each other, and may be each independently a substituent composed of at least one of hydrogen; deuterium; a halogen group; an alkyl group; an alkenyl group; an alkoxy group; a cycloalkyl group; a heterocycloalkyl group; an aryl group; and a heteroaryl group. Specific examples of the silyl group include 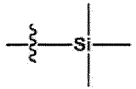 (a trimethylsilyl group), 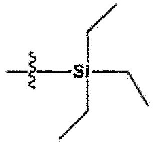 (a triethylsilyl group), 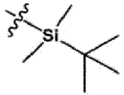 (a t-butyldimethylsilyl group), 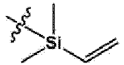 (a vinyldimethylsilyl group), 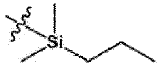 (a propyldimethylsilyl group), 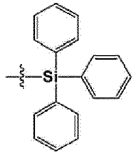 (a triphenylsilyl group), 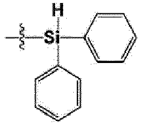 (a diphenylsilyl group), 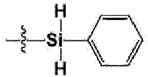 (a phenylsilyl group) and the like, but are not limited thereto.

In the present specification, a phosphine oxide group is represented by -P(=O)(R110)(R111), and R110 and R111 are the same as or different from each other, and may be each independently a substituent composed of at least one of hydrogen; deuterium; a halogen group; an alkyl group; an alkenyl group; an alkoxy group; a cycloalkyl group; a heterocycloalkyl group; an aryl group; and a heteroaryl group. Specifically, the phosphine oxide group may be substituted with an alkyl group or an aryl group, and the above-described example may be applied to the alkyl group and the aryl group. Examples of the phosphine oxide group include a dimethylphosphine oxide group, a diphenylphosphine oxide group, dinaphthylphosphine oxide, and the like, but are not limited thereto.

In the present specification, an amine group is represented by -N(R112)(R113), and R112 and R113 are the same as or different from each other, and are each independently a substituent composed of at least one of hydrogen; deuterium; a halogen group; an alkyl group; an alkenyl group; an alkoxy group; a cycloalkyl group; a heterocycloalkyl group; an aryl group; and a heteroaryl group. The amine group may be selected from the group consisting of -NH₂; a monoalkylamine group; a monoarylamine group; a monoheteroarylamine group; a dialkylamine group; a diarylamine group; a diheteroarylamine group; an alkylarylamine group; an alkylheteroarylamine group; and an arylheteroarylamine group, and the number of carbon atoms thereof is not particularly limited, but is preferably 1 to 30. Specific examples of the amine group include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group, and the like, but are not limited thereto.

In the present specification, the above-described examples of the aryl group may be applied to an arylene group except for a divalent arylene group.

In the present specification, the above-described examples of the heteroaryl group may be applied to a heteroarylene group except for a divalent heteroarylene group.

In the present specification, the "adjacent" group may mean a substituent substituted with an atom directly linked to an atom in which the corresponding substituent is substituted, a substituent disposed to be sterically closest to the corresponding substituent, or another substituent substituted with an atom in which the corresponding substituent is substituted. For example, two substituents substituted at the ortho position in a benzene ring and two substituents substituted with the same carbon in an aliphatic ring may be interpreted as groups which are "adjacent" to each other.

Hydrocarbon rings and hetero rings that adjacent groups may form include an aliphatic hydrocarbon ring, an aromatic hydrocarbon ring, an aliphatic hetero ring and an aromatic hetero ring, and structures exemplified by the above-describe cycloalkyl group, aryl group, heterocycloalkyl group and heteroaryl group may be applied to the rings, except for those that are not monovalent groups.

In an exemplary embodiment of the present application, provided is the compound represented by Chemical Formula 1.

In an exemplary embodiment of the present application, a group not represented by a substituent; or a group represented by hydrogen may mean being all substitutable with deuterium. That is, it may be shown that hydrogen; or deuterium can be substituted with each other.

In an exemplary embodiment of the present application, the deuterium content of the heterocyclic compound of Chemical Formula 1 may be more than 0% and 100% or less.

In another exemplary embodiment, the deuterium content of the heterocyclic compound of Chemical Formula 1 may be 5% to 100%, 7% to 100%, 10% to 100%, 15% to 100%, or 20% to 100%.

In general, compounds bonded with hydrogen and compounds substituted with deuterium exhibit a difference in thermodynamic behavior. The reason for this is that the mass of a deuterium atom is 2-fold higher than that of hydrogen, but due to the difference in the mass of atoms, deuterium is characterized by having even lower vibration energy. In addition, the bond length of carbon and deuterium is shorter than that of a bond with hydrogen, and a dissociation energy used to break the bond is also stronger than that of the bond with hydrogen. This is because the van der Waals radius of deuterium is smaller than that of hydrogen, and thus the extension amplitude of a bond between carbon and deuterium becomes even narrower.

The deuterium-substituted compound in the heterocyclic compound of Chemical Formula 1 of the present invention is characterized in that the energy in the ground state is further lower than that of the hydrogen-substituted compound, and the shorter the bond length between carbon and deuterium is, the smaller the molecular hardcore volume is. Accordingly, the electrical polarizability may be reduced and the intermolecular interaction can be weakened, so that the volume of the device thin film may be increased. These characteristics induce an effect of lowering the crystallinity by creating the amorphous state of a thin film. Therefore, deuterium substitution in the heterocyclic compound of Chemical Formula 1 may be effective in improving the heat resistance of an OLED device, thereby improving the service life and driving characteristics.

In an exemplary embodiment of the present application, the 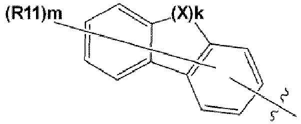 of Chemical Formula 1 may be represented by the following Chemical Formula 1-A-1 or Chemical Formula 1-A-2.

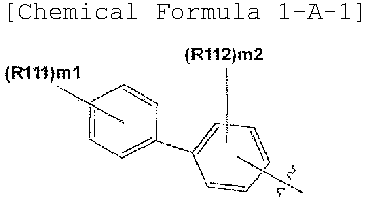

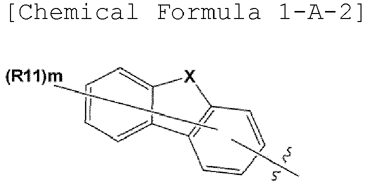

In Chemical Formulae 1-A-1 and 1-A-2,
R111 and R112 are the same as or different from each other, and are each independently hydrogen; deuterium; a halogen group; a cyano group; a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group,
m1 is an integer from 0 to 5, m2 is an integer from 0 to 4, and when m1 and m2 are each 2 or higher, substituents in the parenthesis are the same as or different from each other, and
the definitions of X, R11 and m are the same as the definitions in Chemical Formula 1.

In an exemplary embodiment of the present application, when k of 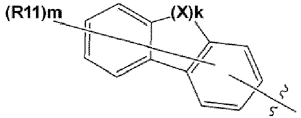 of Chemical Formula 1 is 0, it means that there is no bond rather than a direct bond. That is, R11 may be represented by a substituted or unsubstituted biphenyl group.

In an exemplary embodiment of the present application, when k is 0, the 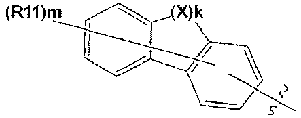 of Chemical Formula 1 may be represented by Chemical Formula 1-A-1.

In an exemplary embodiment of the present application, when k is 1, the 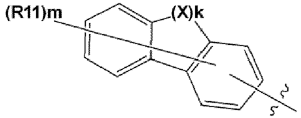 of Chemical Formula 1 may be represented by Chemical Formula 1-A-2.

In an exemplary embodiment of the present application, 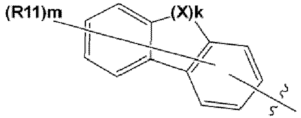 in the structure of Chemical Formula 1 may be any one of the following structures.

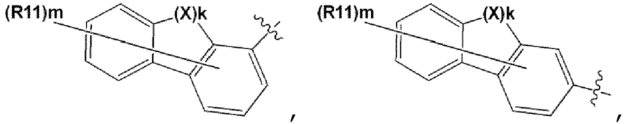

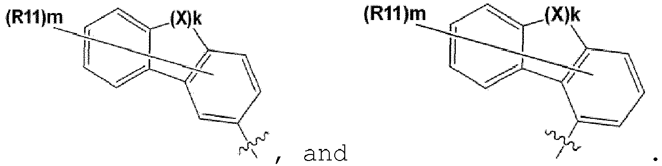

In the structural formulae,
the definition of each substituent is the same as the definition in Chemical Formula 1.

In an exemplary embodiment of the present application, Chemical Formula 1 may be dividedly represented by the following Structures 1-A to 1-D.

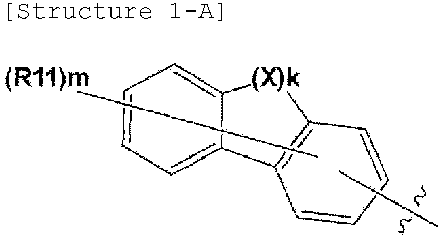

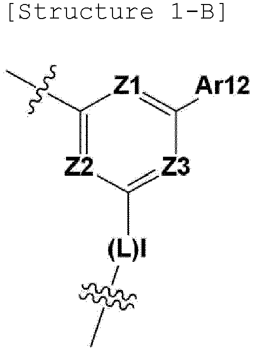

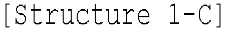

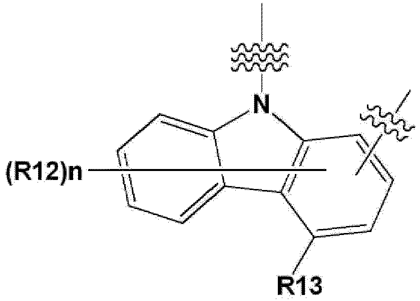

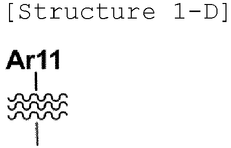

In Structures 1-A to 1-D,
the definitions of Z1 to Z3, L, l, X, R11 to R13, k, m, n, Ar11 and Ar12 are the same as the definitions in Chemical Formula 1, and 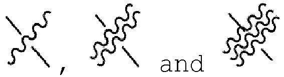 are each a binding position where those which are the same are linked to each other.

In another exemplary embodiment, the deuterium content of the following Structure 1-A may be 0% to 100%.

In still another exemplary embodiment, the deuterium content of the following Structure 1-A may be 0% to 70%.

In yet another exemplary embodiment, the deuterium content of the following Structure 1-A may be 0% to 50%.

In yet another exemplary embodiment, the deuterium content of the following Structure 1-A may be 0% to 30%.

In yet another exemplary embodiment, the deuterium content of the following Structure 1-A may be 0%.

In an exemplary embodiment of the present application, the deuterium content of the following Structure 1-B may be 0% to 100%.

In yet another exemplary embodiment, the deuterium content of the following Structure 1-B may be 0% to 70%.

In yet another exemplary embodiment, the deuterium content of the following Structure 1-B may be 0% to 50%.

In yet another exemplary embodiment, the deuterium content of the following Structure 1-B may be 0% to 30%.

In yet another exemplary embodiment, the deuterium content of the following Structure 1-B may be 0%.

In an exemplary embodiment of the present application, the deuterium content of the following Structure 1-C may be more than 0% and 100% or less.

In another exemplary embodiment, the deuterium content of the following Structure 1-C may be 30% to 100%.

In still another exemplary embodiment, the deuterium content of the following Structure 1-C may be 50% to 100%.

In yet another exemplary embodiment, the deuterium content of the following Structure 1-C may be 70% to 100%.

In yet another exemplary embodiment, the deuterium content of the following Structure 1-C may be 90% to 100%.

In yet another exemplary embodiment, the deuterium content of the following Structure 1-C may be 100%.

In an exemplary embodiment of the present application, the deuterium content of the following Structure 1-D may be 0% to 100%.

In yet another exemplary embodiment, the deuterium content of the following Structure 1-D may be 30% to 100%.

In yet another exemplary embodiment, the deuterium content of the following Structure 1-D may be 50% to 100%.

In yet another exemplary embodiment, the deuterium content of the following Structure 1-D may be 70% to 100%.

In yet another exemplary embodiment, the deuterium content of the following Structure 1-D may be 90% to 100%.

In yet another exemplary embodiment, the deuterium content of the following Structure 1-D may be 100%.

In an exemplary embodiment of the present application, Chemical Formula 1 may be represented by any one of the following Chemical Formulae 1-1-1 to 1-1-3.

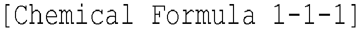

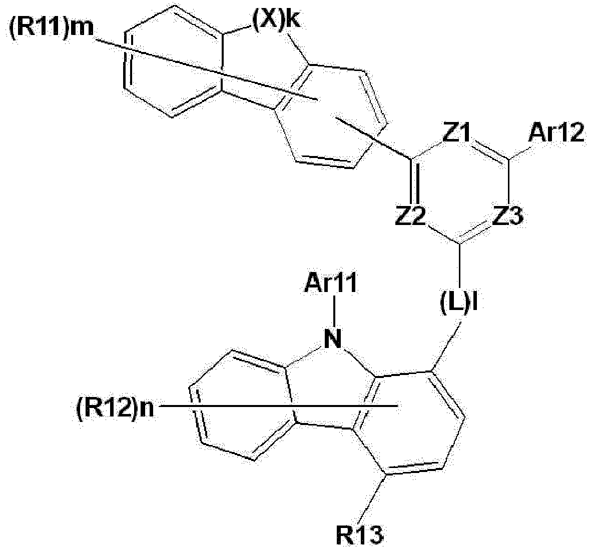

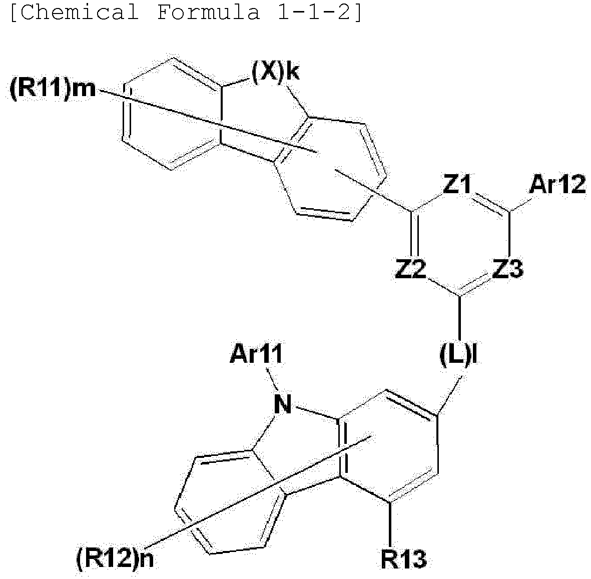

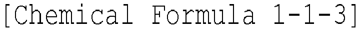

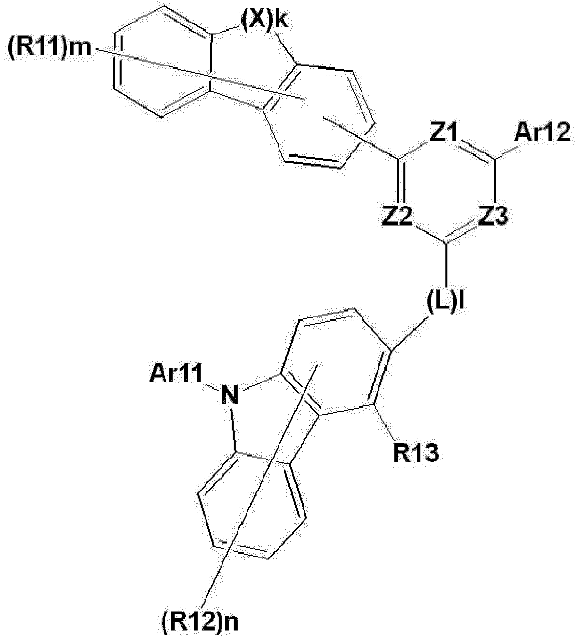

In Chemical Formulae 1-1-1 to 1-1-3,
the definitions of Z1 to Z3, L, l, X, R11 to R13, k, m, n, Ar11 and Ar12 are the same as the definitions in Chemical Formula 1.

In an exemplary embodiment of the present application, Z1 to Z3 are the same as or different from each other, and may be each independently N; or CR, and at least one may be N.

In another exemplary embodiment, Z1 is N, and Z2 and Z3 may be CR.

In still another exemplary embodiment, Z2 is N, and Z1 and Z3 may be CR.

In yet another exemplary embodiment, Z3 is N, and Z1 and Z2 may be CR.

In yet another exemplary embodiment, Z1 and Z2 are N, and Z3 may be CR.

In yet another exemplary embodiment, Z2 and Z3 are N, and Z1 may be CR.

In yet another exemplary embodiment, Z1 and Z3 are N, and Z2 may be CR.

In yet another exemplary embodiment, Z1 to Z3 may be all N.

In an exemplary embodiment of the present application, R may be hydrogen; deuterium; a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group.

In another exemplary embodiment, R may be hydrogen; deuterium; a substituted or unsubstituted C1 to C40 alkyl group; a substituted or unsubstituted C3 to C40 cycloalkyl group; a substituted or unsubstituted C2 to C40 heterocycloalkyl group; a substituted or unsubstituted C6 to C40 aryl group; or a substituted or unsubstituted C2 to C40 heteroaryl group.

In still another exemplary embodiment, R may be hydrogen; deuterium; a substituted or unsubstituted C1 to C20 alkyl group; a substituted or unsubstituted C3 to C20 cycloalkyl group; a substituted or unsubstituted C2 to C20 heterocycloalkyl group; a substituted or unsubstituted C6 to C20 aryl group; or a substituted or unsubstituted C2 to C20 heteroaryl group.

In yet another exemplary embodiment, R may be hydrogen; deuterium; a C1 to C20 alkyl group; a C3 to C20 cycloalkyl group; a C2 to C20 heterocycloalkyl group; a C6 to C20 aryl group; or a C2 to C20 heteroaryl group.

In yet another exemplary embodiment, R may be hydrogen; deuterium; a C1 to C10 alkyl group; a C3 to C10 cycloalkyl group; a C2 to C10 heterocycloalkyl group; a C6 to C10 aryl group; or a C2 to C10 heteroaryl group.

In yet another exemplary embodiment, R may be hydrogen; deuterium; a C1 to C10 alkyl group; or a C6 to C10 aryl group.

In yet another exemplary embodiment, R may be hydrogen; or deuterium.

In an exemplary embodiment of the present application, L may be a direct bond; or a substituted or unsubstituted C6 to C60 arylene group.

In another exemplary embodiment, L may be a direct bond; or a substituted or unsubstituted C6 to C40 arylene group.

In still another exemplary embodiment, L may be a direct bond; or a substituted or unsubstituted C6 to C20 arylene group.

In yet another exemplary embodiment, L may be a direct bond; a substituted or unsubstituted phenylene group; or a substituted or unsubstituted biphenylene group.

In yet another exemplary embodiment, L may be a direct bond; a phenylene group which is unsubstituted or substituted with deuterium or a phenyl group; or a biphenylene group which is unsubstituted or substituted with deuterium.

In an exemplary embodiment of the present application, X may be O; or S.

In another exemplary embodiment, X may be O.

In still another exemplary embodiment, X may be S.

In an exemplary embodiment of the present application, R11 to R13 are the same as or different from each other, and may be each independently hydrogen; deuterium; a halogen group; a cyano group; a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group.

In another exemplary embodiment, R11 to R13 are the same as or different from each other, and may be each independently hydrogen; deuterium; a halogen group; a cyano group; a substituted or unsubstituted C1 to C40 alkyl group; a substituted or unsubstituted C3 to C40 cycloalkyl group; a substituted or unsubstituted C2 to C40 heterocycloalkyl group; a substituted or unsubstituted C6 to C40 aryl group; or a substituted or unsubstituted C2 to C40 heteroaryl group.

In still another exemplary embodiment, R11 to R13 are the same as or different from each other, and may be each independently hydrogen; deuterium; a halogen group; a cyano group; a substituted or unsubstituted C1 to C20 alkyl group; a substituted or unsubstituted C3 to C20 cycloalkyl group; a substituted or unsubstituted C2 to C20 heterocycloalkyl group; a substituted or unsubstituted C6 to C20 aryl group; or a substituted or unsubstituted C2 to C20 heteroaryl group.

In yet another exemplary embodiment, R11 to R13 are the same as or different from each other, and may be each independently hydrogen; deuterium; or a substituted or unsubstituted C6 to C20 aryl group.

In yet another exemplary embodiment, R11 to R13 are the same as or different from each other, and may be each independently hydrogen; deuterium; or a substituted or unsubstituted phenyl group.

In yet another exemplary embodiment, R11 to R13 are the same as or different from each other, and may be each independently hydrogen; deuterium; or a phenyl group which is unsubstituted or substituted with deuterium.

In an exemplary embodiment of the present invention, Ar11 and Ar12 are the same as or different from each other, and may be each independently a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group.

In another exemplary embodiment, Ar11 and Ar12 are the same as or different from each other, and may be each independently a substituted or unsubstituted C1 to C40 alkyl group; a substituted or unsubstituted C3 to C40 cycloalkyl group; a substituted or unsubstituted C2 to C40 heterocycloalkyl group; a substituted or unsubstituted C6 to C40 aryl group; or a substituted or unsubstituted C2 to C40 heteroaryl group.

In still another exemplary embodiment, Ar11 and Ar12 are the same as or different from each other, and may be each independently a substituted or unsubstituted C1 to C20 alkyl group; a substituted or unsubstituted C3 to C20 cycloalkyl group; a substituted or unsubstituted C2 to C20 heterocycloalkyl group; a substituted or unsubstituted C6 to C20 aryl group; or a substituted or unsubstituted C2 to C20 heteroaryl group.

In yet another exemplary embodiment, Ar11 and Ar12 are the same as or different from each other, and may be each independently a substituted or unsubstituted C6 to C20 aryl group; or a substituted or unsubstituted C2 to C20 heteroaryl group.

In yet another exemplary embodiment, Ar11 and Ar12 are the same as or different from each other, and may be each independently a substituted or unsubstituted phenyl group; a substituted or unsubstituted biphenyl group; a substituted or unsubstituted terphenyl group; a substituted or unsubstituted dibenzofuran group; or a substituted or unsubstituted dibenzothiophene group.

In yet another exemplary embodiment, Ar11 and Ar12 are the same as or different from each other, and may be each independently a phenyl group which is unsubstituted or substituted with deuterium; a biphenyl group which is unsubstituted or substituted with deuterium; a terphenyl group which is unsubstituted or substituted with deuterium; a dibenzofuran group which is unsubstituted or substituted with a phenyl group; or a dibenzothiophene group which is unsubstituted or substituted with a phenyl group.

In an exemplary embodiment of the present application, Chemical Formula 1 may be represented by any one of the following Chemical Formulae 1-1 to 1-3.

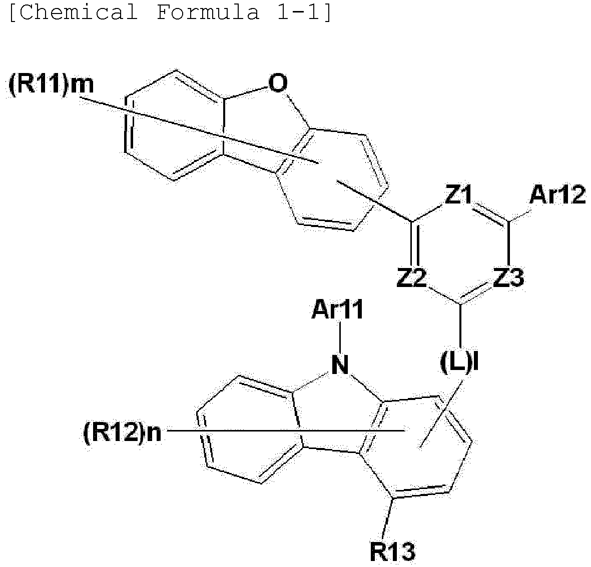

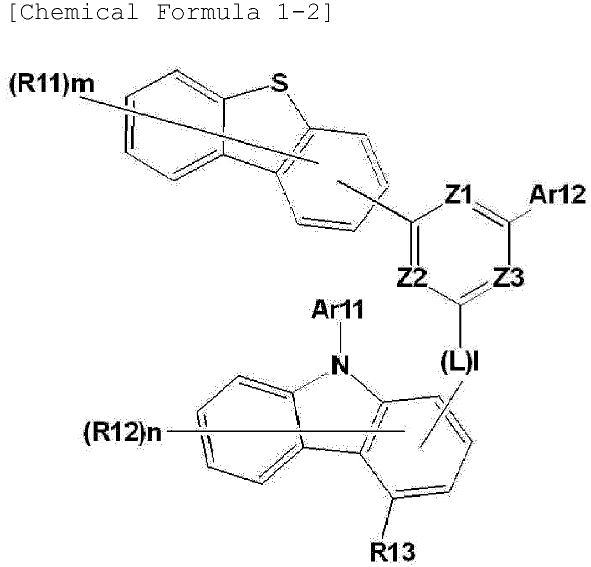

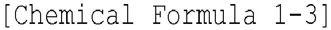

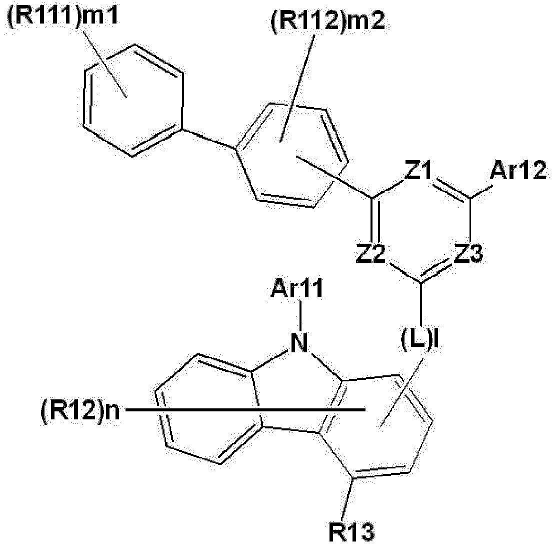

In Chemical Formulae 1-1 to 1-3,
the definitions of Z1 to Z3, L, l, R11 to R13, m, n, Ar11 and Ar12 are the same as the definitions in Chemical Formula 1,
R111 and R112 are the same as or different from each other, and are each independently hydrogen; deuterium; a halogen group; a cyano group; a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group, and
m1 is an integer from 0 to 5, m2 is an integer from 0 to 4, and when m1 and m2 are each 2 or higher, substituents in the parenthesis are the same as or different from each other.

In an exemplary embodiment of the present application, R111 and R112 are the same as or different from each other, and may be each independently hydrogen; deuterium; a halogen group; a cyano group; a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group.

In another exemplary embodiment, R111 and R112 are the same as or different from each other, and may be each independently hydrogen; deuterium; a halogen group; a cyano group; a substituted or unsubstituted C1 to C40 alkyl group; a substituted or unsubstituted C3 to C40 cycloalkyl group; a substituted or unsubstituted C2 to C40 heterocycloalkyl group; a substituted or unsubstituted C6 to C40 aryl group; or a substituted or unsubstituted C2 to C40 heteroaryl group.

In still another exemplary embodiment, R111 and R112 are the same as or different from each other, and may be each independently hydrogen; deuterium; a halogen group; a cyano group; a substituted or unsubstituted C1 to C20 alkyl group; a substituted or unsubstituted C3 to C20 cycloalkyl group; a substituted or unsubstituted C2 to C20 heterocycloalkyl group; a substituted or unsubstituted C6 to C20 aryl group; or a substituted or unsubstituted C2 to C20 heteroaryl group.

In yet another exemplary embodiment, R111 and R112 are the same as or different from each other, and may be each independently hydrogen; deuterium; or a substituted or unsubstituted C6 to C20 aryl group.

In yet another exemplary embodiment, R111 and R112 are the same as or different from each other, and may be each independently hydrogen; deuterium; or a substituted or unsubstituted phenyl group.

In yet another exemplary embodiment, R111 and R112 are the same as or different from each other, and may be each independently hydrogen; deuterium; or a phenyl group which is unsubstituted or substituted with deuterium.

In an exemplary embodiment of the present application, provided is a heterocyclic compound in which Chemical Formula 1 is represented by any one of the following compounds.

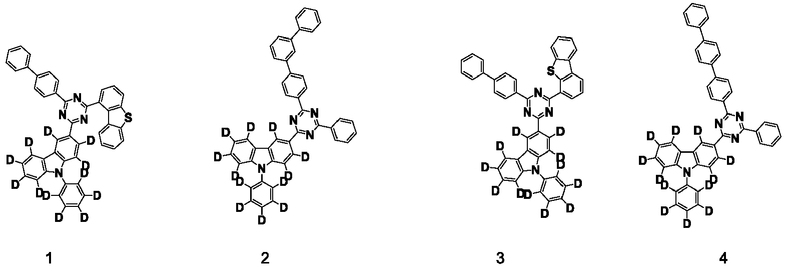

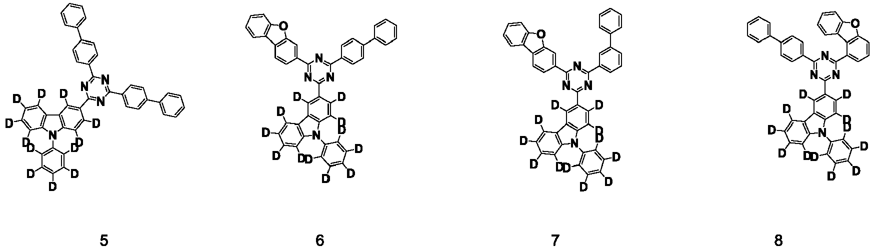

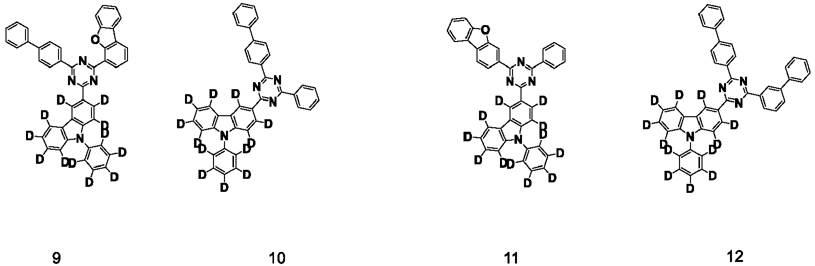

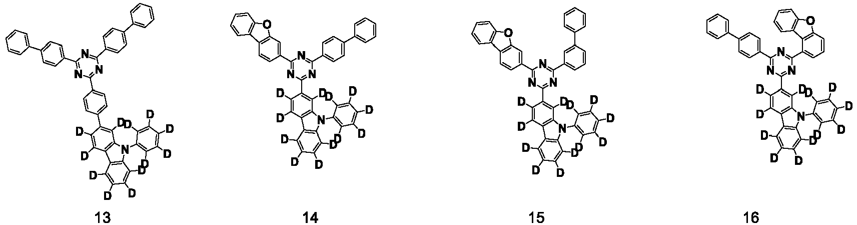

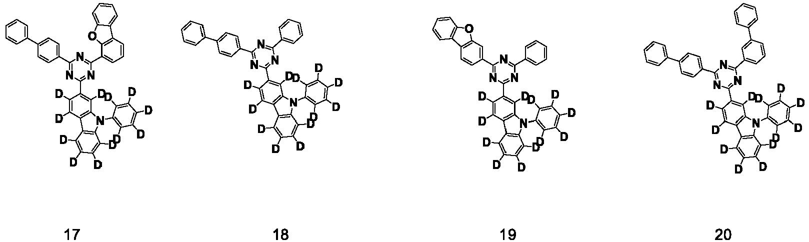

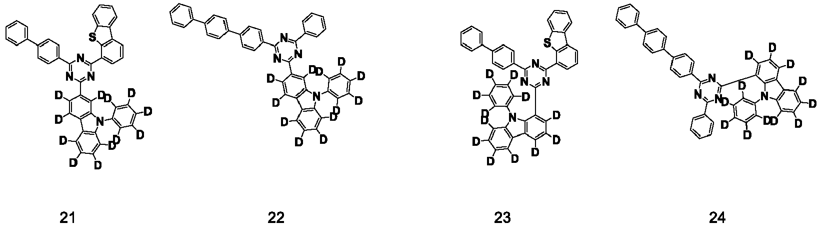

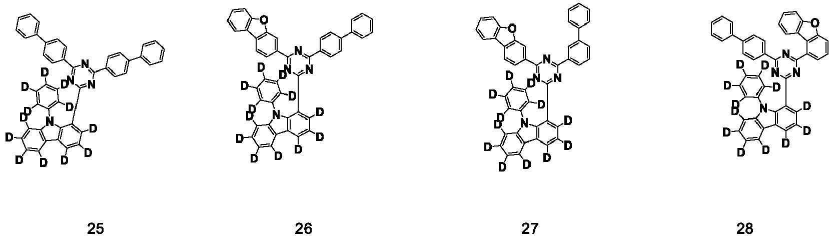

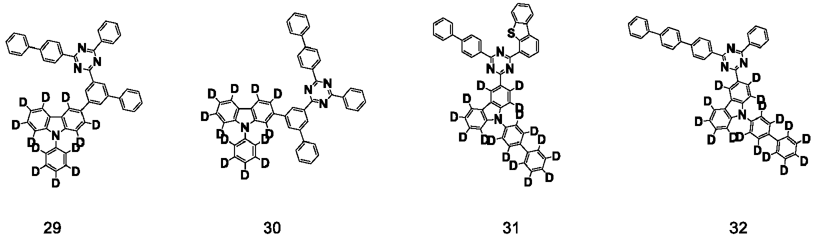

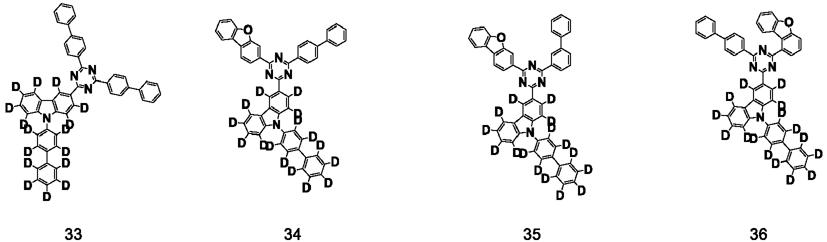

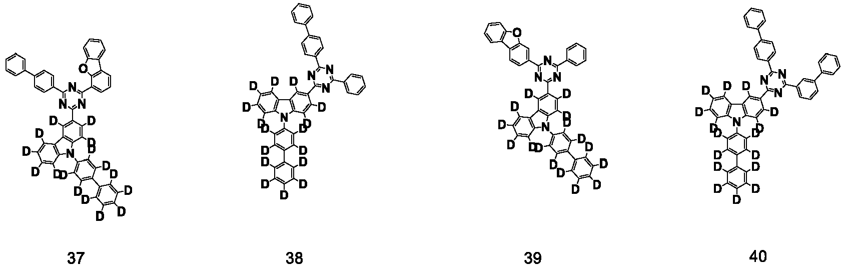

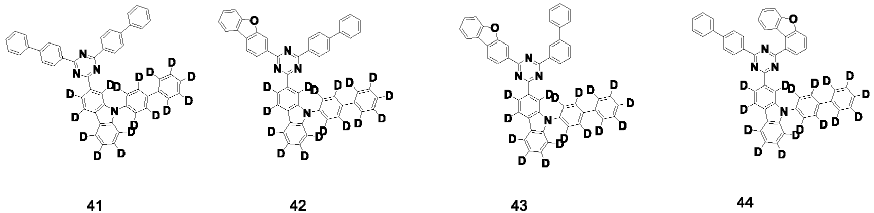

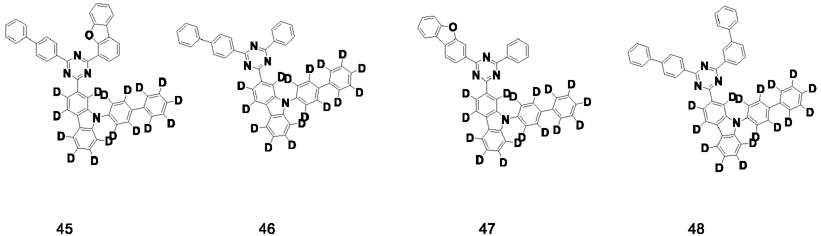

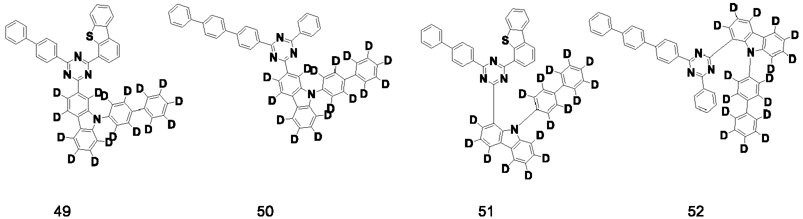

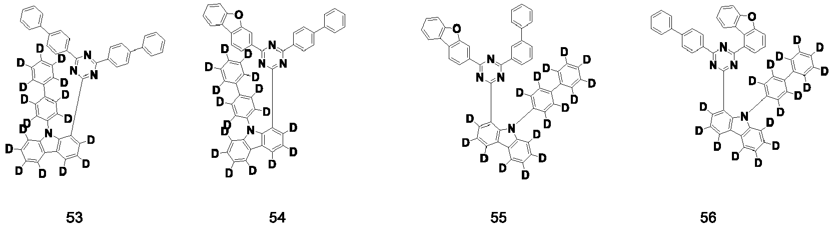

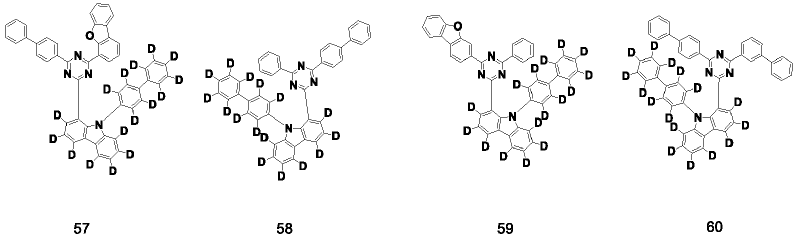

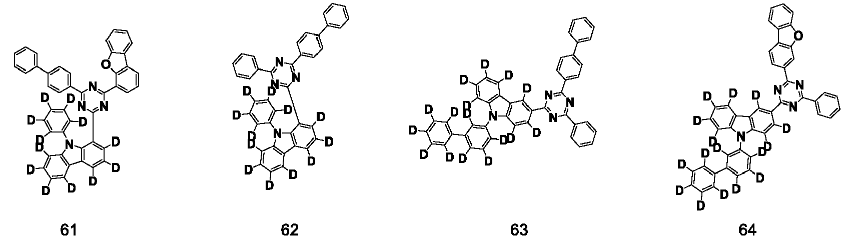

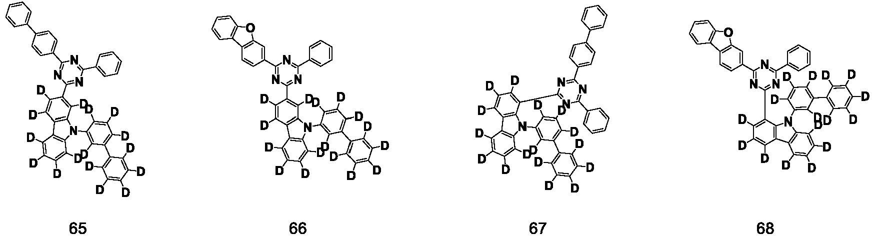

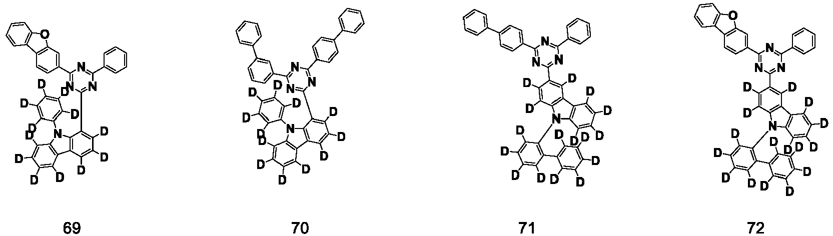

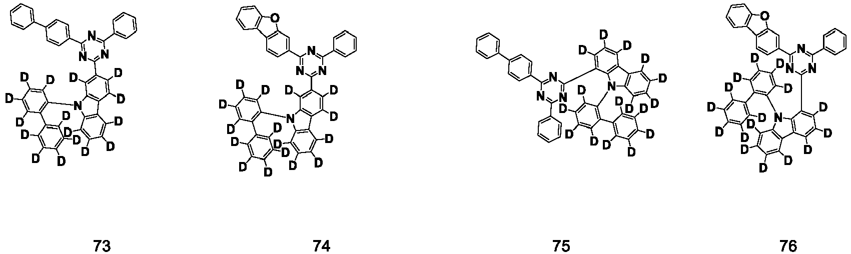

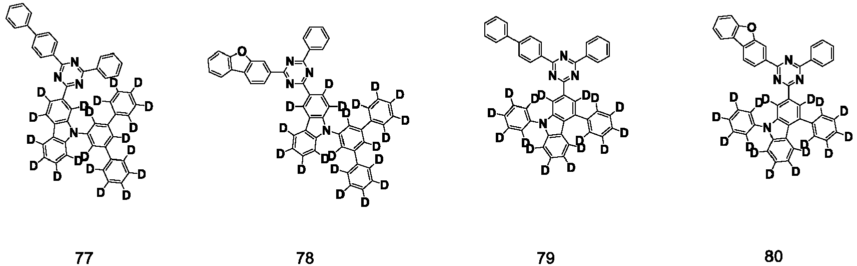

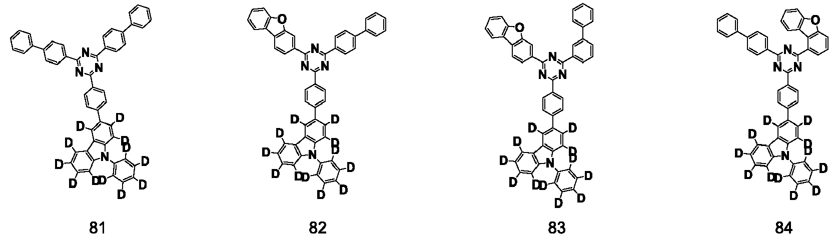

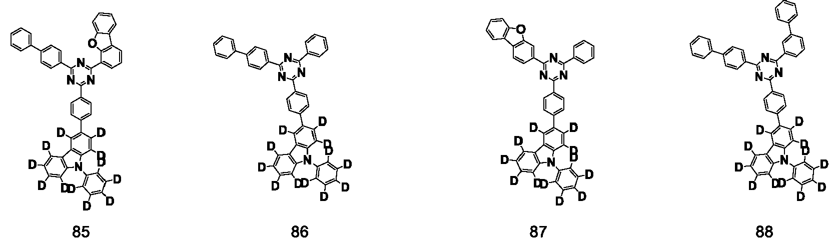

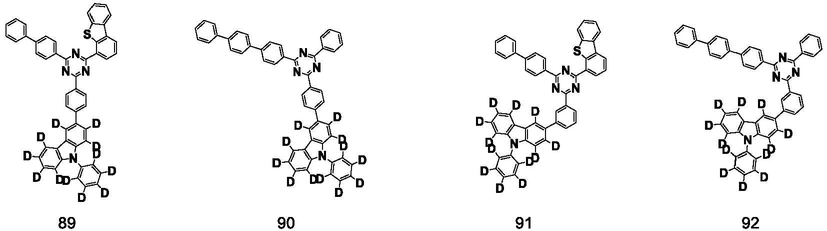

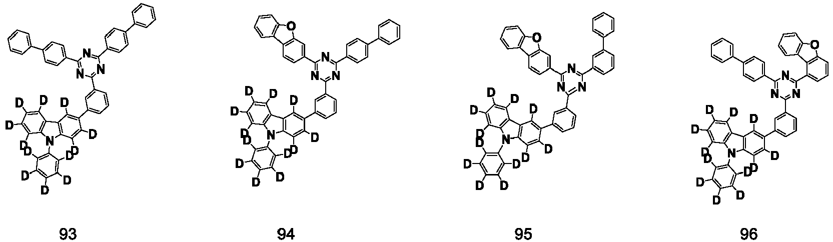

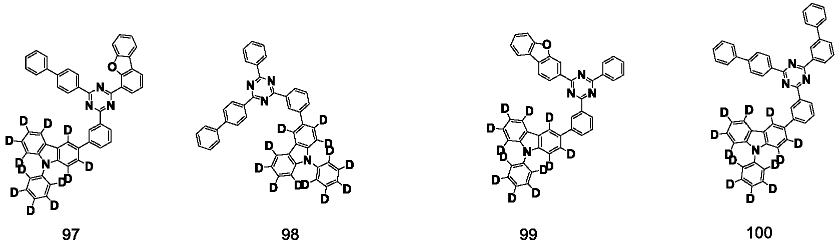

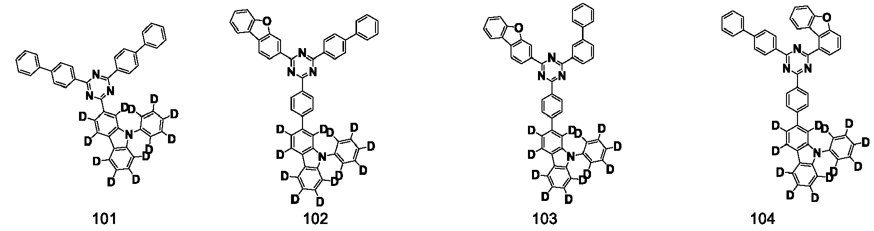

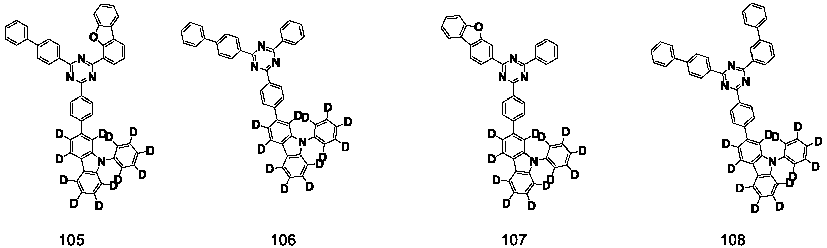

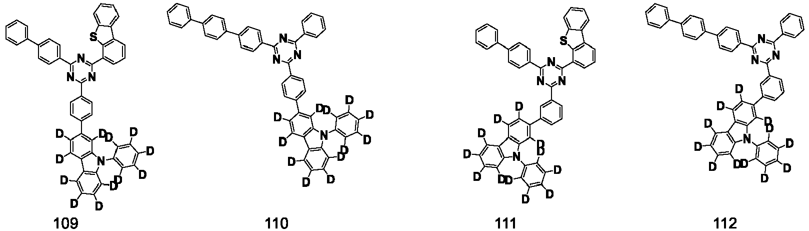

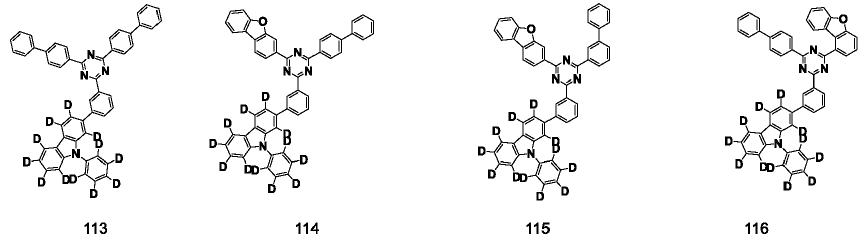

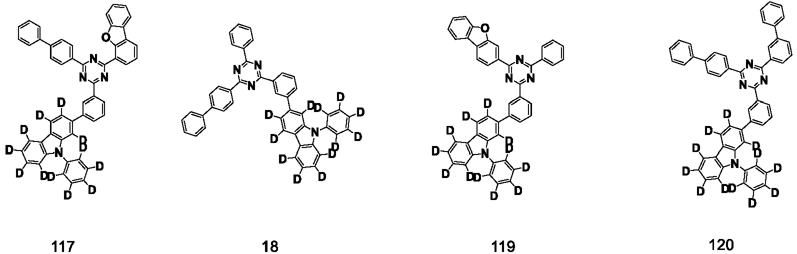

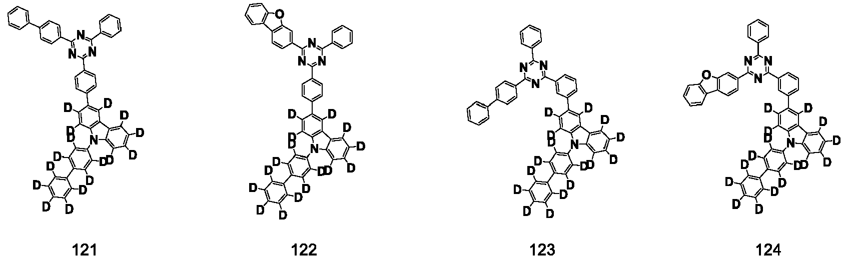

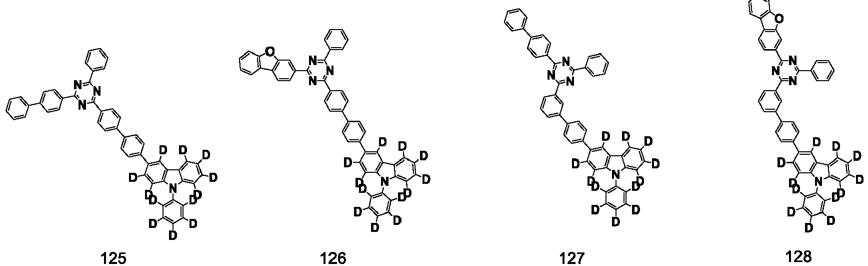

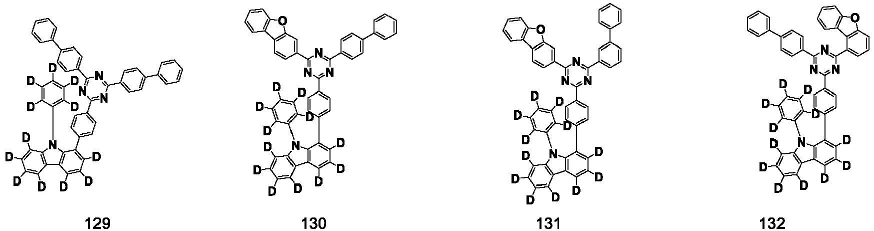

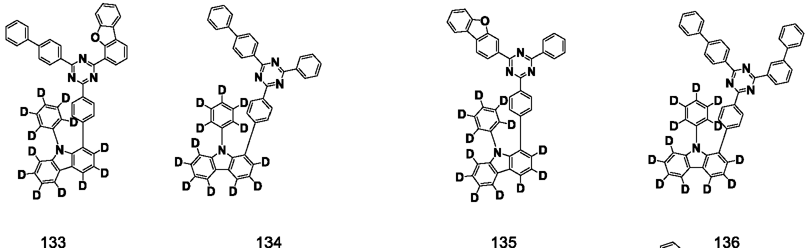

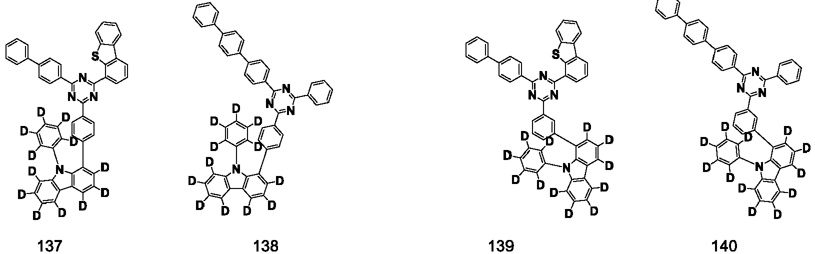

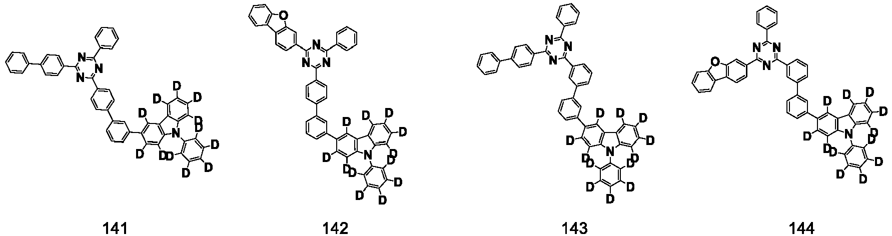

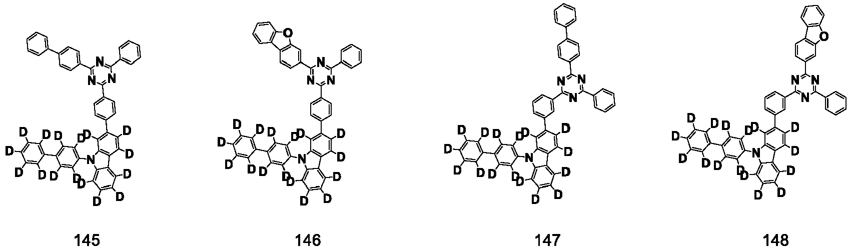

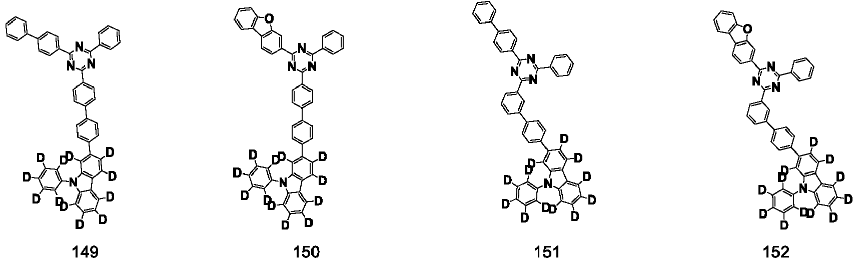

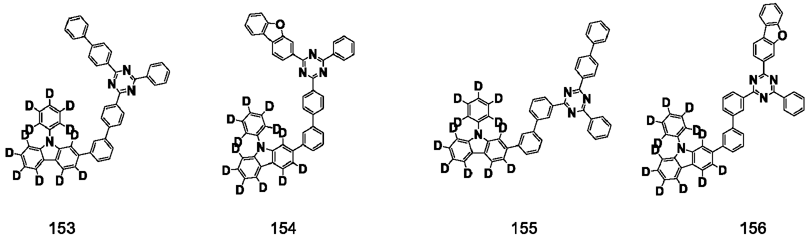

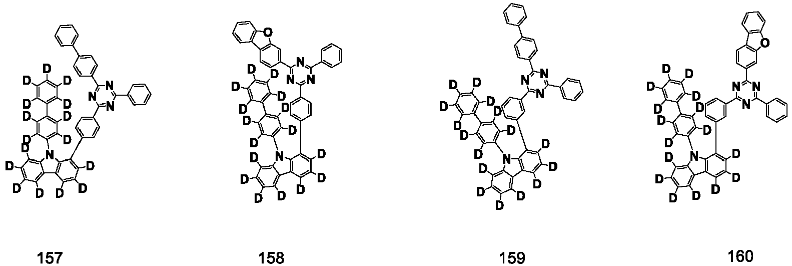

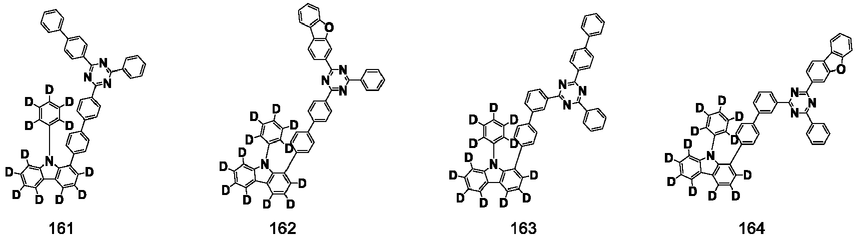

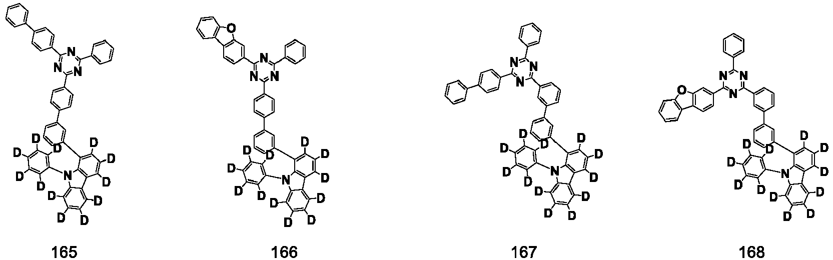

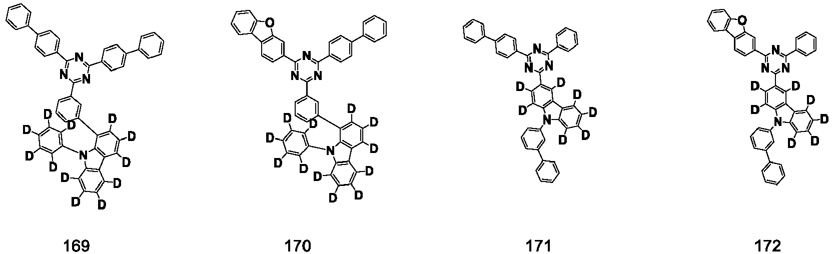

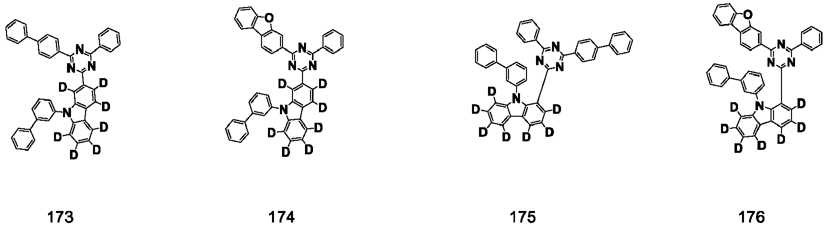

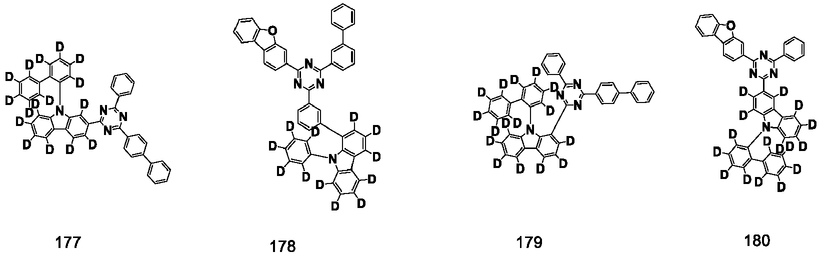

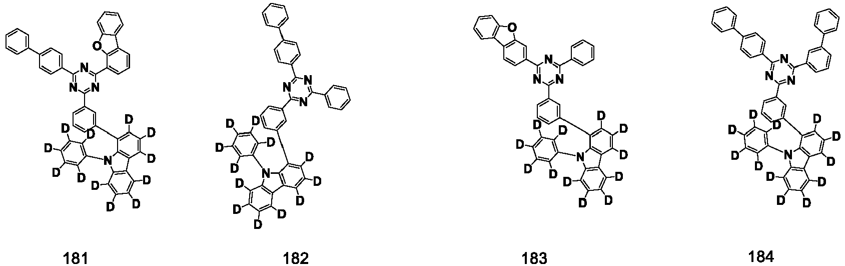

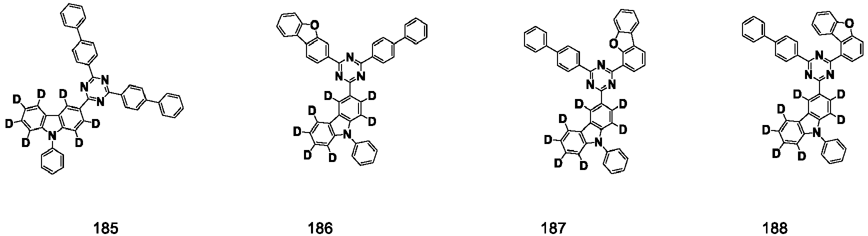

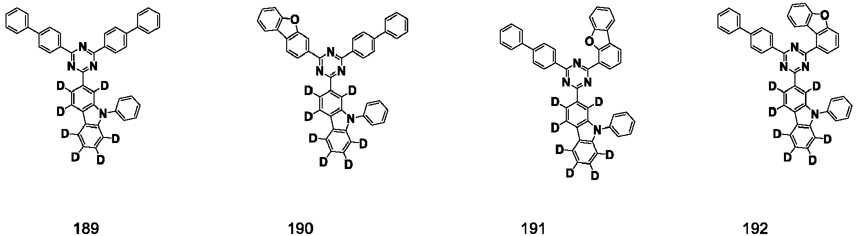

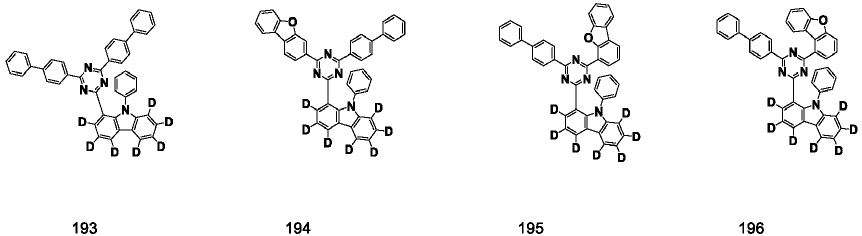

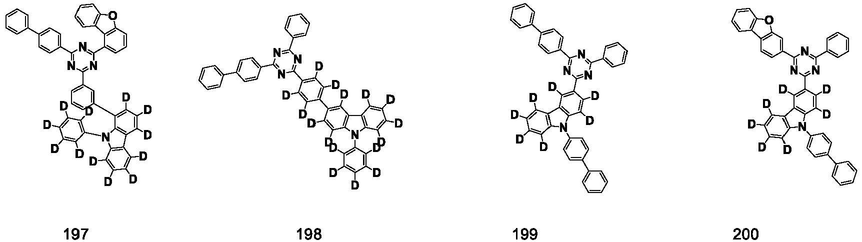

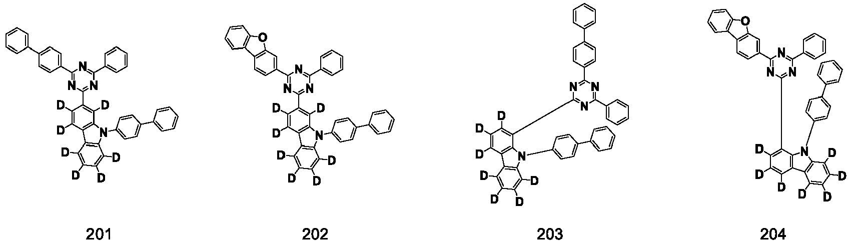

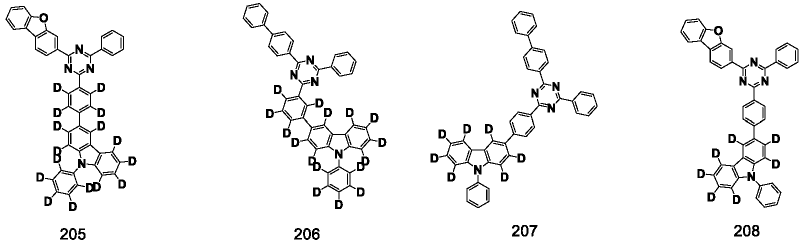

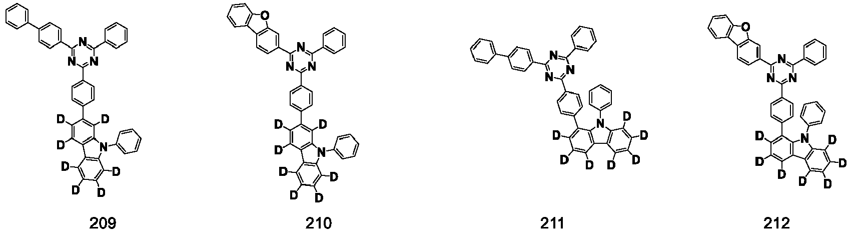

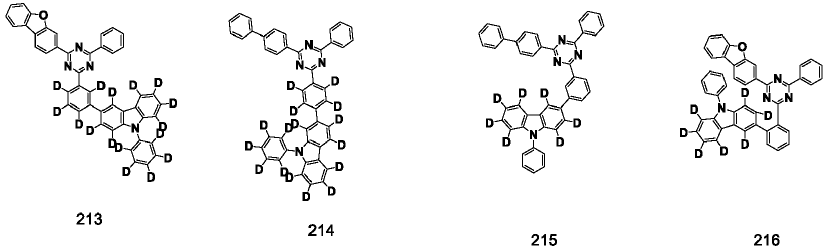

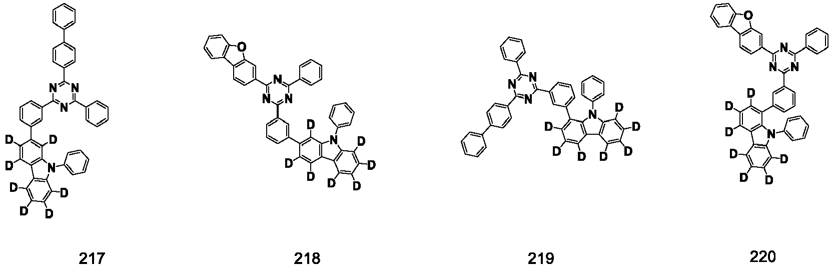

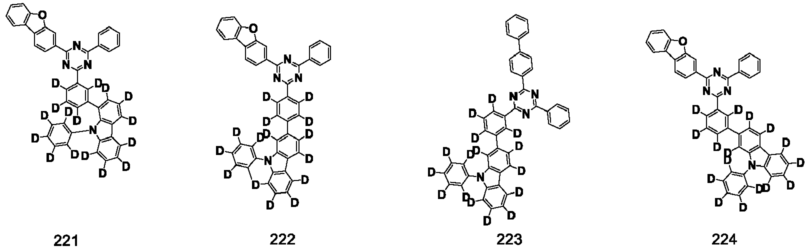

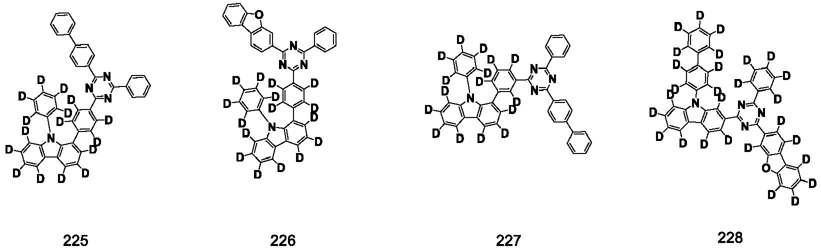

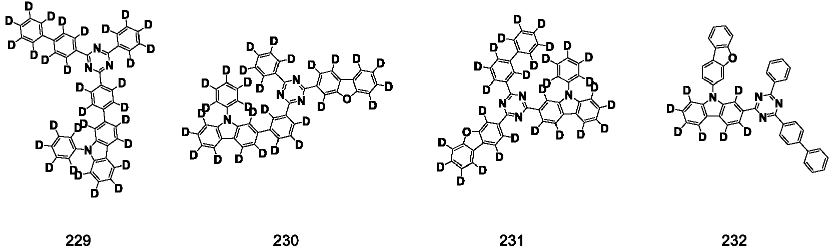

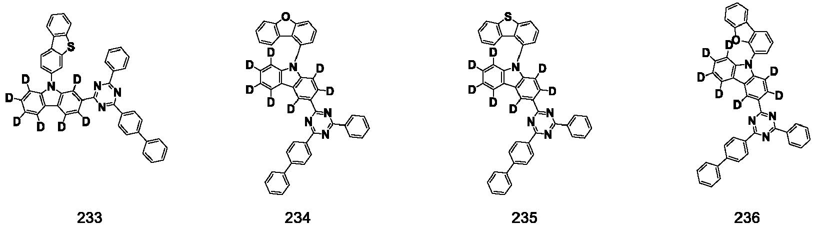

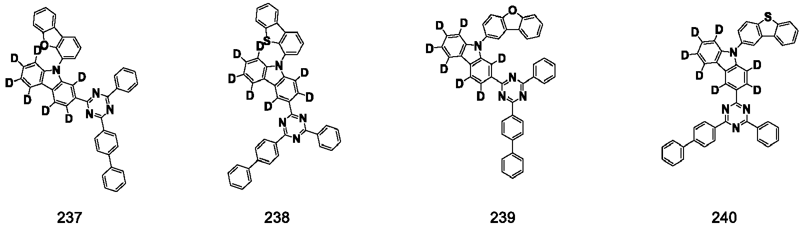

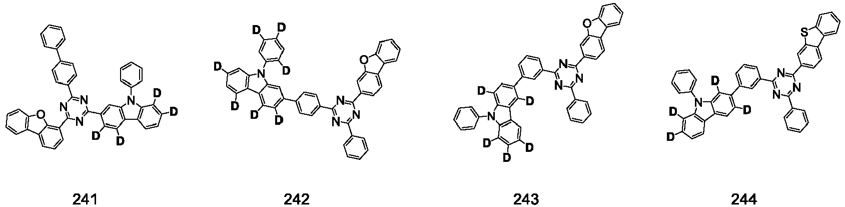

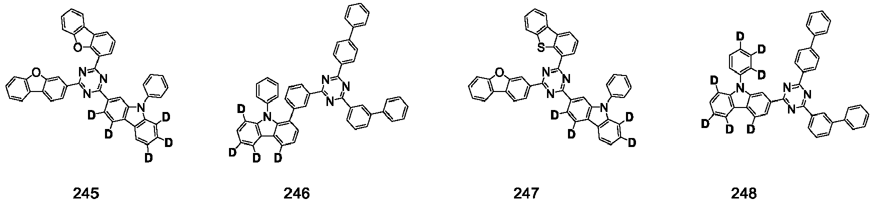

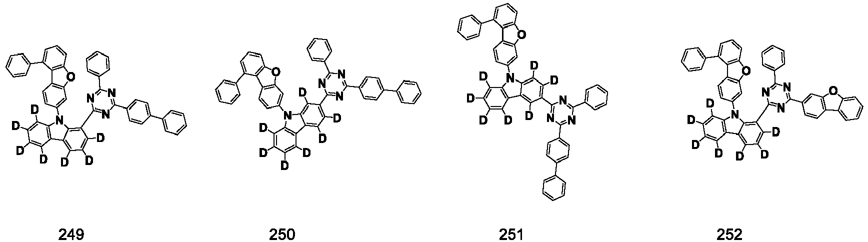

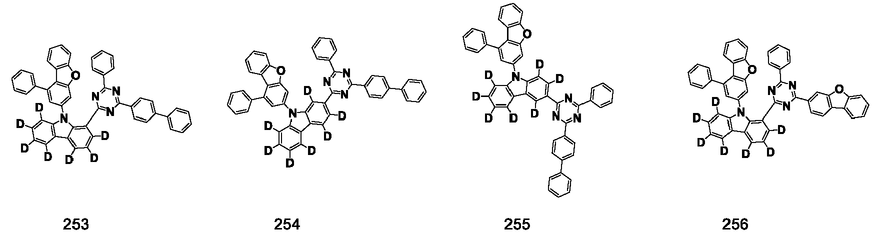

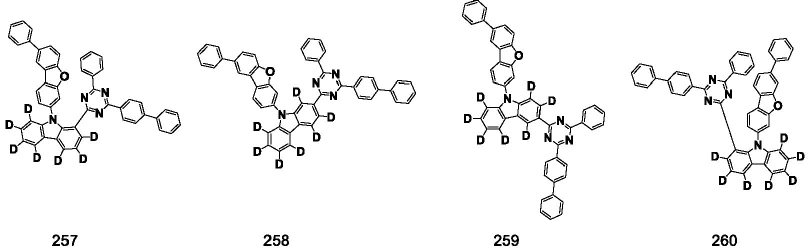

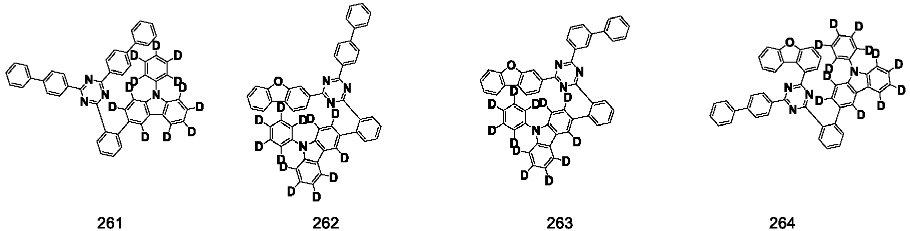

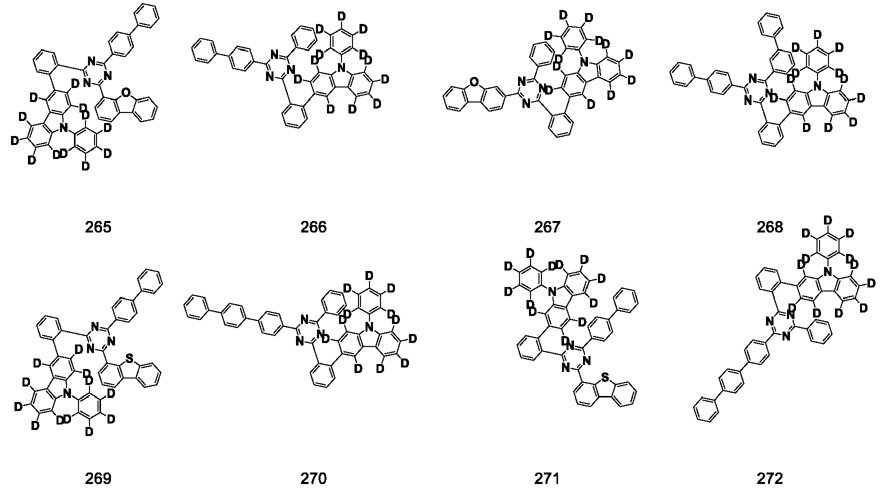

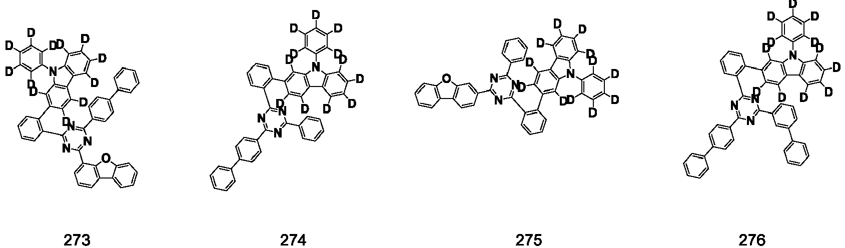

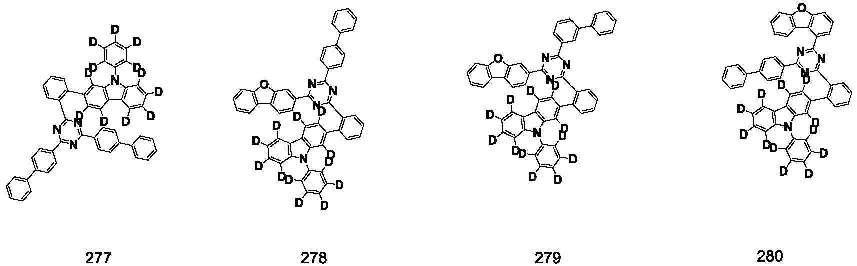

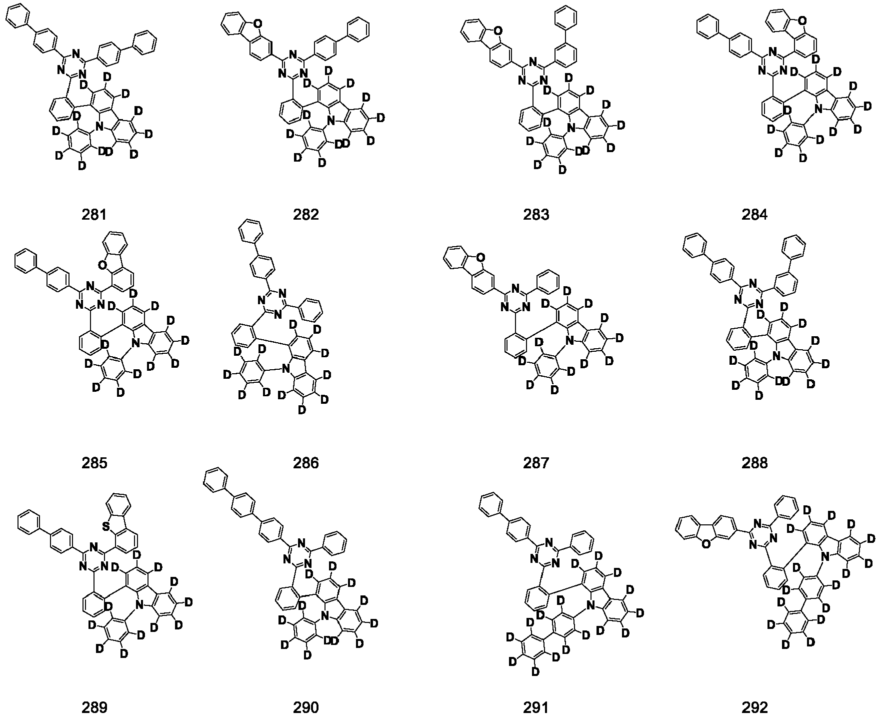

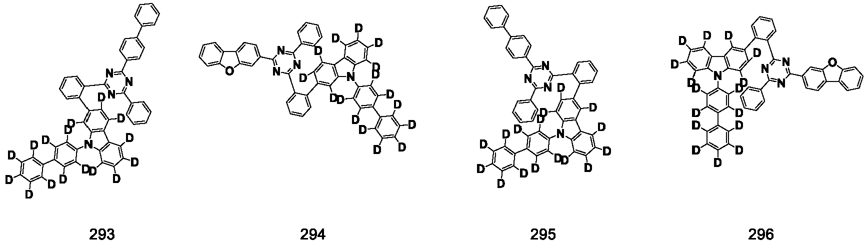

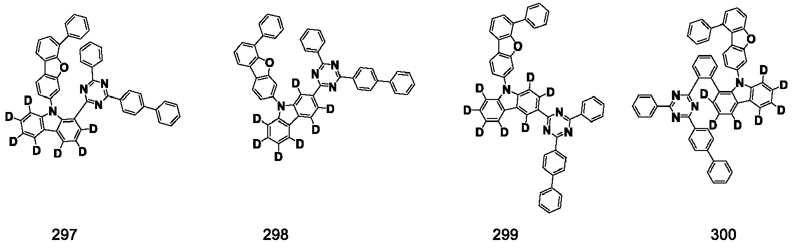

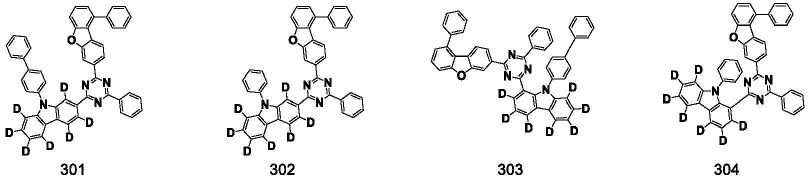

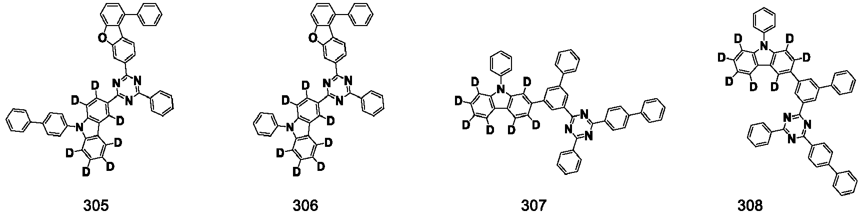

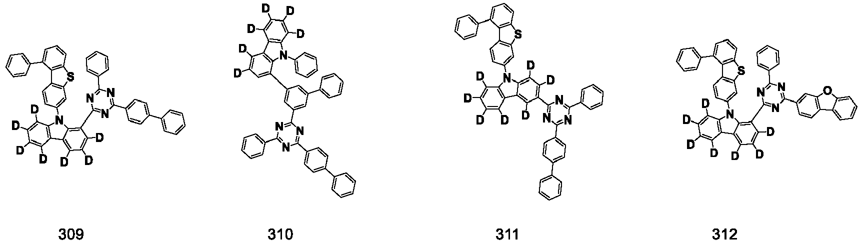

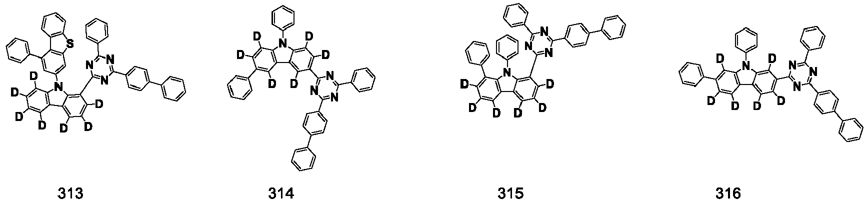

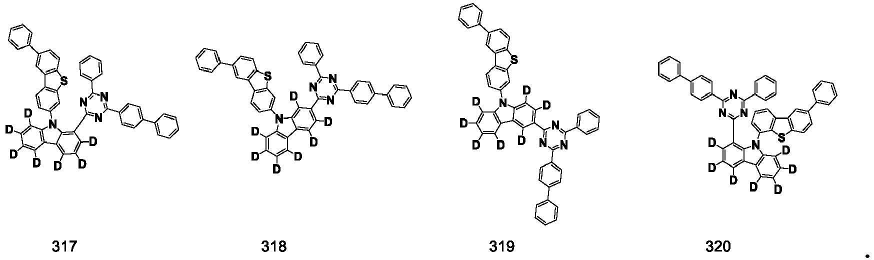

Further, various substituents may be introduced into the structure of Chemical Formula 1 to synthesize a compound having inherent characteristics of a substituent introduced. For example, a substituent usually used for a hole injection material, a hole transport material, a light emitting material, an electron transport material and an electron injection material, which are used when manufacturing an organic light emitting device, may be introduced into the core structure to synthesize a material which satisfies conditions required for each organic material layer.

In addition, by introducing various substituents into the structure of Chemical Formula 1, the bandgap may be finely adjusted, and meanwhile, the characteristics at the interface between the organic material layers may be improved.

In addition, the compound of Chemical Formula 1 has excellent thermal stability, and such thermal stability provides driving stability to the organic light emitting device and improves service life characteristics.

In an exemplary embodiment of the present application, provided is an organic light emitting device including: a first electrode; a second electrode provided to face the first electrode; and an organic material layer having one or more layers provided between the first electrode and the second electrode, in which one or more layers of the organic material layer include the heterocyclic compound represented by Chemical Formula 1.

In another exemplary embodiment, provided is an organic light emitting device including: a first electrode; a second electrode provided to face the first electrode; and an organic material layer having one or more layers provided between the first electrode and the second electrode, in which one or more layers of the organic material layer include one heterocyclic compound represented by Chemical Formula 1.

In still another exemplary embodiment, provided is an organic light emitting device including: a first electrode; a second electrode provided to face the first electrode; and an organic material layer having one or more layers provided between the first electrode and the second electrode, in which one or more layers of the organic material layer include two or more of the heterocyclic compound represented by Chemical Formula 1.

In yet another exemplary embodiment, the heterocyclic compound represented by Chemical Formula 1 may be used as a light emitting material for a light emitting layer of an organic light emitting device, and may be used as a phosphorescent green light emitting material.

In yet another exemplary embodiment, the heterocyclic compound represented by Chemical Formula 1 may be used as a light emitting material for a light emitting layer of an organic light emitting device, and may be used as an n-host material.

In an exemplary embodiment of the present application, the first electrode may be a positive electrode, and the second electrode may be a negative electrode.

In another exemplary embodiment, the first electrode may be a negative electrode, and the second electrode may be a positive electrode.

In an exemplary embodiment of the present application, the organic light emitting device may be a blue organic light emitting device, and the heterocyclic compound according to Chemical Formula 1 may be used as a material for the blue organic light emitting device.

In an exemplary embodiment of the present application, the organic light emitting device may be a green organic light emitting device, and the heterocyclic compound represented by Chemical Formula 1 may be used as a material for the green organic light emitting device.

In an exemplary embodiment of the present application, the organic light emitting device may be a red organic light emitting device, and the heterocyclic compound represented by Chemical Formula 1 may be used as a material for the red organic light emitting device.

In an exemplary embodiment of the present application, the organic light emitting device may be a blue organic light emitting device, and the heterocyclic compound according to Chemical Formula 1 may be used as a material for a light emitting layer of the blue organic light emitting device.

In an exemplary embodiment of the present application, the organic light emitting device may be a green organic light emitting device, and the heterocyclic compound represented by Chemical Formula 1 may be used as a material for a light emitting layer of the green organic light emitting device.

In an exemplary embodiment of the present application, the organic light emitting device may be a red organic light emitting device, and the heterocyclic compound represented by Chemical Formula 1 may be used as a material for a light emitting layer of the red organic light emitting device.

In an exemplary embodiment of the present application, the specific content on the heterocyclic compound represented by Chemical Formula 1 is the same as that described above.

The organic light emitting device of the present invention may be manufactured using typical manufacturing methods and materials of an organic light emitting device, except that the above-described heterocyclic compound is used to form an organic material layer having one or more layers.

The heterocyclic compound may be formed as an organic material layer by not only a vacuum deposition method, but also a solution application method when an organic light emitting device is manufactured. Here, the solution application method means spin coating, dip coating, inkjet printing, screen printing, a spray method, roll coating, and the like, but is not limited thereto.

The organic material layer of the organic light emitting device of the present invention may be composed of a single-layered structure, but may be composed of a multilayered structure in which two or more organic material layers are stacked. For example, the organic light emitting device of the present invention may have a structure including a hole injection layer, a hole transport layer, a light emitting layer, an electron transport layer, an electron injection layer, and the like as organic material layers. However, the structure of the organic light emitting device is not limited thereto, and may include a fewer number of organic material layers.

In an exemplary embodiment of the present application, as the iridium-based dopant, Ir(ppy)₃ as a green phosphorescent dopant may be used.

In an exemplary embodiment of the present application, provided is an organic light emitting device, in which the organic material layer of the organic light emitting device includes a light emitting layer, and the light emitting layer includes the heterocyclic compound.

In an exemplary embodiment of the present application, provided is an organic light emitting device, in which the organic material layer of the organic light emitting device includes a light emitting layer, and the light emitting layer includes a host material, and the host material includes the heterocyclic compound.

In the organic light emitting device of the present invention, the organic material layer includes an electron injection layer or an electron transport layer, and the electron injection layer or electron transport layer may include the heterocyclic compound.

In another organic light emitting device, the organic material layer includes an electron blocking layer or a hole blocking layer, and the electron blocking layer or the hole blocking layer may include the heterocyclic compound.

In still another organic light emitting device, the organic material layer includes an electron transport layer, a light emitting layer or a hole blocking layer, and the electron transport layer, the light emitting layer or the hole blocking layer may include the heterocyclic compound.

In the organic light emitting device of the present application, as a positive electrode material, materials having a relatively high work function may be used, and a transparent conductive oxide, a metal or a conductive polymer, and the like may be used. Specific examples of the positive electrode material include: a metal such as vanadium, chromium, copper, zinc, and gold, or an alloy thereof; a metal oxide such as zinc oxide, indium oxide, indium tin oxide (ITO), and indium zinc oxide (IZO); a combination of a metal and an oxide, such as ZnO:Al or SnO₂:Sb; a conductive polymer such as poly(3-methylthiophene), poly[3,4-(ethylene-1,2-dioxy)thiophene] (PEDOT), polypyrrole, and polyaniline; and the like, but are not limited thereto.

As a negative electrode material, materials having a relatively low work function may be used, and a metal, a metal oxide, or a conductive polymer, and the like may be used. Specific examples of the negative electrode material include: a metal such as magnesium, calcium, sodium, potassium, titanium, indium, yttrium, lithium, gadolinium, aluminum, silver, tin, and lead, or an alloy thereof; a multi-layer structured material, such as LiF/Al or LiO₂/Al; and the like, but are not limited thereto.

As a hole injection material, a publicly-known hole injection material may also be used, and it is possible to use, for example, a phthalocyanine compound such as copper phthalocyanine disclosed in US Patent No. 4,356,429 or starburst-type amine derivatives described in the document [Advanced Material, 6, p. 677 (1994)], for example, tris(4-carbazoyl-9-ylphenyl)amine (TCTA), 4,4',4"-tri[phenyl(m-tolyl)amino]triphenylamine (m-MTDATA), 1,3,5-tris[4-(3-methylphenylphenylamino)phenyl]benzene (m-MTDAPB), polyaniline/dodecylbenzenesulfonic acid or poly(3,4-ethylenedioxythiophene)/poly(4-styrenesulfonate), which is a soluble conductive polymer, polyaniline/camphor sulfonic acid or polyaniline/poly(4-styrene-sulfonate), and the like.

As a hole transport material, a pyrazoline derivative, an arylamine-based derivative, a stilbene derivative, a triphenyldiamine derivative, and the like may be used, and a low-molecular weight or polymer material may also be used.

As an electron transport material, it is possible to use an oxadiazole derivative, anthraquinodimethane and a derivative thereof, benzoquinone and a derivative thereof, naphthoquinone and a derivative thereof, anthraquinone and a derivative thereof, tetracyanoanthraquinodimethane and a derivative thereof, a fluorenone derivative, diphenyldicyanoethylene and a derivative thereof, a diphenoquinone derivative, a metal complex of 8-hydroxyquinoline and a derivative thereof, and the like, and a low-molecular weight material and a polymer material may also be used.

As an electron injection material, for example, LiF is representatively used in the art, but the present application is not limited thereto.

As a light emitting material, a red, green, or blue light emitting material may be used, and if necessary, two or more light emitting materials may be mixed and used. In this case, two or more light emitting materials are deposited or used as an individual supply source, or pre-mixed to be deposited and used as one supply source. Further, a fluorescent material may also be used as the light emitting material, but may also be used as a phosphorescent material. As the light emitting material, it is also possible to use alone a material which emits light by combining holes and electrons each injected from a positive electrode and a negative electrode, but materials in which a host material and a dopant material are involved in light emission together may also be used.

When hosts of the light emitting material are mixed and used, the same series of hosts may also be mixed and used, and different series of hosts may also be mixed and used. For example, two or more types of materials selected from n-type host materials or p-type host materials may be used as a host material for a light emitting layer.

The organic light emitting device according to an exemplary embodiment of the present application may be a top emission type, a bottom emission type, or a dual emission type according to the material to be used.

The heterocyclic compound according to an exemplary embodiment of the present application may act even in organic electronic devices including organic solar cells, organic photoconductors, organic transistors, and the like, based on the principle similar to those applied to organic light emitting devices.

The organic light emitting device of the present invention may further include one or two or more layers selected from the group consisting of a light emitting layer, a hole injection layer, a hole transport layer, an electron blocking layer, a hole blocking layer, an electron transport layer and an electron injection layer.

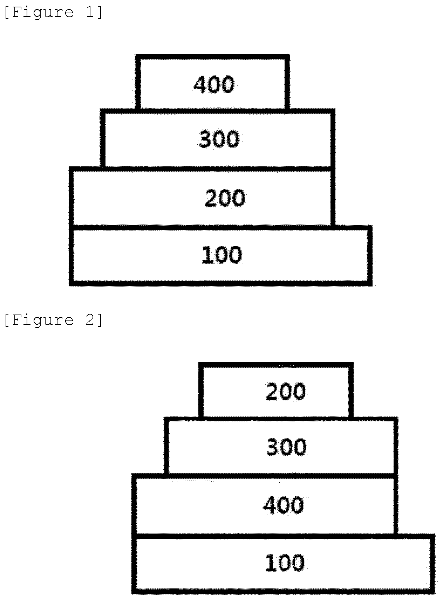

FIGS. 1 to 3 exemplify the stacking sequence of the electrodes and the organic material layer of the organic light emitting device according to an exemplary embodiment of the present application. However, the scope of the present application is not intended to be limited by these drawings, and the structure of the organic light emitting device known in the art may also be applied to the present application.

According to FIG. 1, an organic light emitting device in which a positive electrode 200, an organic material layer 300, and a negative electrode 400 are sequentially stacked on a substrate 100 is illustrated. However, the organic light emitting device is not limited only to such a structure, and as in FIG. 2, an organic light emitting device in which a negative electrode, an organic material layer, and a positive electrode are sequentially stacked on a substrate may also be implemented.

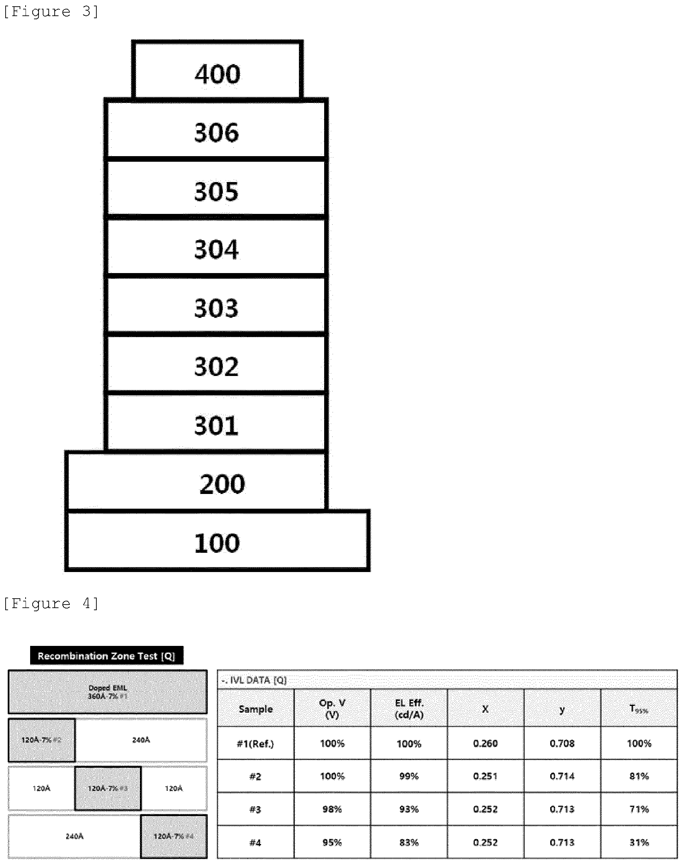

FIG. 3 exemplifies a case where an organic material layer is a multilayer. An organic light emitting device according to FIG. 3 includes a hole injection layer 301, a hole transport layer 302, a light emitting layer 303, a hole blocking layer 304, an electron transport layer 305, and an electron injection layer 306. However, the scope of the present application is not limited by the stacking structure as described above, and if necessary, the other layers except for the light emitting layer may be omitted, and another necessary functional layer may be further added.

In an exemplary embodiment of the present application, provided is an organic light emitting device in which the organic material layer of the organic light emitting device including the heterocyclic compound represented by Chemical Formula 1 further includes a heterocyclic compound represented by the following Chemical Formula 2.

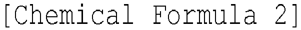

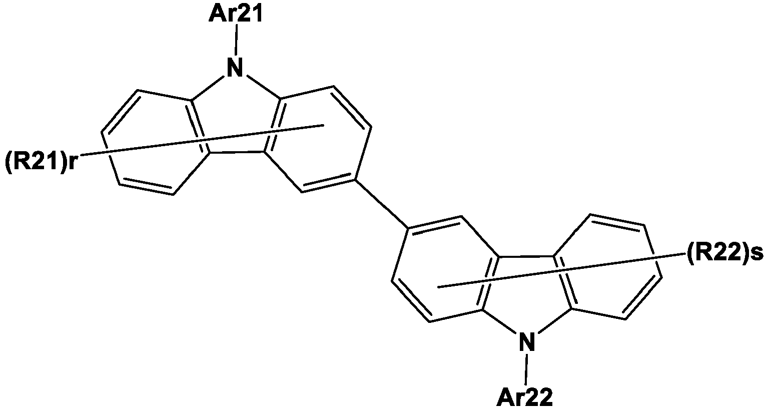

In Chemical Formula 2,
R21 and R22 are the same as or different from each other, and are each independently selected from the group consisting of hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiR'R"R"'; -P(=O)R'R"; and an amine group which is unsubstituted or substituted with a substituted or unsubstituted C1 to C60 alkyl group, a substituted or unsubstituted C6 to C60 aryl group or a substituted or unsubstituted C2 to C60 heteroaryl group, or two or more adjacent groups are bonded to each other to form a substituted or unsubstituted C6 to C60 aliphatic or aromatic hydrocarbon ring or a substituted or unsubstituted C2 to C60 aliphatic or aromatic hetero ring,
R', R" and R"' are the same as or different from each other, and are each independently hydrogen; deuterium; a cyano group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
r and s are an integer from 0 to 7, and when r and s are 2 or higher, substituents in the parenthesis are the same as or different from each other, and
Ar21 and Ar22 are the same as or different from each other, and are each independently a substituted or unsubstituted C6 to C60 aryl group; or a substituted or unsubstituted C2 to C60 heteroaryl group.

In an exemplary embodiment of the present application, R21 and R22 are the same as or different from each other, and are each independently selected from the group consisting of hydrogen; deuterium; a halogen group; a cyano group; a substituted or unsubstituted C1 to C40 alkyl group; a substituted or unsubstituted C2 to C40 alkenyl group; a substituted or unsubstituted C2 to C40 alkynyl group; a substituted or unsubstituted C1 to C40 alkoxy group; a substituted or unsubstituted C3 to C40 cycloalkyl group; a substituted or unsubstituted C2 to C40 heterocycloalkyl group; a substituted or unsubstituted C6 to C40 aryl group; a substituted or unsubstituted C2 to C40 heteroaryl group; -SiR'R"R"'; -P(=O)R'R"; and an amine group which is unsubstituted or substituted with a substituted or unsubstituted C1 to C40 alkyl group, a substituted or unsubstituted C6 to C40 aryl group or a substituted or unsubstituted C2 to C40 heteroaryl group, or two or more adjacent groups may be bonded to each other to form a substituted or unsubstituted C6 to C40 aliphatic or aromatic hydrocarbon ring or a substituted or unsubstituted C2 to C40 aliphatic or aromatic hetero ring.

In another exemplary embodiment, R21 and R22 are the same as or different from each other, and are each independently selected from the group consisting of hydrogen; deuterium; a halogen group; a cyano group; a substituted or unsubstituted C1 to C20 alkyl group; a substituted or unsubstituted C2 to C20 alkenyl group; a substituted or unsubstituted C2 to C20 alkynyl group; a substituted or unsubstituted C1 to C20 alkoxy group; a substituted or unsubstituted C3 to C20 cycloalkyl group; a substituted or unsubstituted C2 to C20 heterocycloalkyl group; a substituted or unsubstituted C6 to C20 aryl group; a substituted or unsubstituted C2 to C20 heteroaryl group; -SiR'R"R"'; -P(=O)R'R"; and an amine group which is unsubstituted or substituted with a substituted or unsubstituted C1 to C20 alkyl group, a substituted or unsubstituted C6 to C20 aryl group or a substituted or unsubstituted C2 to C20 heteroaryl group, or two or more adjacent groups may be bonded to each other to form a substituted or unsubstituted C6 to C20 aliphatic or aromatic hydrocarbon ring or a substituted or unsubstituted C2 to C20 aliphatic or aromatic hetero ring.

In still another exemplary embodiment, R21 and R22 are the same as or different from each other, and are each independently selected from the group consisting of hydrogen; deuterium; a halogen group; a cyano group; a C1 to C20 alkyl group; a C2 to C20 alkenyl group; a C2 to C20 alkynyl group; a C1 to C20 alkoxy group; a C3 to C20 cycloalkyl group; a C2 to C20 heterocycloalkyl group; a C6 to C20 aryl group; a C2 to C20 heteroaryl group; - SiR'R"R"'; -P(=O)R'R"; and an amine group, or two or more adjacent groups may be bonded to each other to form a C6 to C20 aliphatic or aromatic hydrocarbon ring, or a C2 to C20 aliphatic or aromatic hetero ring.

In yet another exemplary embodiment, R21 and R22 are the same as or different from each other, and may be each independently hydrogen; deuterium; a C1 to C20 alkyl group; or a C6 to C20 aryl group; or a C2 to C20 heteroaryl group.

In yet another exemplary embodiment, R21 and R22 are the same as or different from each other, and may be each independently hydrogen; deuterium; or a phenyl group.

In yet another exemplary embodiment, R21 and R22 are the same as or different from each other, and may be each independently hydrogen; or deuterium.

In an exemplary embodiment of the present invention, R', R" and R"' are the same as or different from each other, and may be each independently hydrogen; deuterium; a cyano group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group.

In another exemplary embodiment, R', R" and R"' are the same as or different from each other, and may be each independently hydrogen; deuterium; a cyano group; a substituted or unsubstituted C1 to C40 alkyl group; a substituted or unsubstituted C3 to C40 cycloalkyl group; a substituted or unsubstituted C6 to C40 aryl group; or a substituted or unsubstituted C2 to C40 heteroaryl group.

In still another exemplary embodiment, R', R" and R"' are the same as or different from each other, and may be each independently hydrogen; deuterium; a cyano group; a substituted or unsubstituted C1 to C20 alkyl group; a substituted or unsubstituted C3 to C20 cycloalkyl group; a substituted or unsubstituted C6 to C20 aryl group; or a substituted or unsubstituted C2 to C20 heteroaryl group.

In yet another exemplary embodiment, R', R" and R"' are the same as or different from each other, and may be each independently hydrogen; deuterium; a cyano group; a C1 to C20 alkyl group; a C3 to C20 cycloalkyl group; a C6 to C20 aryl group; or a C2 to C20 heteroaryl group.

In yet another exemplary embodiment, R', R" and R"' are the same as or different from each other, and may be each independently hydrogen; deuterium; or a phenyl group.

In an exemplary embodiment of the present invention, Ar21 and Ar22 are the same as or different from each other, and may be each independently a substituted or unsubstituted C6 to C20 aryl group; or a substituted or unsubstituted C2 to C60 heteroaryl group.

In another exemplary embodiment, Ar21 and Ar22 are the same as or different from each other, and may be each independently a substituted or unsubstituted C6 to C40 aryl group; or a substituted or unsubstituted C2 to C40 heteroaryl group.

In still another exemplary embodiment, Ar21 and Ar22 are the same as or different from each other, and may be each independently a substituted or unsubstituted C6 to C20 aryl group; or a substituted or unsubstituted C2 to C20 heteroaryl group.

In yet another exemplary embodiment, Ar21 and Ar22 are the same as or different from each other, and may be each independently a substituted or unsubstituted phenyl group; a substituted or unsubstituted biphenyl group; a substituted or unsubstituted terphenyl group; a substituted or unsubstituted triphenylene group; a substituted or unsubstituted dimethylfluorene group; a substituted or unsubstituted dibenzofuran group; or a substituted or unsubstituted dibenzothiophene group.

In yet another exemplary embodiment, Ar21 and Ar22 are the same as or different from each other, and may be each independently a phenyl which is unsubstituted or substituted with deuterium; a biphenyl group which is unsubstituted or substituted with deuterium; a terphenyl group which is unsubstituted or substituted with deuterium; a triphenylene group which is unsubstituted or substituted with deuterium; a dimethylfluorene group which is unsubstituted or substituted with deuterium; a dibenzofuran group which is unsubstituted or substituted with deuterium; or a dibenzothiophene group which is unsubstituted or substituted with deuterium.

In an exemplary embodiment of the present invention, the deuterium content of the heterocyclic compound represented by Chemical Formula 2 may be 0% or more and 100% or less.

In another exemplary embodiment, the deuterium content of the heterocyclic compound represented by Chemical Formula 2 may be more than 0% and 100% or less.

In still another exemplary embodiment, the deuterium content of the heterocyclic compound represented by Chemical Formula 2 may be 0%, or 5% or more and 100% or less.

In yet another exemplary embodiment, the deuterium content of the heterocyclic compound represented by Chemical Formula 2 may be 0%, or 10% or more and 100% or less.

In yet another exemplary embodiment, the deuterium content of the heterocyclic compound represented by Chemical Formula 2 may be 0%, or 20% or more and 100% or less.

In yet another exemplary embodiment, the deuterium content of the heterocyclic compound represented by Chemical Formula 2 may be 0%, or 40% or more and 100% or less.

In yet another exemplary embodiment, the deuterium content of the heterocyclic compound represented by Chemical Formula 2 may be 0%, or 60% or more and 100% or less.

In yet another exemplary embodiment, the deuterium content of the heterocyclic compound represented by Chemical Formula 2 may be 0%, or 80% or more and 100% or less.

In yet another exemplary embodiment, the deuterium content of the heterocyclic compound represented by Chemical Formula 2 may be 0%, or 100%.

In an exemplary embodiment of the present invention, provided is an organic light emitting device in which Chemical Formula 2 is represented by any one of the following compounds.

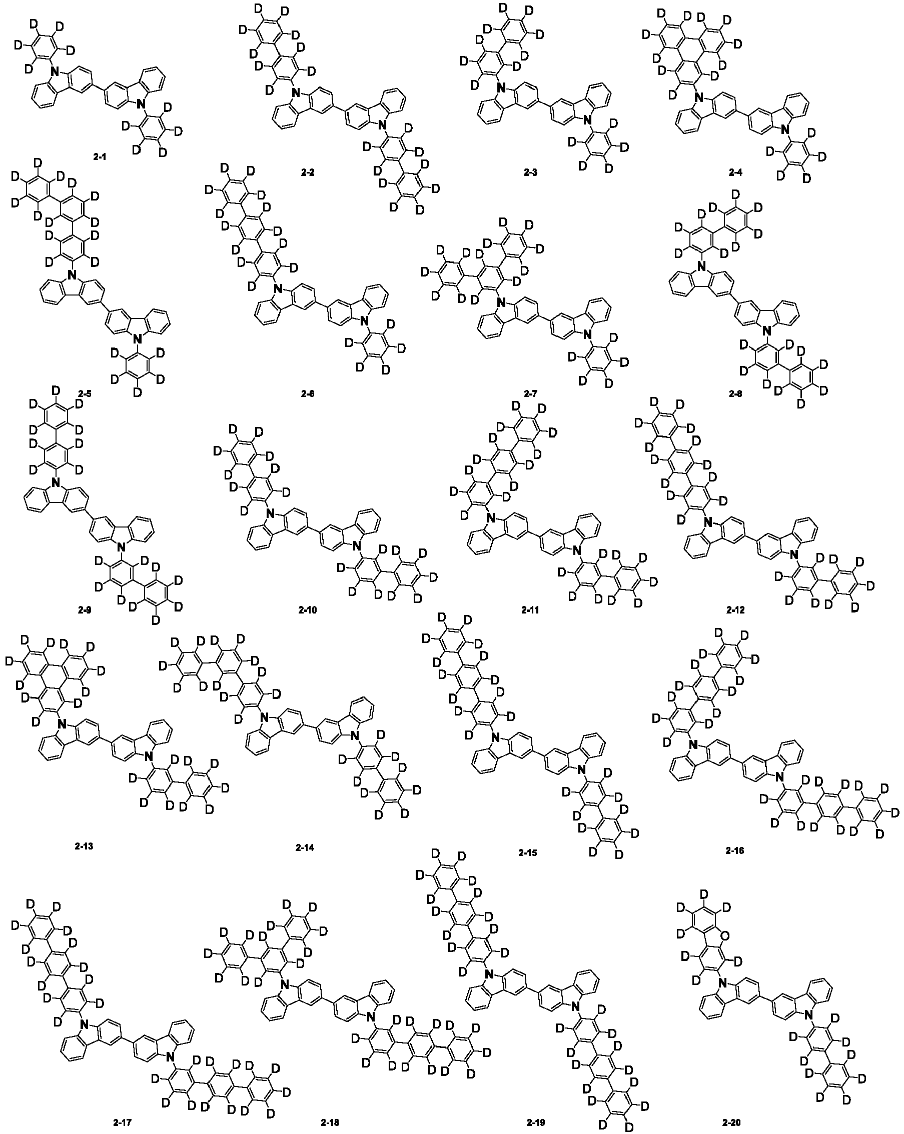

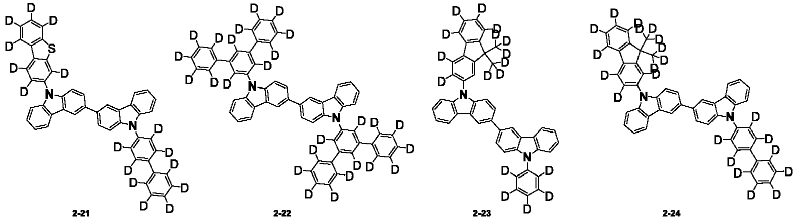

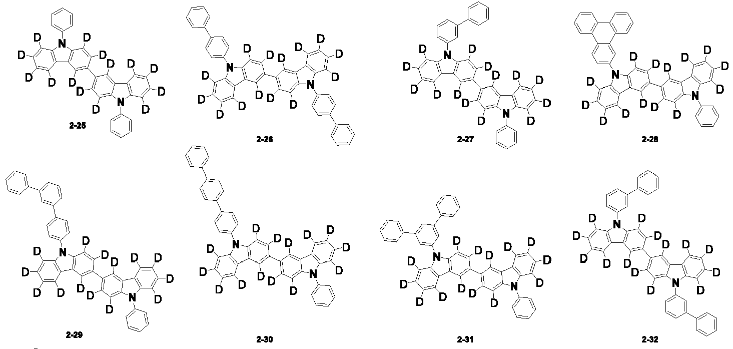

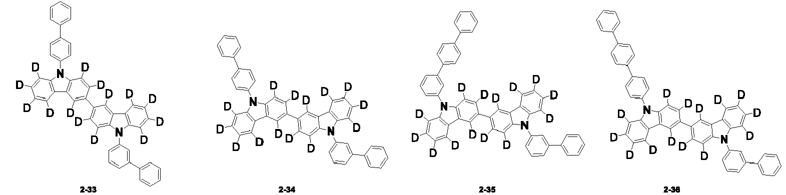

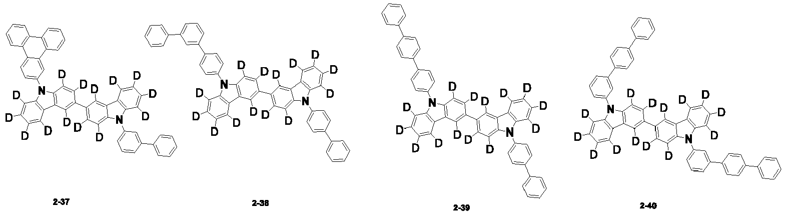

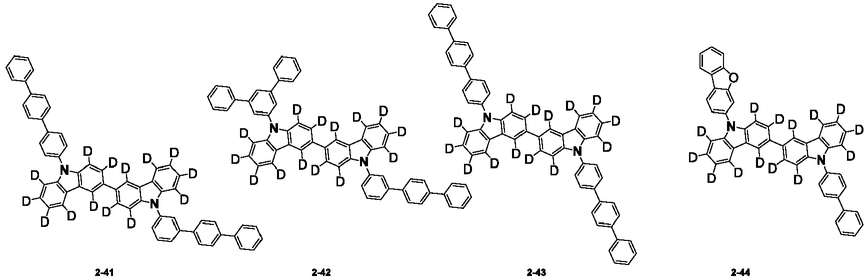

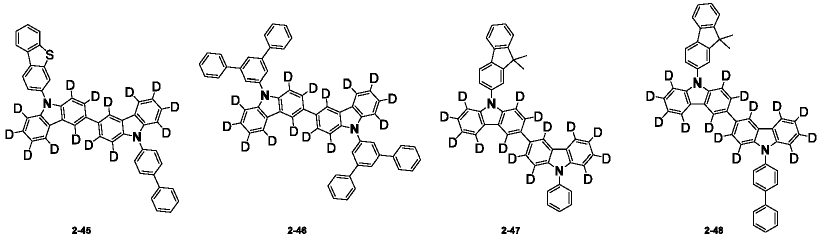

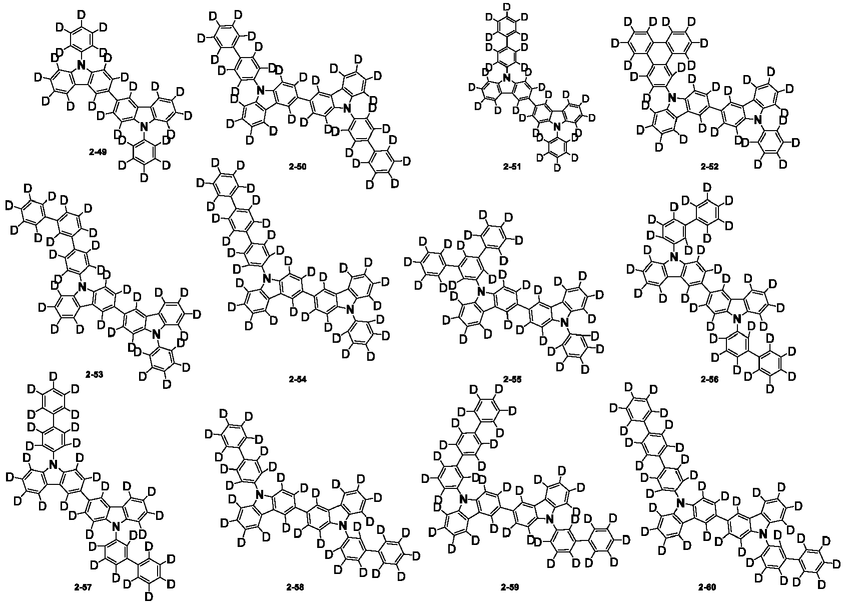

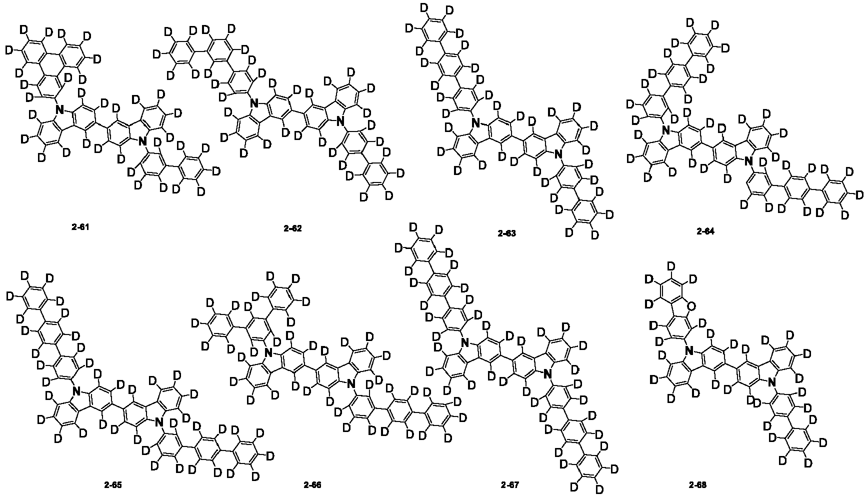

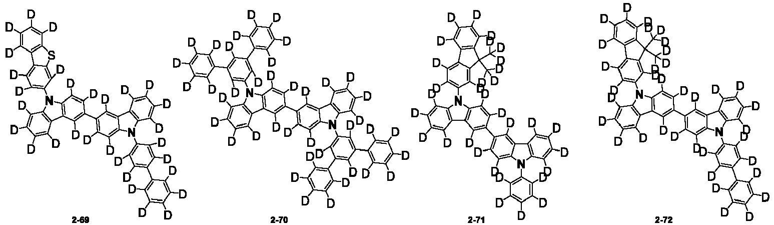

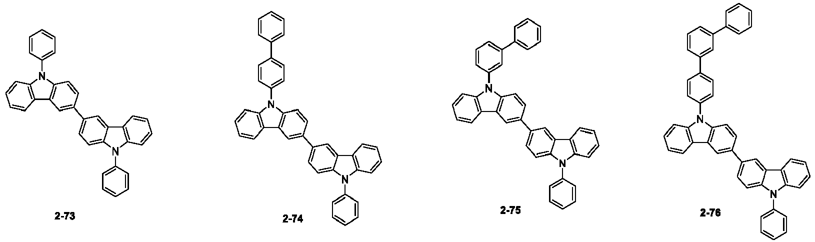

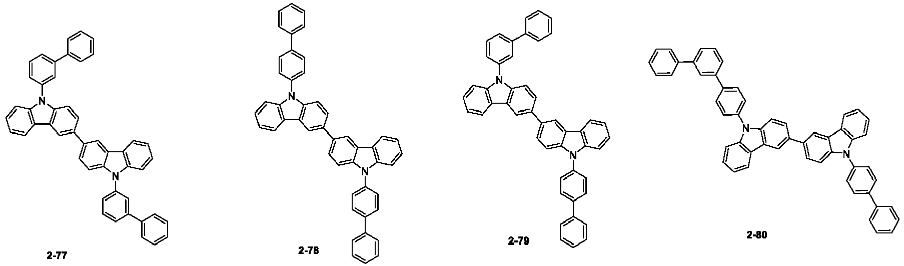

A content on the organic light emitting device including the heterocyclic compound represented by Chemical Formula 1 may be applied to the organic light emitting device further including the heterocyclic compound represented by Chemical Formula 2.

In another exemplary embodiment, the heterocyclic compound represented by Chemical Formula 2 may be used as a light emitting material for a light emitting layer of an organic light emitting device, and may be used as a phosphorescent green light emitting material.

In yet another exemplary embodiment, the heterocyclic compound represented by Chemical Formula 2 may be used as a light emitting material for a light emitting layer of an organic light emitting device, and may be used as a p-host material.

In the organic light emitting device of the present invention, the organic material layer includes a light emitting layer, the light emitting layer includes a host material, and the host material may include the heterocyclic compound represented by Chemical Formula 1 and the heterocyclic compound represented by Chemical Formula 2.

In another organic light emitting device, the organic material layer includes a light emitting layer, includes the heterocyclic compound represented by Chemical Formula 1 as an n-host material for the light emitting layer, and may include the heterocyclic compound represented by Chemical Formula 2 as a p-host material.

In an exemplary embodiment of the present application, provided is a composition for an organic material layer of an organic light emitting device, which includes the heterocyclic compound represented by Chemical Formula 1 and the heterocyclic compound represented by Chemical Formula 2.

The weight ratio of the heterocyclic compound represented by Chemical Formula 1 : the heterocyclic compound represented by Chemical Formula 2 in the composition may be 1 : 10 to 10 : 1, 1 : 8 to 8 : 1, 1 : 5 to 5 : 1, and 1 : 2 to 2 : 1, but is not limited thereto.

The composition may be used when an organic material layer of an organic light emitting device is formed, and particularly, may be more preferably used as a host material for the light emitting layer.

The composition is in a form in which two or more compounds are simply mixed, materials in a powder state may also be mixed before an organic material layer of an organic light emitting device is formed, and it is possible to mix compounds in a liquid state at a temperature which is equal to or more than a suitable temperature. The composition is in a solid state at a temperature which is equal to or less than the melting point of each material, and may be maintained as a liquid phase when the temperature is adjusted.

The composition may additionally include materials publicly known in the art such as solvents and additives.

The organic light emitting device according to an exemplary embodiment of the present application may be manufactured by typical methods and materials for manufacturing an organic light emitting device, except that one or more organic material layers are formed by using the heterocyclic compound represented by Chemical Formula 1, or one or more organic material layers are formed by using the heterocyclic compound represented by Chemical Formula 1 and the heterocyclic compound represented by Chemical Formula 2.

In an exemplary embodiment of the present application, provided is a method for manufacturing an organic light emitting device, the method including: preparing a substrate; forming a first electrode on the substrate; forming an organic material layer having one or more layers on the first electrode; and forming a second electrode on the organic material layer, in which the forming of the organic material layer includes forming the organic material layer having one or more layers by using the composition for an organic material layer according to an exemplary embodiment of the present application.

In an exemplary embodiment of the present application, provided is a method for manufacturing an organic light emitting device, in which the forming of the organic material layer forms the organic material layer by pre-mixing one heterocyclic compound represented by Chemical Formula 1 and one heterocyclic compound represented by Chemical Formula 2, and using a thermal vacuum deposition method.

The pre-mixing means that before the heterocyclic compound represented by Chemical Formula 1 and the heterocyclic compound represented by Chemical Formula 2 are deposited onto an organic material layer, the materials are first mixed and the mixture is contained in one common container and mixed.

The pre-mixed material may be referred to as a composition for an organic material layer according to an exemplary embodiment of the present application.

Hereinafter, the present specification will be described in more detail through Examples, but these Examples are provided only for exemplifying the present application, and are not intended to limit the scope of the present application.

### [Preparation Examples]

### <Preparation Example 1> Preparation of Compound 5

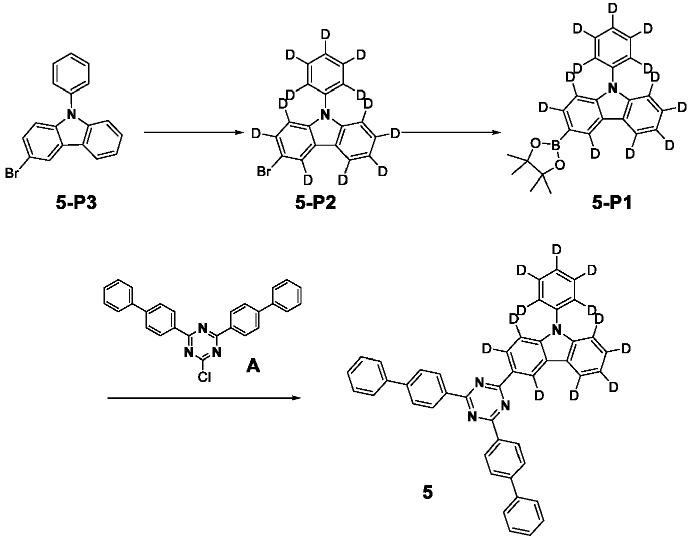

### 1) Preparation of Compound 5-P2

Reaction compounds and reaction conditions are shown in the following Table 1.

**[Table 1]**

| Experimental Example | Compound (g, eq.) | Solvent (g, eq.) | Acid (g, eq.) | Time, temperature | obtained amount, yield |
|---|---|---|---|---|---|
| 1 | 5-P3 (1 g, 1 eq.) | Benzene-D6 (100 g, 383.3 eq.) | CF₃SO₃H (23 g, 50 eq.) | 80°C, 1 h | 0.68g, 66% |
| 2 | 5-P3 (1 g, 1 eq.) | Benzene-D6 (100 g, 383.3 eq.) | CF₃SO₃H (23 g, 50 eq.) | RT., 5 h | 0.5g, 48% |
| 3 | 5-P3 (1 g, 1 eq.) | Benzene-D6 (100g, 383.3 eq.) | CF₃SO₃H (23 g, 50 eq.) | 50°C, 1 h | 0.65g, 62% |
| 4 | 5-P3 (1 g, 1 eq.) | Benzene-D6 (50 g, 191.6 eq.) | CF₃SO₃H (11.5 g, 25 eq.) | 80°C, 1 h | 0.71g, 72% |
| 5 | 5-P3 (1 g, 1 eq.) | Benzene-D6 (50 g, 191.6 eq.) | CF₃SO₃H (11.5 g, 25 eq.) | RT., 1h | 0.55 g, 53% |
| 6 | 5-P3 (1 g, 1 eq.). | Benzene-D6 (50 g, 191.6 eq.) | CF₃SO₃D (11.5 g, 25 eq.) | 50°C, 1 h | 0.68g, 66% |
| 7 | 5-P3 (1 g, 1 eq.) | DMSO-D6 (50 g, 191.6 eq.) | CF₃SO₃H (11.5 g, 25 eq.) | 50°C, 1 h | 0.5g, 48% |
| 8 | 5-P3 (1 g, 1 eq.) | DMF-D6 (50 g, 185.1 eq.) | CF₃SO₃H (11.5, 25 eq.) | 50°C, 1 h | 0.5 g, 480 |

In Table 1, Compound 5-P2 was synthesized under the No. 4 reaction conditions of the Experimental Example, which had the highest yield.

20 g (62.07 mmol) of Compound 5-P3 was dissolved in 1000 g of benzene-D6 and 230 g of CF₃SO₃H, and the resulting solution was stirred at 80°C for 1 hour. After the reaction was completed, the reaction product was quenched with Na₂CO₃ in D₂O. After quenching, ethyl acetate was added to the mixed solution and dissolved, then the organic layer was separated and dried over anhydrous MgSO₄, and then the solvent was removed using a rotary evaporator. Thereafter, purification was performed by column chromatography using dichloromethane and hexane as eluting solvents, thereby obtaining 14.9 g (yield 72%) of Compound 5-P2.

### 2) Preparation of Compound 5-P1

After 14.9 g (44.69 mmol) of Compound 5-P2 and 14.72 g (57.97 mmol) of 4,4,4',4',5,5,5',5'-octamethyl-2,2'-bi(1,3,2-dioxaborolane) were dissolved in 120 mL of 1,4-dioxane, 1.63 g (2.23 mmol) of Pd(dppf)Cl₂ and 13.14 g (134.07 mmol) of potassium acetate were added thereto, and the resulting mixture was stirred under reflux for 16 hours. After the reaction was completed, ethyl acetate was added to the reaction solution for dissolution, and then the resulting solution was extracted with distilled water, the organic layer was dried over anhydrous MgSO₄, and then the solvent was removed using a rotary evaporator, and then the residue was purified by column chromatography using dichloromethane and hexane as eluting solvents, thereby obtaining 13.6 g (yield = 80%) of Compound 5-P1.

### 3) Preparation of Compound 5

After 13.6 g (35.75 mmol) of Compound 5-P1 and 15.01 g (18.49 mmol) of 2,4-di([1,1'-biphenyl]-4-yl)-6-chloro-1,3,5-triazine were dissolved in 150 mL of 1,4-dioxane and 30 mL of distilled water, 2.06 g (1.79 mmol) of Pd(PPh₃)₄ and 9.88 g (71.5 mmol) of K₂CO₃ were added thereto, and the resulting mixture was stirred under reflux for 6 hours. After the reaction was completed, dichloromethane was added to the reaction solution for dissolution, and then the resulting solution was extracted with distilled water, the organic layer was dried over anhydrous MgSO₄, and then the solvent was removed using a rotary evaporator, and then the residue was purified by column chromatography using dichloromethane and hexane as eluting solvents, thereby obtaining 15.99 g (yield = 70%) of Compound 5.

Target compounds in the following Table 2 were synthesized by performing the preparation in the same manner as in Preparation Example 1, except that Intermediate 1 in the following Table 2 was used instead of Compound 5-P3, and Intermediate 2 in the following Table 2 was used instead of Compound A.

**[Table 2]**

| Compound No. | Intermediate 1 | Intermediate 2 | Target compound | yield |
|---|---|---|---|---|
| 23 | 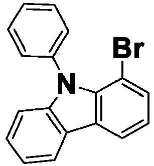 | 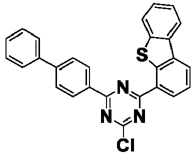 | 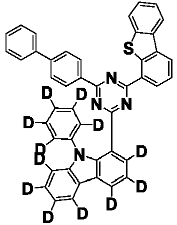 | 70% |
| 27 | 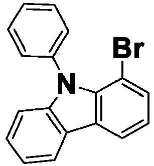 | 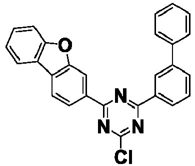 | 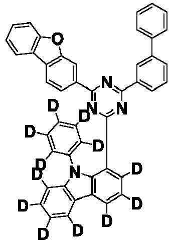 | 69% |
| 62 | 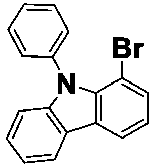 | 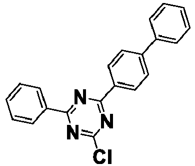 | 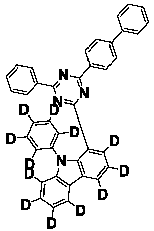 | 68% |
| 101 | 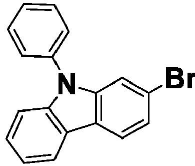 | 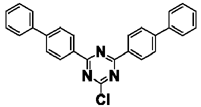 | 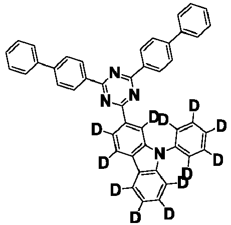 | 70% |
| 14 | 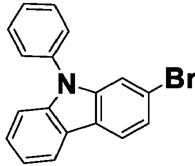 | 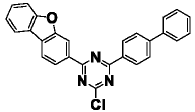 | 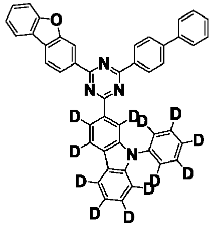 | 68% |
| 15 | 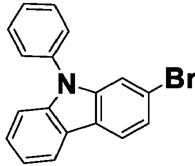 | 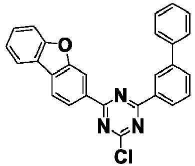 | 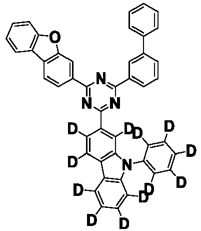 | 69% |
| 19 | 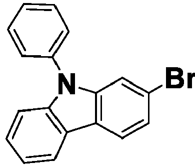 | 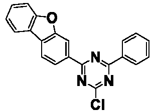 | 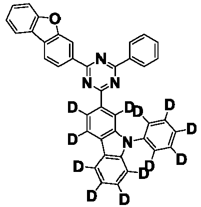 | 70% |
| 21 | 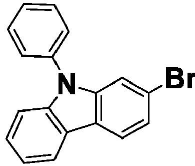 | 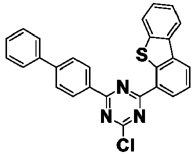 | 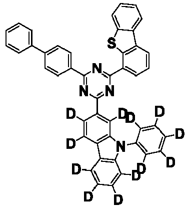 | 69% |
| 46 | 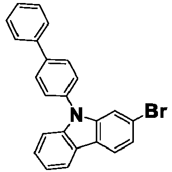 | 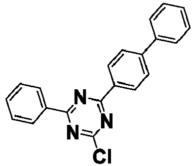 | 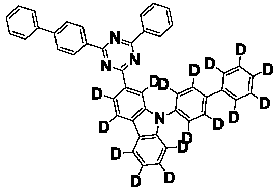 | 67% |
| 47 | 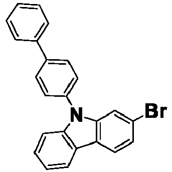 | 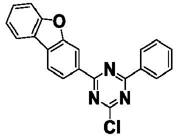 | 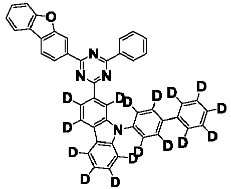 | 66% |
| 66 | 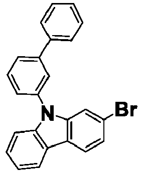 | 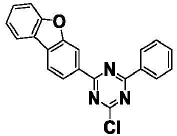 | 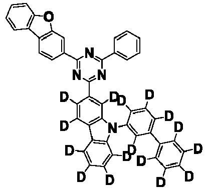 | 70% |
| 107 | 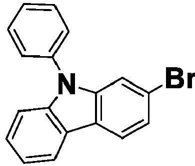 | 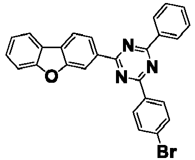 | 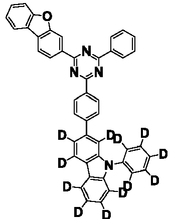 | 69% |
| 108 | 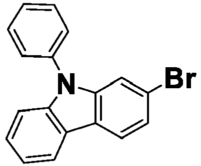 | 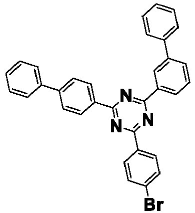 | 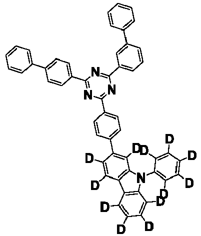 | 67% |
| 116 | 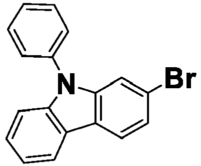 | 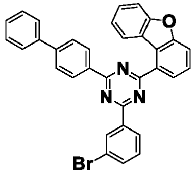 | 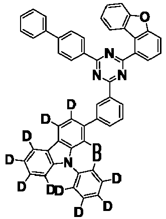 | 71% |
| 117 | 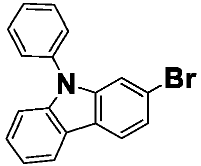 | 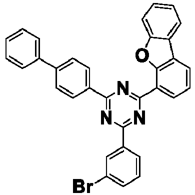 | 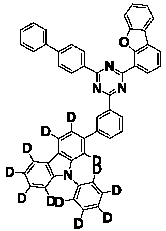 | 70% |
| 119 | 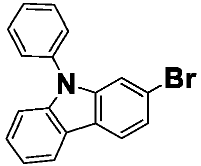 | 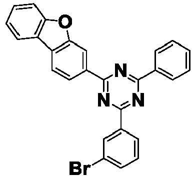 | 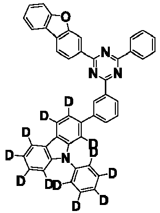 | 68% |
| 120 | 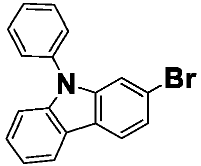 | 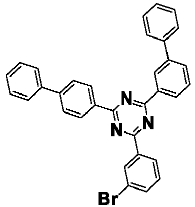 | 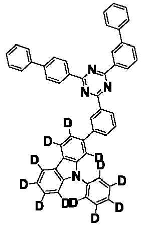 | 69% |
| 11 | 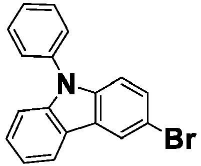 | 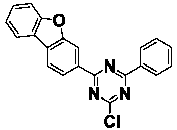 | 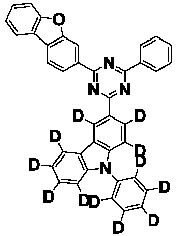 | 67% |
| 37 | 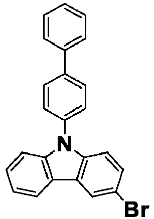 | 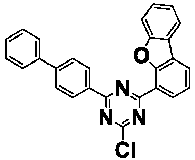 | 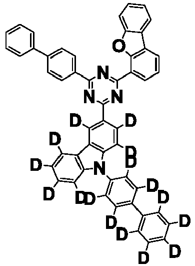 | 69% |
| 38 | 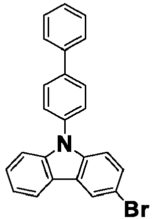 | 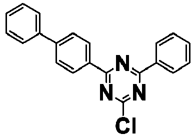 | 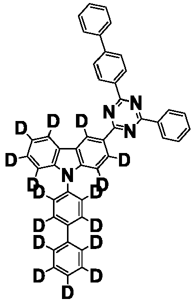 | 70% |
| 63 | 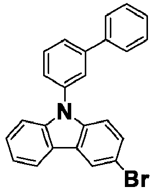 | 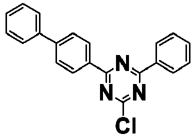 | 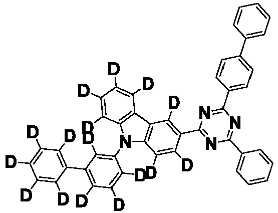 | 70% |
| 86 | 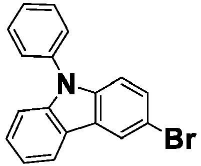 | 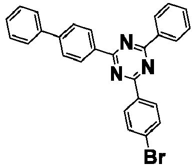 | 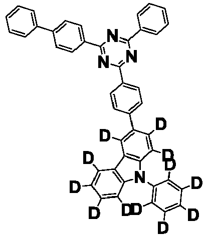 | 68% |
| 95 | 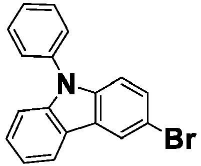 | 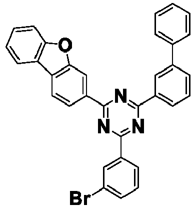 | 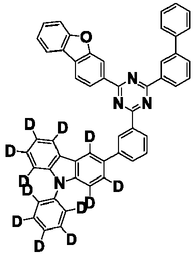 | 67% |
| 96 | 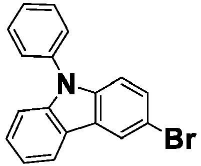 | 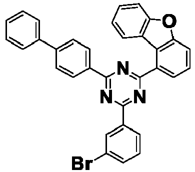 | 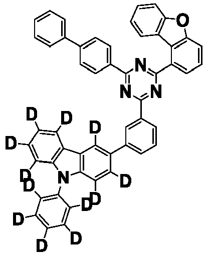 | 69% |
| 97 | 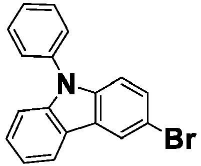 | 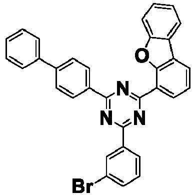 | 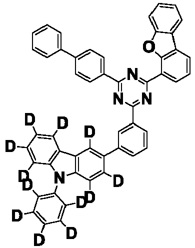 | 70% |
| 99 | 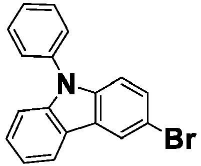 | 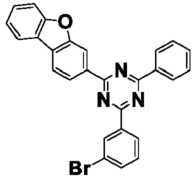 | 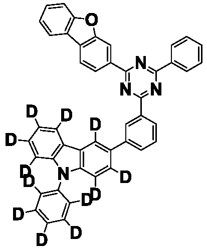 | 68% |
| 100 | 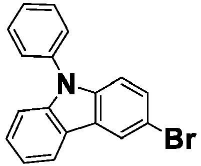 | 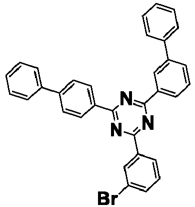 | 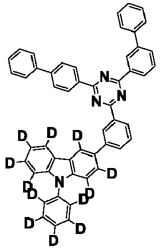 | 70% |
| 134 | 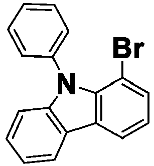 | 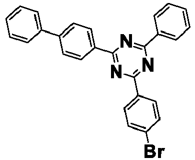 | 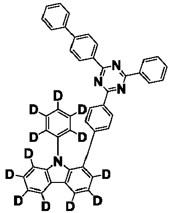 | 68% |
| 143 | 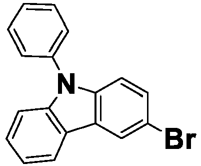 | 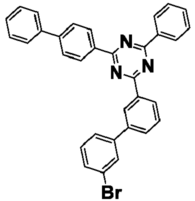 | 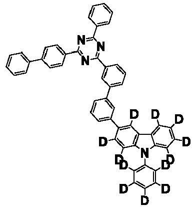 | 72% |
| 160 | 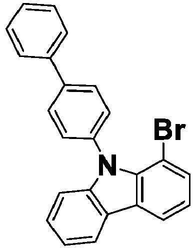 | 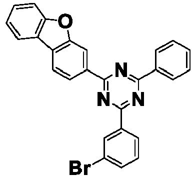 | 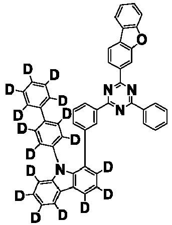 | 69% |
| 263 | 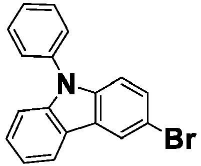 | 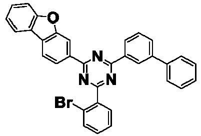 | 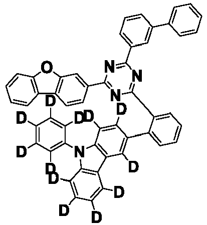 | 67% |
| 287 | 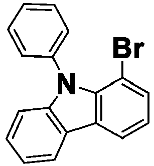 | 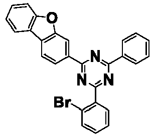 | 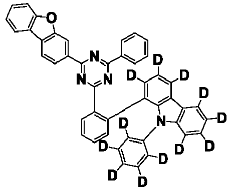 | 68% |

### <Preparation Example 2> Preparation of Compound 79

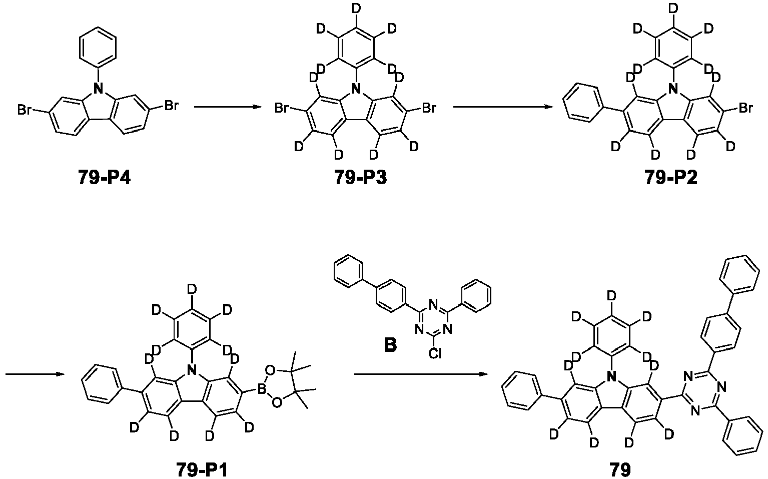

### 1) Preparation of Compound 79-P3

20 g (49.86 mmol) of Compound 79-P4 was dissolved in 804 g of benzene-D6 and 187 g of CF₃SO₃H, and the resulting solution was stirred at 80°C for 1 hour. After the reaction was completed, the reaction product was quenched with Na₂CO₃ in D₂O. After quenching, ethyl acetate was added to the mixed solution and dissolved, then the organic layer was separated and dried over anhydrous MgSO₄, and then the solvent was removed using a rotary evaporator. Thereafter, purification was performed by column chromatography using dichloromethane and hexane as eluting solvents, thereby obtaining 14.8 g (yield 72%) of Compound 79-P3.

### 2) Preparation of Compound 79-P2

After 14.8 g (35.90 mmol) of Compound 79-P3 and 4.38 g (35.90 mmol) of phenylboronic acid were dissolved in 150 mL of 1,4-dioxane and 30 mL of distilled water, 2.06 g (1.79 mmol) of Pd(PPh₃)₄ and 9.88 g (71.5 mmol) of K₂CO₃ were added thereto, and the resulting mixture was stirred under reflux for 5 hours. After the reaction was completed, dichloromethane was added to the reaction solution for dissolution, and then the resulting solution was extracted with distilled water, the organic layer was dried over anhydrous MgSO₄, and then the solvent was removed using a rotary evaporator, and then the residue was purified by column chromatography using dichloromethane and hexane as eluting solvents, thereby obtaining 8.82 g (yield = 65%) of Compound 79-P2.

### 3) Preparation of Compound 79-P1

After 8.82 g (23.34 mmol) of Compound 79-P2 and 8.88 g (35.00 mmol) of 4,4,4',4',5,5,5',5'-octamethyl-2,2'-bi(1,3,2-dioxaborolane) were dissolved in 120 mL of 1,4-dioxane, 0.85 g (1.17 mmol) of Pd(dppf)Cl₂ and 6.57 g (67.04 mmol) of potassium acetate were added thereto, and the resulting mixture was stirred under reflux for 6 hours. After the reaction was completed, ethyl acetate was added to the reaction solution for dissolution, and then the resulting solution was extracted with distilled water, the organic layer was dried over anhydrous MgSO₄, and then the solvent was removed using a rotary evaporator, and then the residue was purified by column chromatography using dichloromethane and hexane as eluting solvents, thereby obtaining 8.53 g (yield = 80%) of Compound 79-P1.

### 4) Preparation of Compound 79

After 8.53 g (18.70 mmol) of Compound 79-P1 and 6.43 g (18.70 mmol) of 2-([1,1'-biphenyl]-4-yl)-4-chloro-6-phenyl-1,3,5-triazine were dissolved in 100 mL of 1,4-dioxane and 20 mL of distilled water, 1.08 g (0.94 mmol) of Pd(PPh₃)₄ and 5.17 g (37.4 mmol) of K₂CO₃ were added thereto, and the resulting mixture was stirred under reflux for 5 hours. After the reaction was completed, dichloromethane was added to the reaction solution for dissolution, and then the resulting solution was extracted with distilled water, the organic layer was dried over anhydrous MgSO₄, and then the solvent was removed using a rotary evaporator, and then the residue was purified by column chromatography using dichloromethane and hexane as eluting solvents, thereby obtaining 8.35 g (yield = 70%) of Compound 79.

A target compound in the following Table 3 was synthesized by performing the preparation in the same manner as in Preparation Example 2, except that Intermediate 3 in the following Table 3 was used instead of Compound B.

**[Table 3]**

| Compound No. | Intermediate 3 | Target compound | yield |
|---|---|---|---|
| 80 | 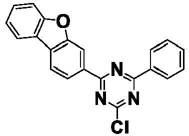 | 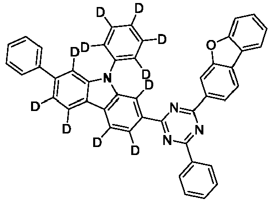 | 68% |

### <Preparation Example 3> Preparation of Compound 2-79

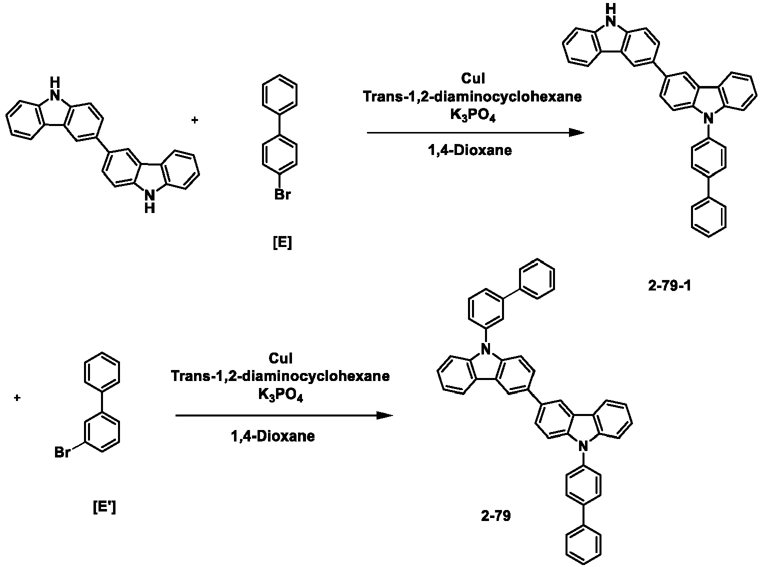

### 1) Preparation of Intermediate 2-79-1

After 9H,9'H-3,3'-bicarbazole (10 g, 0.030 mol), 4-bromo-1,1'-biphenyl (Compound E) (7.26 g, 0.030 mol), CuI (0.57 g, 0.003 mol), trans-1,2-diaminocyclohexane (0.34 g, 0.003 mol), and K₃PO₄ (12.74 g, 0.06 mol) were dissolved in 100 mL of 1,4-dioxane in a one-neck round bottom flask, the resulting solution was refluxed at 125°C for 8 hours. After the reaction was completed, distilled water and DCM were added thereto at room temperature, extraction was performed, the organic layer was dried over MgSO₄, and then the solvent was removed using a rotary evaporator. The reaction product was purified by column chromatography (DCM:hexane = 1:3) and recrystallized with methanol to obtain Intermediate 2-79-1. (13.92 g, yield 94%)

### 2) Preparation of Compound 2-79

After Intermediate 2-79-1 (13.92 g, 0.028 mol), 3-bromo-1,1'-biphenyl (Compound E') (6.83 g, 0.028 mol), CuI (0.53 g, 0.0028 mol), trans-1,2-diaminocyclohexane (0.32 g, 0.0028 mol), and K₃PO₄ (11.89 g, 0.056 mol) were dissolved in 140 mL of 1,4-dioxane in a one-neck round bottom flask, the resulting solution was refluxed at 125°C for 8 hours. After the reaction was completed, distilled water and DCM were added thereto at room temperature, extraction was performed, the organic layer was dried over MgSO₄, and then the solvent was removed using a rotary evaporator. The reaction product was purified by column chromatography (DCM:hexane = 1:3) and recrystallized with methanol to obtain Target Compound 2-79. (16.14 g, yield 88%)

When Compound E and Compound E' are the same, the target compound may be immediately synthesized by adding 2 equivalents of Compound E in 1) of Preparation Example 3. That is, when Compound E and Compound E' are the same, the aforementioned 2) of Preparation Example 3 may be omitted.

The following Target Compound G1 was synthesized by performing the synthesis in the same manner as in Preparation Example 3, except that Compounds E1 and E'1 in the following Table 4 were used instead of 4-bromo-1,1'-biphenyl (Compound E) and 3-bromo-1,1'-biphenyl (Compound E') in Preparation Example 3.

**[Table 4]**

| Compound | Compound E1 | Compound E'1 | Compound G1 | Yield |
|---|---|---|---|---|
| 2-76 | 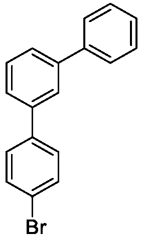 | 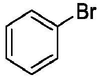 | 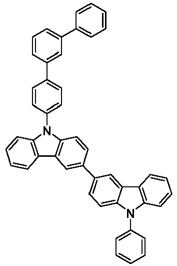 | 72% |
| 2-77 | 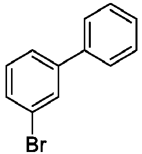 | 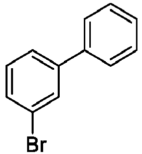 | 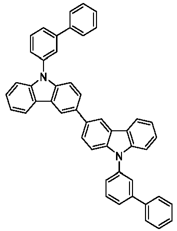 | 83% |
| 2-78 | 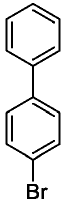 | 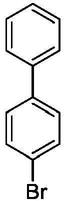 | 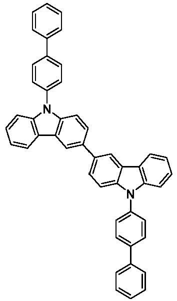 | 88% |
| 2-74 | 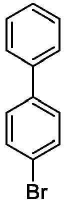 | 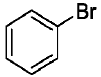 | 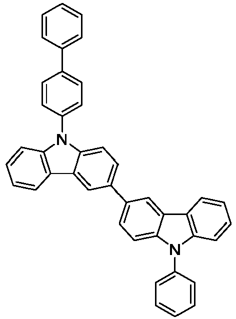 | 73% |

### <Preparation Example 4> Preparation of Compound 2-57

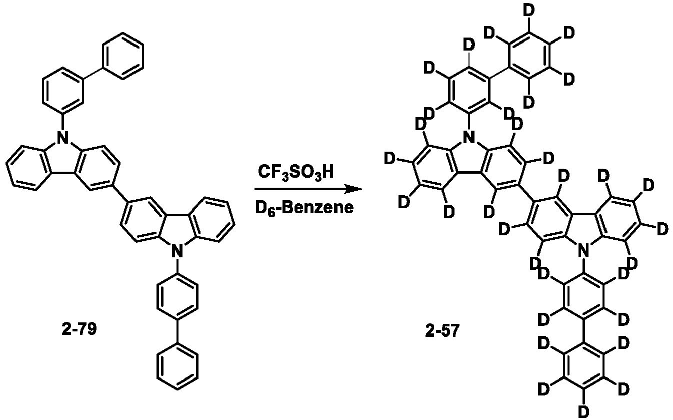

### 1) Preparation of Compound 2-57

A mixture of Intermediate 2-79 (12.17 g, 0.017 mol), 51.5 g of triflic acid and D₆-benzene (608.5 mL) was stirred in a one-neck round bottom flask at 50°C for 1 hour. After the reaction was completed, the reaction product was quenched with Na₂CO₃ in D₂O. After quenching, DCM was added to the mixed solution and dissolved, then the organic layer was separated and dried over anhydrous MgSO₄, and then the solvent was removed using a rotary evaporator. Thereafter, purification was performed by column chromatography using dichloromethane and hexane as eluting solvents, thereby obtaining Target Compound 2-57. (8.01 g, yield 70%)

The following Target Compound G2 was synthesized by performing the synthesis in the same manner as in Preparation Example 4, except that Compound E3 in the following Table 5 was used instead of Compound 2-79 in Preparation Example 4.

**[Table 5]**

| Compound | Compound E3 | Compound G3 | Yield |
|---|---|---|---|
| 2-51 | 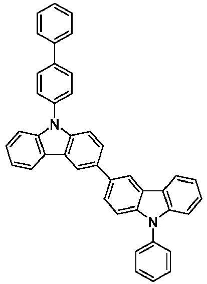 | 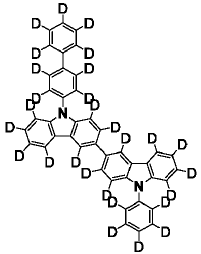 | 68% |
| 2-53 | 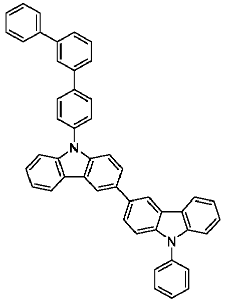 | 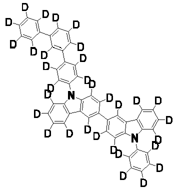 | 70% |
| 2-56 | 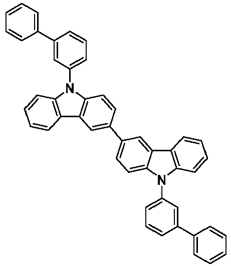 | 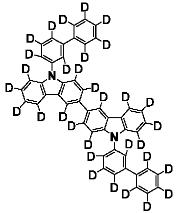 | 69% |
| 2-50 | 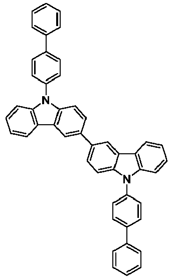 | 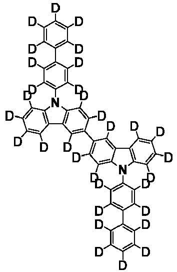 | 68% |
| 228 | 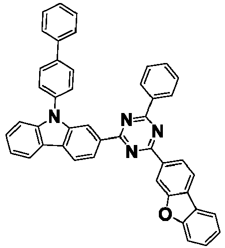 | 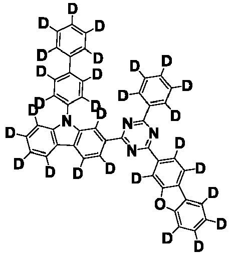 | 67% |
| 230 | 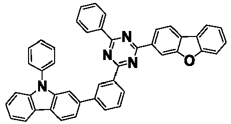 | 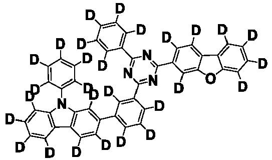 | 67% |

### <Preparation Example 5> Preparation of Compound 185

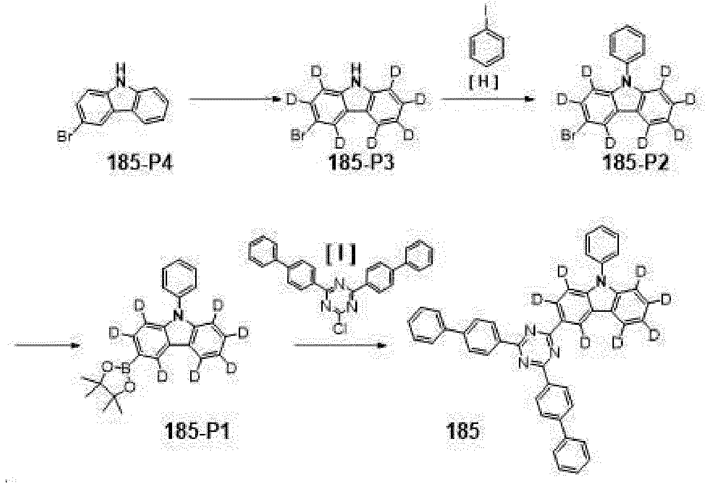

### 1) Preparation of Compound 185-P3

20 g (81.26 mmol) of Compound 185-P4 was dissolved in 653 g of benzene-d6 and 152 g of CF₃SO₃H, and the resulting solution was stirred at 80°C for 1 hour. After the reaction was completed, the reaction product was quenched with Na₂CO₃ in D₂O. After quenching, ethyl acetate was added to the mixed solution and dissolved, then the organic layer was separated and dried over anhydrous MgSO₄, and then the solvent was removed using a rotary evaporator. Thereafter, purification was performed by column chromatography using dichloromethane and hexane as eluting solvents, thereby obtaining 14.4g (yield 70%) of Compound 185-P3.

### 2) Preparation of Compound 185-P2

14.4 g (56.88 mmol) of Compound 185-P3 and 15.7 g (113.76 mmol) of K₂CO₃ were dissolved in 250 mL of iodobenzene, and 0.54 g (2.84 mmol) of CuI was injected thereinto, and then the resulting mixture was stirred at 120°C for 16 hours. After the reaction was completed, dichloromethane was added to the reaction solution for dissolution, and then the resulting solution was extracted with distilled water, the organic layer was dried over anhydrous MgSO₄, and then the solvent was removed using a rotary evaporator, and then the residue was purified by column chromatography using dichloromethane and hexane as eluting solvents, thereby obtaining 11.23 g (yield = 60%) of Compound 185-P2.

### 3) Preparation of Compound 185-P1

After 11.23 g (34.13 mmol) of Compound 185-P2 and 13 g (51.19 mmol) of 4,4,4',4',5,5,5',5'-octamethyl-2,2'-bi(1,3,2-dioxaborolane) were dissolved in 110 mL of 1,4-dioxane, 1.47 g (2.01 mmol) of Pd(dppf)Cl₂ and 11.83 g (120.66 mmol) of potassium acetate were added thereto, and the resulting mixture was stirred under reflux for 16 hours. After the reaction was completed, ethyl acetate was added to the reaction solution for dissolution, and then the resulting solution was extracted with distilled water, the organic layer was dried over anhydrous MgSO₄, and then the solvent was removed using a rotary evaporator, and then the residue was purified by column chromatography using dichloromethane and hexane as eluting solvents, thereby obtaining 10.14 g (yield = 79%) of Compound 185-P1.

### 4) Preparation of Compound 185

After 10.14 g (26.96 mmol) of Compound 185-P1 and 11.32 g (26.96 mmol) of 2,4-di([1,1'-biphenyl]-4-yl)-6-chloro-1,3,5-triazine were dissolved in 125 mL of 1,4-dioxane and 25 mL of distilled water, 1.54 g (1.34 mmol) of Pd(PPh₃)₄ and 7.41 g (53.62 mmol) of K₂CO₃ were added thereto, and the resulting mixture was stirred under reflux for 6 hours. After the reaction was completed, dichloromethane was added to the reaction solution for dissolution, and then the resulting solution was extracted with distilled water, the organic layer was dried over anhydrous MgSO₄, and then the solvent was removed using a rotary evaporator, and then the residue was purified by column chromatography using dichloromethane and hexane as eluting solvents, thereby obtaining 12.13 g (yield = 71%) of Compound 185.

Target compounds in the following Table 6 were synthesized by performing the preparation in the same manner as in Preparation Example 5, except that Intermediate 1 in the following Table 6 was used instead of Compound 185-P4, Intermediate 2 in the following Table 6 was used instead of Compound H, and Intermediate 3 in the following Table 6 was used instead of Compound I.

**[Table 6]**

| Compound No. | Intermediate 1 | Intermediate 2 | Intermediate 3 | Target compound | yield |
|---|---|---|---|---|---|
| 190 | 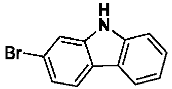 | 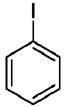 | 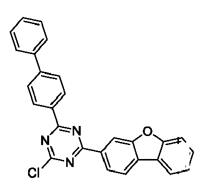 | 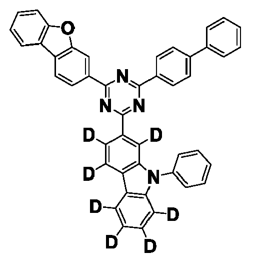 | 70% |
| 200 | 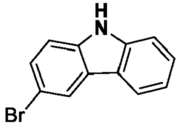 | 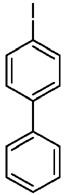 | 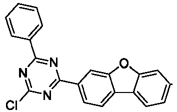 | 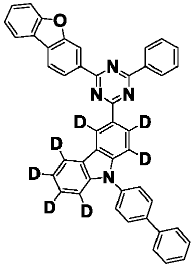 | 69% |
| 220 | 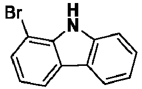 | 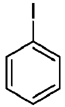 | 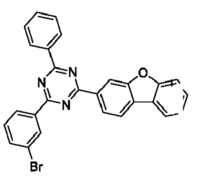 | 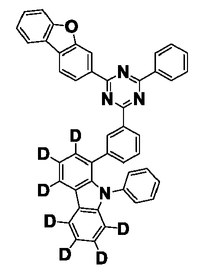 | 68% |
| 302 | 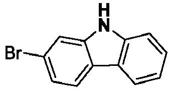 | 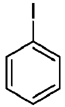 | 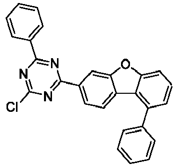 | 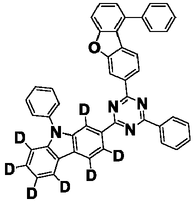 | 70% |

### <Preparation Example 6> Preparation of Compound 251

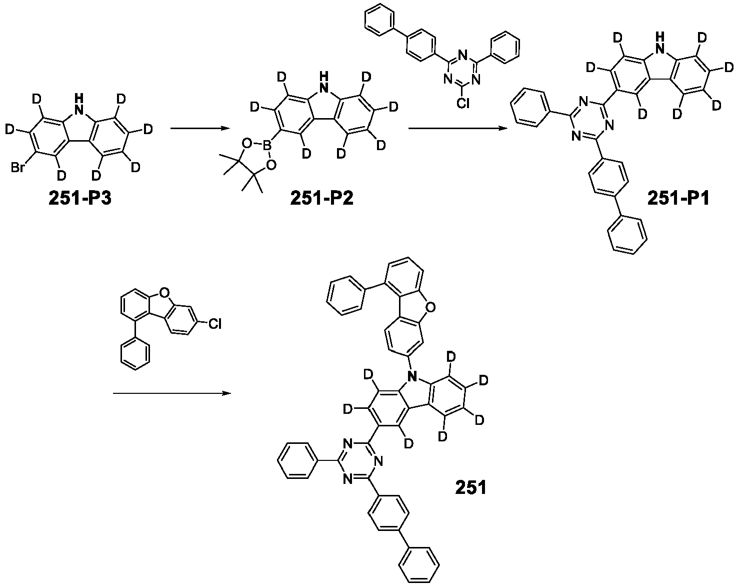

### 1) Preparation of Compound 251-P2

After 15 g (59.25 mmol) of Compound 251-P3 and 22.5 g (88.58 mmol) of 4,4,4',4',5,5,5',5'-octamethyl-2,2'-bi(1,3,2-dioxaborolane) were dissolved in 150 mL of 1,4-dioxane, 2.83 g (3.47 mmol) of Pd(dppf)Cl₂ and 20.46 g (208.74 mmol) of potassium acetate were added thereto, and the resulting mixture was stirred under reflux for 16 hours. After the reaction was completed, ethyl acetate was added to the reaction solution for dissolution, and then the resulting solution was extracted with distilled water, the organic layer was dried over anhydrous MgSO₄, and then the solvent was removed using a rotary evaporator, and then the residue was purified by column chromatography using dichloromethane and hexane as eluting solvents, thereby obtaining 22.83 g (yield = 80%) of Compound 251-P2.

### 2) Preparation of Compound 251-P1

After 22.83 g (47.4 mmol) of Compound 251-P2 and 16.3 g (47.4 mmol) of 2-([1,1'-biphenyl]-4-yl)-4-chloro-6-phenyl-1,3,5-triazine were dissolved in 300 mL of 1,4-dioxane and 60 mL of distilled water, 2.72 g (2.35 mmol) of Pd(PPh₃)₄ and 13.04 g (94.37 mmol) of K₂CO₃ were added thereto, and the resulting mixture was stirred under reflux for 5 hours. After the reaction was completed, dichloromethane was added to the reaction solution for dissolution, and then the resulting solution was extracted with distilled water, the organic layer was dried over anhydrous MgSO₄, and then the solvent was removed using a rotary evaporator, and then the residue was purified by column chromatography using dichloromethane and hexane as eluting solvents, thereby obtaining 15.52 g (yield = 68%) of Compound 251-P1.

### 3) Preparation of Compound 251

15.52 g (32.23 mmol) of Compound 251-P1, 8.98 g (32.23 mmol) of 7-chloro-1-phenyl-dibenzofuran, 7.74 g (80.57 mmol) of sodium tert-butoxide, 2.65 g (6.45 mmol) of S Phos, and 2.95 g (3.22 mmol) of Pd₂(dba)₃ were dissolved in 200 mL of toluene, and the resulting solution was stirred under reflux for 6 hours. After the reaction was completed, dichloromethane was added to the reaction solution for dissolution, and then the resulting solution was extracted with distilled water, the organic layer was dried over anhydrous MgSO₄, and then the solvent was removed using a rotary evaporator, and then the residue was purified by column chromatography using dichloromethane and hexane as eluting solvents, thereby obtaining 14.0 g (yield = 60%) of Compound 251.

A target compound in the following Table 7 was synthesized by performing the preparation in the same manner as in Preparation Example 6, except that Intermediate 1 in the following Table 7 was used instead of Compound 251-P3.

**[Table 7]**

| Compound No. | Intermediate 1 | Target compound | yield |
|---|---|---|---|
| 250 | 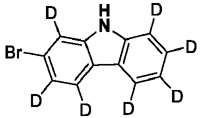 | 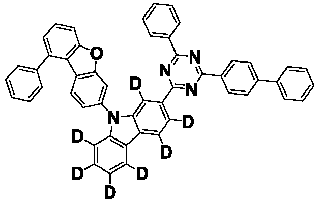 | 60% |

The other compounds other than the compounds described in Preparation Examples 1 to 6 and Tables 2 to 7 were also prepared in the same manner as in the above-described Preparation Examples, and synthesis results are shown in the following Tables 8 and 9. The following Table 8 is about the measurement values of 1H NMR (CDCl3, 400 MHz), and the following Table 9 is about the measurement values of field desorption mass spectrometry (FD-MS).

**[Table 8]**

| Compound | ¹H NMR(CDCl₃, 400MHz) |
|---|---|
| 5 | δ= 7.96 (4H, d), 7.75 (4H, d), 7.49-7.41 (6H, m), 7.25(4H, d) |
| 11 | δ= 8.36 (2H, d), 8.03-7.98 (2H, m), 7.82 (1H, d), 7.76 (1H, s), 7.54-7.50 (4H, m), 7.39-7.31 (2H, m) |
| 101 | δ= 7.96 (4H, d), 7.75 (4H, d), 7.49-7.41 (6H, m), 7.25(4H, d) |
| 14 | δ= 8.03-7.96 (4H, m), 7.82-7.75 (4H, m), 7.54-7.25 (8H, m) |
| 15 | δ= 8.38 (1H, d), 8.03-7.94 (3H, m), 7.82-7.73 (5H, m), 7.61-7.31 (7H,m) |
| 19 | δ= 8.36 (2H, d), 8.03-7.98 (2H, m), 7.82 (1H, d), 7.76 (1H, s), 7.54-7.50 (4H, m), 7.39-7.31 (2H, m) |
| 21 | δ= 8.55 (1H, d), 8.45 (1H, d), 7.96-7.92 (4H, m), 7.75-7.70 (3H, m), 7.56-7.41 (5H, m), 7.25 (2H, d) |
| 23 | δ= 8.55 (1H, d), 8.45 (1H, d), 7.96-7.92 (4H, m), 7.75-7.70 (3H, m), 7.56-7.41 (5H, m), 7.25 (2H, d) |
| 27 | δ= 8.38 (1H, d), 8.03-7.94 (3H, m), 7.82-7.73 (5H, m), 7.61-7.31 (7H,m) |
| 37 | δ= 8.08 (1H, d), 7.98-7.88 (4H, m), 7.75 (2H, d), 7.54-7.25 (7H, m) |
| 38 | δ= 8.36 (2H, d), 7.96 (2H, d), 7.75 (2H, d), 7.50-7.41 (6H, m), 7.25 (2H, d) |
| 46 | δ= 8.36 (2H, d), 7.96 (2H, d), 7.75 (2H, d), 7.50-7.41 (6H, m), 7.25 (2H, d) |
| 47 | δ= 8.36 (2H, d), 8.03-7.98 (2H, m), 7.82 (1H, d), 7.76 (1H, s), 7.54-7.50 (4H, m), 7.39-7.31 (2H, m) |
| 62 | δ= 8.36 (2H, d), 7.96 (2H, d), 7.75 (2H, d), 7.50-7.41 (6H, m), 7.25 (2H, d) |
| 63 | δ= 8.36 (2H, d), 7.96 (2H, d), 7.75 (2H, d), 7.50-7.41 (6H, m), 7.25 (2H, d) |
| 66 | δ= 8.36 (2H, d), 8.03-7.98 (2H, m), 7.82 (1H, d), 7.76 (1H, s), 7.54-7.50 (4H, m), 7.39-7.31 (2H, m) |
| 79 | δ= 8.36 (2H, d), 7.96 (2H, d), 7.75 (2H, d), 7.50-7.41 (6H, m), 7.25 (2H, d) |
| 80 | δ= 8.36 (2H, d), 8.03-7.98 (2H, m), 7.82 (1H, d), 7.76 (1H, s), 7.54-7.50 (4H, m), 7.39-7.31 (2H, m) |
| 86 | δ= 8.36 (2H, d), 7.96 (4H, d), 7.75 (2H, d), 7.50-7.41 (6H, m), 7.25 (4H, d) |
| 95 | δ= 8.38 (2H, d), 8.03-7.94 (4H, m), 7.82-7.76 (6H, m), 7.61-7.31 (8H, m) |
| 96 | δ= 8.38 (1H, d), 7.98-7.94 (4H, m), 7.82-7.25 (15H, m) |
| 97 | δ= 8.38 (1H, d), 8.08 (1H, d), 7.98-7.88 (5H, m), 7.75-7.73 (3H, m), 7.61-7.25 (10H, m) |
| 99 | δ= 8.38-8.36 (3H, m), 8.03-7.94 (3H, m), 7.82-7.73 (3H, m), 7.61-7.50 (5H, m), 7.39-7.31 (2H, m) |
| 100 | δ= 8.38 (2H, d), 7.96-7.94 (4H, m), 7.75-7.73 (6H, m), 7.61 (2H, d), 7.49-7.41 (6H, m), 7.25 (2H, d) |
| 107 | δ= 8.36 (2H, d), 8.03-7.96 (4H, m), 7.82-7.76 (2H, m), 7.54-7.50 (4H, m), 7.39-7.25 (4H, m) |
| 108 | δ= 8.38 (1H, d), 7.96-7.94 (5H, m), 7.73-7.75 (5H, m), 7.61 (1H, d), 7.49-7.41 (6H, m), 7.25 (4H, d) |
| 116 | δ= 8.38 (1H, d), 7.98-7.94 (4H, m), 7.82-7.25 (15H, m) |
| 117 | δ= 8.38 (1H, d), 8.08 (1H, d), 7.96-7.88 (5H, m), 7.75-7.73 (3H, m), 7.61-7.25 (10H, m) |
| 119 | δ= 8.38-8.36 (3H, m), 8.03-7.94 (3H, m), 7.82-7.73 (3H, m), 7.61-7.50 (5H, m), 7.39-7.31 (2H, m) |
| 120 | δ= 8.38 (2H, m), 7.96-7.94 (4H, m), 7.75-7.73 (6H, m), 7.61 (2H, d), 7.49-7.41 (6H, m), 7.25 (2H, d) |
| 134 | δ= 8.36 (2H, d), 7.96 (4H, d), 7.75 (2H, d), 7.50-7.41 (6H, m), 7.25 (4H, d) |
| 143 | δ= 8.38-8.36 (3H, m), 7.96-7.94 (4H, m), 7.75-7.73 (4H, m), 7.61 (2H, d), 7.50-7.41 (6H, m), 7.25 (2H, d) |
| 160 | δ= 8.38-8.36 (3H, m), 8.03-7.94 (3H, m), 7.82-7.73 (3H, m), 7.61-7.50 (5H, m), 7.39-7.31 (2H, m) |
| 185 | δ= 7.96 (4H, d), 7.75 (4H, d), 7.62-7.41 (11H, m), 7.25 (4H, d) |
| 190 | δ= 8.03-7.96 (4H, m), 7.82-7.75 (4H, m), 7.62-7.25 (13H, m) |
| 200 | δ= 8.36 (2H, d), 8.03-7.91 (6H, m), 7.82-7.75 (4H, m), 7.54-7.31 (9H, m) |
| 220 | δ= 8.38-8.36 (3H, m), 8.03-7.94 (3H, m), 7.82-7.73 (3H, m), 7.62-7.50 (10H, m), 7.39-7.31 (2H, m) |
| 228 | δ = deuterium content of 100% with no ¹H NMR peak |
| 230 | δ = deuterium content of 100% with no ¹H NMR peak |
| 250 | δ= 8.36 (2H, d), 7.98-7.96 (3H, m), 7.82-7.69 (6H, m), 7.57-7.41(11H, m), 7.25 (3H, m) |
| 251 | δ= 8.36 (2H, d), 7.98-7.96 (3H, m), 7.82-7.69 (6H, m), 7.57-7.41 (11H, m), 7.25 (3H, d) |
| 263 | δ= 8.38 (1H, d), 8.03-7.94 (5H, m), 7.82-7.73 (5H, m), 7.61-7.31 (9H, m) |
| 287 | δ= 8.36 (2H, d), 8.03-7.96 (4H, m), 7.82-7.76 (2H, m), 7.60-7.50 (6H, m), 7.39-7.31 (2H, m) |
| 302 | δ= 8.36 (2H, d) 8.03 (1H, d), 7.82-7.76 (5H, m), 7.69-7.41 (13H, m) |
| 2-50 | δ = deuterium content of 100% with no ¹H NMR peak |
| 2-51 | δ = deuterium content of 100% with no ¹H NMR peak |
| 2-53 | δ = deuterium content of 100% with no ¹H NMR peak |
| 2-56 | δ = deuterium content of 100% with no ¹H NMR peak |
| 2-57 | δ = deuterium content of 100% with no ¹H NMR peak |
| 2-74 | δ= 8.55 (1H, d), 8.30 (1H, d), 8.19-8.13 (2H, m), 7.94-7.89 (8H, m), 7.77-7.75 (3H, m), 7.62-7.35 (11H, m), 7.20-7.16 (2H m) |
| 2-76 | δ = 8.55 (1H, d), 8.30 (1H, d), 8.13-8.19 (2H, m), 7.89-7.99 (9H, m), 7.73-7.77 (4H, m), 7.35-7.62 (13H, m), 7.16-7.20 (2H, m) |
| 2-77 | δ = 8.55 (1H, d), 8.30 (1H, d), 8.13-8.21 (4H, m), 7.89-7.99 (4H, m), 7.35-7.77 (20H, m), 7.16-7.20 (2H, t) |
| 2-78 | δ = 8.55 (1H, d), 8.30 (1H, d), 8.13-8.19 (2H, m), 7.89-7.99 (12H, m), 7.75-7.77 (5H, m), 7.58 (1H, d), 7.35-7.50 (8H, m), 7.16-7.20 (2H, m) |
| 2-79 | δ= 8.55 (1H, d), 8.30 (1H, d), 8.21-8.13 (3H, m), 7.99-7.89 (8H, m), 7.77-7.35 (12H, m), 7.20-7.16 (2H, m) |

**[Table 9]**

| Compound | FD-MS | Compound | FD-MS |
|---|---|---|---|
| 5 | m/z= 638.84(C₄₅H₁₈D₁₂N₄=638 .32) | 79 | m/z= 642.86(C₄₅H₁₄D₁₆N₄=642.35) |
| 11 | m/z= 576.72(C₃₉H₁₂D₁₂N₄O=57 6.27) | 80 | m/z= 656.84 (C₄₅H₁₂D₁₆N₄O=656.33) |
| 14 | m/z= 652.82(C₄₅H₁₆D₁₂N₄O=65 2.30) | 86 | m/z= 638.84 (C₄₅H₁₈D₁₂N₄=63 8.32) |
| 15 | m/z= 652.82 (C₄₅H₁₆D₁₂N₄O=65 2.30) | 95 | m/z=728.92(C₅₁H₂₀D₁₂N₄O=728. 33) |
| 19 | m/z= 576.72(C₃₉H₁₂D₁₂N₄O=57 6.27) | 96 | m/z=728.92(C₅₁H₂₀D₁₂N₄O=728. 33) |
| 21 | m/z= 668.88(C₄₅H₁₆D₁₂N₄S=66 8.28) | 97 | m/z=728.92(C₅₁H₂₀D₁₂N₄O=728. 33) |
| 23 | m/z= 668.88(C₄₅H₁₆D₁₂N₄S=66 8.28) | 99 | m/z= 652.82(C₄₅H₁₆D₁₂N₄O=652.30) |
| 27 | m/z= 652.82(C₄₅H₁₆D₁₂N₄O=65 2.30) | 100 | m/z= 714.93(C₅₁H₂₂D₁₂N₄=714.35) |
| 37 | m/z= 732.94(C₅₁H₁₆D₁₆N₄O=73 2.36) | 101 | m/z= 638.84(C₄₅H₁₈D₁₂N₄=638.32) |
| 38 | m/z= 642.86(C₄₅H₁₄D₁₆N₄=642 .35) | 107 | m/z= 652.82(C₄₅H₁₆D₁₂N₄O=652.30) |
| 46 | m/z= 642.86(C₄₅H₁₄D₁₆N₄=642 .35) | 108 | m/z= 714.93(C₅₁H₂₂D₁₂N₄=714. 35) |
| 47 | m/z= 656.84(C₄₅H₁₂D₁₆N₄O=65 6.33) | 116 | m/z=728.92(C₅₁H₂₀D₁₂N₄O=728. 33) |
| 62 | m/z= 562.74(C₃₉H₁₄D₁₂N₄=562 .29) | 117 | m/z=728.92(C₅₁H₂₀D₁₂N₄O=728. 33) |
| 63 | m/z= 642.86(C₄₅H₁₄D₁₆N₄=642.35) | 119 | m/z= 652.82(C₄₅H₁₆D₁₂N₄O=652.30) |
| 66 | m/ z= 656.84(C₄₅H₁₂D₁₆N₄O=65 6.33) | 120 | m/z= 714.93(C₅₁H₂₂D₁₂N₄=714.35) |
| 134 | m/ z= 638.84(C₄₅H₁ₑD₁₂N₄=638 .32) | 228 | m/z= 668.92(C₄₅D₂₈N₄O=668.40) |
| 143 | m/z= 714.93(C₅₁H₂₂D₁₂N₄=714 .35) | 230 | m/z= 668.92(C₄₅D₂₈N₄O=668.40) |
| 160 | m/z= 732.94(C₅₁H₁₆D₁₆N₄O=73 2.36) | 250 | m/z= 723.89(C₅₁H₂₅D₇N₄O=723.30) |
| 185 | m/ z= 633.81(C₄₅H₂₃D₇N₄=633. 29) | 251 | m/z= 723.89(C₅₁H₂₅D₇N₄O=723.30) |
| 190 | m/z= 647.79(C₄₅H₂₁D₇N₄O=647 .27) | 263 | m/z=728.92(C₅₁H₂₀D₁₂N₄O=728. 33) |
| 200 | m/z= 647.79(C₄₅H₂₁D₇N₄O=647 .27) | 287 | m/z= 652.82(C₄₅H₁₆D₁₂N₄O=652.30) |
| 220 | m/z= 647.79(C₄₅H₂₁D₇N₄O=647.27) | 302 | m/z= 647.79(C₄₅H₂₁D₇N₄O=647.27) |
| 2-79 | m/z= 636.80(C₄₈H₃₂N₂=636.2 6) | 2-74 | m/z= 560.23(C₄₂H₂₈N₂=560.70) |
| 2-51 | m/z= 588.87(C₄₂D₂₈N₂=588.4 0) | 2-53 | m/z= 668.99(C₄₈D₃₂N₂=668.46) |
| 2-56 | m/z= 668.99(C₄₈D₃₂N₂=668.4 6) | 2-50 | m/z= 668.99(C₄₈D₃₂N₂=668.46) |
| 2-76 | m/z= 636.80(C₄₈H₃₂N₂=636.2 6) | 2-77 | m/z= 636.80(C₄₈H₃₂N₂=636.26) |
| 2-78 | m/z= 636.80(C₄₈H₃₂N₂=636.2 6) | 2-57 | m/z=668.46(C₄₈D₃₂N₂=668.99) |

### [Experimental Examples]

### <Experimental Example 1>

### 1) Manufacture of Organic Light Emitting Device

A glass substrate, in which ITO was thinly coated to have a thickness of 1,500 Å, was ultrasonically washed with distilled water. When the washing with distilled water is finished, the glass substrate was ultrasonically washed with a solvent such as acetone, methanol, and isopropyl alcohol, was dried and then was subjected to UVO treatment for 5 minutes by using UV in a UV washing machine. Thereafter, the substrate was transferred to a plasma washing machine (PT), and then was subjected to plasma treatment in a vacuum state for an ITO work function and in order to remove a residual film, and was transferred to a thermal deposition apparatus for organic deposition.

As the common layers, the hole injection layer 4,4',4''-tris[2-naphthyl(phenyl)amino]triphenylamine (2-TNATA) and the hole transport layer N,N'-di(1-naphthyl)-N,N'-diphenyl-(1,1'-biphenyl)-4,4'-diamine (NPB) were formed on the ITO transparent electrode (positive electrode).

A light emitting layer was thermally vacuum deposited thereon as follows. The light emitting layer was deposited to have a thickness of 360 Å by using a heterocyclic compound of Chemical Formula 1 in the following Table 10 as a host and tris(2-phenylpyridine) iridium (Ir(ppy)₃) as a green phosphorescent dopant to dope the host with Ir(ppy)₃ in an amount of 7%.

Thereafter, BCP as a hole blocking layer was deposited to have a thickness of 60 Å, and Alq₃ as an electron transport layer was deposited to have a thickness of 200 Å thereon. Finally, lithium fluoride (LiF) was deposited to have a thickness of 10 Å on the electron transport layer to form an electron injection layer, and then an aluminum (Al) negative electrode was deposited to have a thickness of 1200 Å on the electron injection layer to form a negative electrode, thereby manufacturing an organic electroluminescence device.

In addition to those separately indicated as red hosts in the following Table 10, the Examples and Comparative Examples were used as green hosts. Ir(piq)₂(acac) as a red phosphorescent dopant was used in an amount of 7 wt%.

Meanwhile, all the organic compounds required for manufacturing an OLED device were subjected to vacuum sublimed purification under 10⁻⁸ to 10⁻⁶ torr for each material, and used for the manufacture of OLED.

The organic light emitting devices of Comparative Examples 1 to 8 were manufactured in the same manner as in the Experimental Example, except that the following comparative compounds were used as the host of the light emitting layer.

### [Comparative Examples]

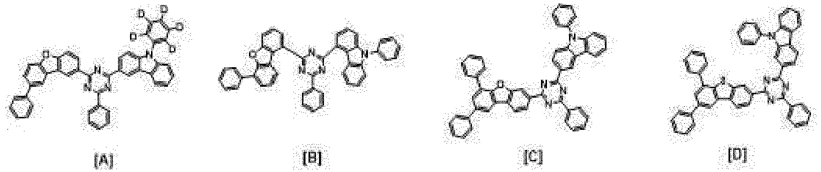

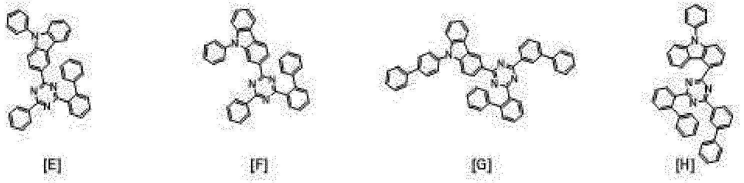

The organic light emitting devices of Reference Examples 1 to 5 were manufactured in the same manner as in the Experimental Example, except that the following reference compounds were used as the host of the light emitting layer.

### [Reference Examples]

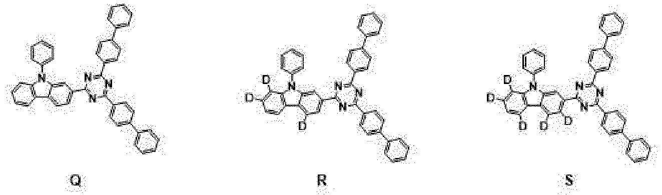

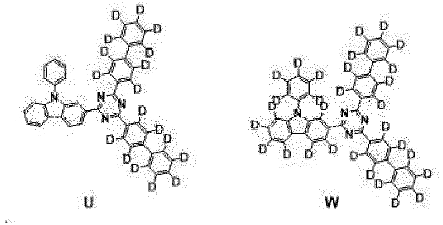

### 2) Driving voltage, light emitting efficiency, color coordinate and service life of organic light emitting device

For the organic electroluminescence device manufactured as described above, electroluminescence (EL) characteristics were measured by M7000 manufactured by McScience Inc., and based on the measurement result thereof, T₉₀ was measured by a service life measurement device (M6000) manufactured by McScience Inc., when the reference luminance was 6,000 cd/m².

The results of measuring the driving voltage, light emitting efficiency, color coordinate (CIE) and service life of the organic light emitting device manufactured according to the present invention are shown in the following Table 10.

**[Table 10]**

| | Compound | Driving voltage (V) | Light emitting efficiency (cd/A) | Color coordinate (x, y) | Service life (T₉₀) |
|---|---|---|---|---|---|
| Example 1 | 5 | 4.01 | 62.3 | (0.243, 0.714) | 103 |
| Example 2 | 11 | 4.07 | 61.9 | (0.241, 0.711) | 106 |
| Example 3 | 14 | 4.08 | 62.5 | (0.241, 0.714) | 103 |
| Example 4 | 15 | 4.10 | 63.3 | (0.241, 0.715) | 107 |
| Example 5 | 19 | 4.06 | 62.1 | (0.231, 0.712) | 102 |
| Example 6 | 21 | 4.09 | 62.6 | (0.251, 0.714) | 104 |
| Example 7 | 23 | 4.18 | 61.0 | (0.241, 0.711) | 98 |
| Example 8 | 27 | 4.19 | 61.2 | (0.251, 0.714) | 99 |
| Example 9 | 37 (Red host) | 4.28 | 62.1 | (0.241, 0.714) | 90 |
| Example 10 | 38 | 4.15 | 63.5 | (0.242, 0.713) | 108 |
| Example 11 | 46 | 4.06 | 63.0 | (0.248, 0.715) | 113 |
| Example 12 | 47 | 4.11 | 62.4 | (0.251, 0.714) | 118 |
| Example 13 | 62 | 4.19 | 61.1 | (0.251, 0.714) | 98 |
| Example 14 | 63 | 4.12 | 61.9 | (0.247, 0.727) | 117 |
| Example 15 | 66 | 4.07 | 62.7 | (0.231, 0.711) | 112 |
| Example 16 | 79 | 4.06 | 63.3 | (0.246, 0.717) | 107 |
| Example 17 | 80 | 4.15 | 64.2 | (0.231, 0.711) | 105 |
| Example 18 | 86 | 4.16 | 64.0 | (0.671, 0.320) | 114 |
| Example 19 | 95 | 4.07 | 63.4 | (0.246, 0.717) | 100 |
| Example 20 | 96 | 4.03 | 62.7 | (0.233, 0.701) | 106 |
| Example 21 | 97 | 4.01 | 62.4 | (0.251, 0.713) | 118 |
| Example 22 | 99 | 4.07 | 63.0 | (0.254, 0.724) | 113 |
| Example 23 | 100 | 4.03 | 62.4 | (0.233, 0.703) | 115 |
| Example 24 | 101 | 4.00 | 64.8 | (0.234, 0.714) | 119 |
| Example 25 | 107 | 4.08 | 61.9 | (0.243, 0.693) | 104 |
| Example 26 | 108 | 4.04 | 62.4 | (0.251, 0.724) | 102 |
| Example 27 | 116 | 4.13 | 63.0 | (0.242, 0.713) | 114 |
| Example 28 | 117 | 4.01 | 63.5 | (0.243, 0.712) | 115 |
| Example 29 | 119 | 4.02 | 62.2 | (0.242, 0.716) | 115 |
| Example 30 | 120 | 4.12 | 62.7 | (0.241, 0.713) | 118 |
| Example 31 | 134 | 4.10 | 62.5 | (0.251, 0.714) | 108 |
| Example 32 | 143 | 4.16 | 63.9 | (0.247, 0.727) | 109 |
| Example 33 | 160 | 4.12 | 62.8 | (0.231, 0.711) | 111 |
| Example 34 | 185 | 4.17 | 61.3 | (0.246, 0.717) | 96 |
| Example 35 | 190 | 4.18 | 61.4 | (0.231, 0.712) | 96 |
| Example 36 | 200 | 4.16 | 61.6 | (0.251, 0.714) | 95 |
| Example 37 | 220 | 4.19 | 61.2 | (0.241, 0.711) | 95 |
| Example 38 | 228 | 4.19 | 62.2 | (0.251, 0.714) | 99 |
| Example 39 | 230 | 4.20 | 62.3 | (0.242, 0.713) | 99 |
| Example 40 | 250 | 4.15 | 62.0 | (0.248, 0.715) | 98 |
| Example 41 | 251 | 4.16 | 62.1 | (0.251, 0.714) | 98 |
| Example 42 | 263 | 4.10 | 62.3 | (0.251, 0.714) | 105 |
| Example 43 | 287 | 4.11 | 62.2 | (0.246, 0.717) | 106 |
| Example 44 | 302 | 4.18 | 61.9 | (0.251, 0.724) | 95 |
| Comparative Example 1 | A | 4.75 | 56.3 | (0.248, 0.715) | 65 |
| Comparative Example 2 | B | 4.80 | 53.8 | (0.246, 0.717) | 42 |
| Comparative Example 3 | C | 4.65 | 56.0 | (0.231, 0.711) | 60 |
| Comparative Example 4 | D | 4.67 | 56.4 | (0.251, 0.713) | 62 |
| Comparative Example 5 | E | 4.85 | 53.5 | (0.245, 0.716) | 45 |
| Comparative Example 6 | F | 4.77 | 54.0 | (0.242, 0.713) | 50 |
| Comparative Example 7 | G | 4.78 | 53.8 | (0.251, 0.724) | 50 |
| Comparative Example 8 | H | 4.89 | 52.8 | (0.243, 0.693) | 40 |
| Reference Example 1 | Q | 4.25 | 60.0 | (0.251, 0.714) | 88 |
| Reference Example 2 | R | 4.20 | 60.6 | (0.247, 0.727) | 93 |
| Reference Example 3 | S | 4.15 | 61.3 | (0.231, 0.711) | 100 |
| Reference Example 4 | U | 4.35 | 57.8 | (0.246, 0.717) | 70 |
| Reference Example 5 | W | 4.25 | 60.1 | (0.251, 0.724) | 95 |

Referring to the results of Table 10, it can be seen that organic light emitting devices (Examples 1 to 44) including the heterocyclic compound of Chemical Formula 1 of the present invention have excellent driving voltage, light emitting efficiency and service life than Comparative Examples 1 to 8.

Specifically, the compound of the present invention has a heterocyclic carbazole substituted with deuterium. When the organic light emitting device is driven, the molecule is thermally damaged by the transfer of electrons. In particular, a heterocyclic structure including carbazole is likely to be defective at the most unstable site nitrogen having a pentagonal ring.

In order to prevent this problem, the compound of the present invention lowers the energy of the molecule by substituting carbazole which is a hetero ring with deuterium which has a higher molecular weight than hydrogen to reduce the change in vibrational frequency, and accordingly, a compound that increases the stability of the molecule was developed. Further, since the single bond dissociation energy of carbon and deuterium is higher than the single bond dissociation energy of carbon and hydrogen, it can be confirmed that the service life of the device is improved as the thermal stability of the molecule is enhanced.

Additionally, a reference experiment was performed using Compound 101 and the reference compound in order to confirm what changes occurred in the organic light emitting device depending on the position of deuterium substitution.

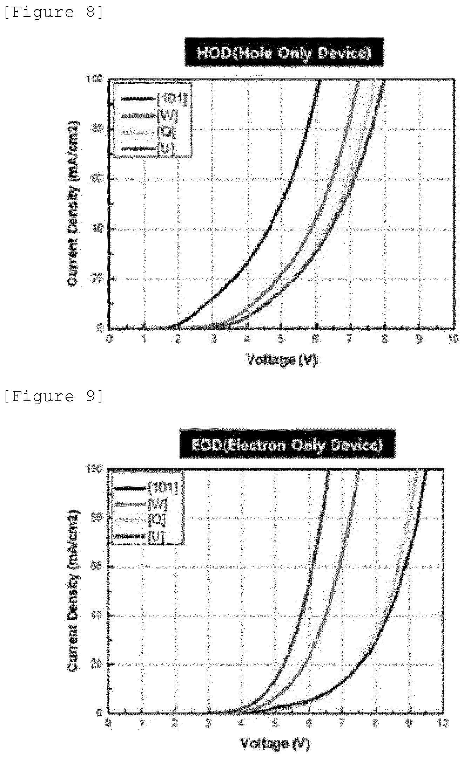

In OLED devices, the mobility of electrons and holes plays an important role in device performance. FIG. 8 illustrates the results of measuring the hole mobility (HOD) of Reference Compounds Q, U, and W and Compound 101, and FIG. 9 illustrates the results of measuring the electron mobility (EOD) of Reference Compounds Q, U, and W and Compound 101. Reference Compound Q is a compound which is not substituted with deuterium. It can be confirmed that EOD appears faster than HOD when the compound is not substituted with deuterium. In this case, the recombination zone (RZ) in the OLED device is located near the hole transport layer (HTL) rather than in the center of the light emitting layer. (See FIG. 4) This can be confirmed in the RZ test experiment in FIG. 4.

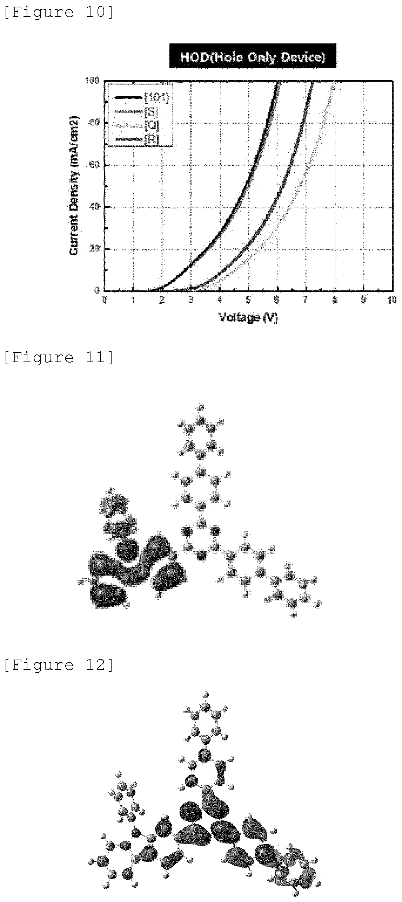

In the case of Compound 101, when the electron cloud distribution of the HOMO and LUMO states is observed, the LUMO is distributed in triazines and the HOMO is distributed in carbazoles. (See FIGS. 11 and 12) in an organic compound molecule, holes move through the HOMO and electrons move through the LUMO. A compound substituted with deuterium has a higher packing density than a compound substituted with hydrogen. Therefore, when the compound is substituted with deuterium, the intermolecular distance is short, so that electrons and holes move faster.

When the triazines responsible for the LUMO are substituted with deuterium, as in Reference Compound U, the electrons move faster and thus are more shifted toward the HTL than when the RZ is not substituted with deuterium. Accordingly, it is determined that a result that both efficiency and service life were reduced occurred.

When the entire compound is substituted with deuterium as in Reference Compound W, the driving voltage and light emitting efficiency are similar to those of Reference Compound Q, but an excellent service life is exhibited. This is interpreted as the result that both electrons and holes become fast and RZ is not changed.

In contrast, in the case of Compound 101, which is the compound of the present invention, carbazoles responsible for the HOMO are substituted with deuterium. Accordingly, it was confirmed that the movement of holes was faster than that of Reference Compound Q which is not substituted with deuterium, and thus, the RZ was located widely in the center of the EML layer, thereby improving the efficiency and service life. In other words, it can be confirmed that when the compound of the present invention is used as a host of a light emitting layer, the driving voltage, light emitting efficiency and service life are remarkably excellent.

A recombination zone (RZ) confirmation experiment was conducted by the same organic light emitting device manufacturing method as in Experimental Example 1. The difference is the doping position of the green phosphorescent dopant in the light emitting layer.

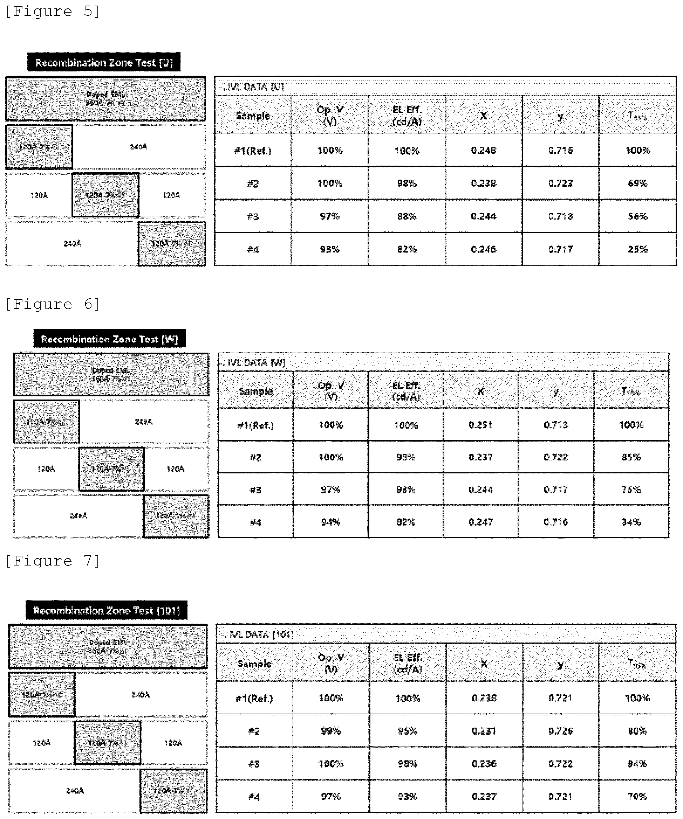

In FIGS. 4 to 7, #1 shows that the entire light emitting layer was doped with a green phosphorescent dopant, and the doped light emitting layer was used as a comparative group. In the case of #2, only 120 Å of a colored portion (located close to a hole transport layer) was doped, and only a host was deposited onto the remaining 240 Å. In the case of #3, only 120 Å of a colored portion (located at the center of a light emitting layer) was doped, and only a host was deposited onto the remaining uncolored portion. In the case of #4, only 120 Å of a colored portion (located close to a hole blocking layer) was doped, and only a host was deposited onto the remaining uncolored portion.

In the case of Reference Compound Q, RZ was located close to the hole transport layer, and #2 was found to have the best efficiency and service life. This may also result in electrons moving relatively faster than holes, as shown in FIGS. 8 and 9.

In the case of reference compound U, the service life was shown to be shorter than that of Reference Compound Q because triazines responsible for the LUMO were substituted with deuterium. It is determined that this result occurred because the electrons move faster than Reference Compound Q, so that the RZ appears more shifted toward the hole transport layer.

In the case of Reference Compound W, the driving voltage and light emitting efficiency are similar to those of Reference Compound Q, but the service life is excellent. Reference Compound W in which Reference Compound Q is fully substituted with deuterium is interpreted to exhibit the result because both electrons and holes become fast, so that the RZ is not changed.

In contrast, Compound 101 exhibits the highest efficiency and service life at #3 (located at the center of a light emitting layer). As described above, the heterocyclic ring responsible for the HOMO is substituted with deuterium. Accordingly, it was confirmed that the movement of holes is faster than that of Reference Compound Q which is not substituted with deuterium, the movement of electrons and holes is well-balanced, and thus, the RZ is widely located in the center of the EML layer.

In the case of a compound with relatively fast electron movement, as in the compound of the present invention, the use of an electron blocking layer is expected to prevent electrons from passing over in the light emitting layer, resulting in higher efficiency.

Additionally, a reference experiment was conducted to determine how the HOD of the organic light emitting device is changed depending on the deuterium content of Structure 1-C of Chemical Formula 1 (FIG. 10). As a result of the experiment, the HOD of Compound R with a deuterium content of 43% was measured to be relatively faster than that of Compound Q not substituted with deuterium. In addition, it was confirmed that Compounds Q and R with a deuterium content of 0% and 43%, respectively, had relatively slow HOD compared to Compound 101 with 100% substitution. It was confirmed that Compound S with a deuterium content of 70% or more exhibited a HOD similar to that of 100% substitution.

Recombination zone (RZ) confirmation experiment results and Hole Only Device (HOD) and Electron Only Device (EOD) experiment results for Reference Compounds Q, U, and W, and Compound 101 can be confirmed in FIGS. 4 to 9. Hole Only Device (HOD) experimental results for Reference Compounds S, R, and Q, and Compound 101 can be confirmed in FIG. 10.

### <Experimental Example 2>

### 1) Manufacture of Organic Light Emitting Device

A glass substrate, in which ITO was thinly coated to have a thickness of 1,500 Å, was ultrasonically washed with distilled water. When the washing with distilled water was finished, the glass substrate was ultrasonically washed with a solvent such as acetone, methanol, and isopropyl alcohol, dried and then was subjected to UVO treatment for 5 minutes using UV in a UV cleaning machine. Thereafter, the substrate was transferred to a plasma washing machine (PT), and then was subjected to plasma treatment in a vacuum state for an ITO work function and in order to remove a residual film, and was transferred to a thermal deposition apparatus for organic deposition.

As the common layers, the hole injection layer 4,4',4''-tris[2-naphthyl(phenyl)amino]triphenylamine (2-TNATA) and the hole transport layer N,N'-di(1-naphthyl)-N,N'-diphenyl-(1,1'-biphenyl)-4,4'-diamine (NPB) were formed on the ITO transparent electrode (positive electrode).

A light emitting layer was thermally vacuum deposited thereon as follows. The light emitting layer was deposited to have a thickness of 360 Å from one common container after pre-mixing one heterocyclic compound of Chemical Formula 1 and one compound of Chemical Formula 2 as hosts, and then was deposited by doping the host with Ir(ppy)₃ as a green phosphorescent dopant in an amount of 7% of the deposition thickness of the light emitting layer. Thereafter, BCP as a hole blocking layer was deposited to have a thickness of 60 Å, and Alq₃ as an electron transport layer was deposited to have a thickness of 200 Å thereon.

Finally, lithium fluoride (LiF) was deposited to have a thickness of 10 Å on the electron transport layer to form an electron injection layer, and then an aluminum (Al) negative electrode was deposited to have a thickness of 1,200 Å on the electron injection layer to form a negative electrode, thereby manufacturing an organic electroluminescence device.

In addition to those separately indicated as red hosts in the following Table 11, the Examples and Comparative Examples were used as green hosts. Ir(piq)₂(acac) as a red phosphorescent dopant was used in an amount of 7 wt%.

Meanwhile, all the organic compounds required for manufacturing an OLED device were subjected to vacuum sublimed purification under 10⁻⁸ to 10⁻⁶ torr for each material, and used for the manufacture of OLED.

### 2) Driving voltage, light emitting efficiency, color coordinate and service life of organic light emitting device

For the organic electroluminescence device manufactured as described above, electroluminescence (EL) characteristics were measured by M7000 manufactured by McScience Inc., and based on the measurement result thereof, T₉₀ was measured by a service life measurement device (M6000) manufactured by McScience Inc., when the reference luminance was 6,000 cd/m².

The results of measuring the driving voltage, light emitting efficiency, color coordinate (CIE) and service life of the organic light emitting device manufactured according to the present invention are shown in the following Table 11.

**[Table 11]**

| | Light emitting layer Compound | Ratio | Driving voltage (V) | Light emitting efficiency (cd/A) | Color coordinate (x, y) | Service life (T₉₀) |
|---|---|---|---|---|---|---|
| Example 45 | 101 : 2-74 | 1 : 1 | 3.80 | 66.3 | (0.248, 0.637) | 155 |
| Example 46 | | 1 : 2 | 3.87 | 65.7 | (0.269, 0.611) | 160 |
| Example 47 | | 1 : 3 | 3.93 | 65.1 | (0.251, 0.693) | 165 |
| Example 48 | 101 : 2-51 | 1 : 1 | 3.81 | 66.4 | (0.248, 0.637) | 290 |
| Example 49 | | 1 : 2 | 3.87 | 65.8 | (0.269, 0.611) | 297 |
| Example 50 | | 1 : 3 | 3.94 | 65.1 | (0.251, 0.693) | 303 |
| Example 51 | 14 : 2-76 | 1 : 1 | 3.82 | 66.7 | (0.245, 0.677) | 151 |
| Example 52 | | 1 : 2 | 3.86 | 65.8 | (0.258, 0.647) | 155 |
| Example 53 | | 1 : 3 | 3.93 | 64.8 | (0.266, 0.645) | 160 |
| Example 54 | 14 : 2-53 | 1 : 1 | 3.81 | 66.8 | (0.245, 0.677) | 295 |
| Example 55 | | 1 : 2 | 3.86 | 65.8 | (0.258, 0.647) | 300 |
| Example 56 | | 1 : 3 | 3.92 | 64.9 | (0.266, 0.645) | 305 |
| Example 57 | 15 : 2-76 | 1 : 1 | 3.83 | 66.5 | (0.256, 0.673) | 152 |
| Example 58 | | 1 : 2 | 3.88 | 65.6 | (0.237, 0.644) | 156 |
| Example 59 | | 1 : 3 | 3.93 | 64.7 | (0.237, 0.624) | 161 |
| Example 60 | 15 : 2-53 | 1 : 1 | 3.83 | 66.7 | (0.256, 0.673) | 294 |
| Example 61 | | 1 : 2 | 3.87 | 65.7 | (0.237, 0.644) | 300 |
| Example 62 | | 1 : 3 | 3.93 | 64.7 | (0.237, 0.624) | 306 |
| Example 63 | 19 : 2-77 | 1 : 1 | 3.85 | 67.7 | (0.245, 0.617) | 155 |
| Example 64 | | 1 : 2 | 3.91 | 66.8 | (0.257, 0.624) | 160 |
| Example 65 | | 1 : 3 | 3.95 | 65.8 | (0.259, 0.712) | 164 |
| Example 66 | 19 : 2-56 | 1 : 1 | 3.84 | 67.6 | (0.245, 0.617) | 300 |
| Example 67 | | 1 : 2 | 3.90 | 66.8 | (0.257, 0.624) | 305 |
| Example 68 | | 1 : 3 | 3.94 | 65.9 | (0.259, 0.712) | 311 |
| Example 69 | 27 : 2-79 | 1 : 1 | 3.79 | 67.3 | (0.243, 0.643) | 156 |
| Example 70 | | 1 : 2 | 3.84 | 66.2 | (0.261, 0.764) | 160 |
| Example 71 | | 1 : 3 | 3.90 | 65.4 | (0.258, 0.628) | 165 |
| Example 72 | 27 : 2-57 | 1 : 1 | 3.78 | 67.2 | (0.243, 0.643) | 301 |
| Example 73 | | 1 : 2 | 3.83 | 66.2 | (0.261, 0.764) | 304 |
| Example 74 | | 1 : 3 | 3.89 | 65.4 | (0.258, 0.628) | 310 |
| Example 75 | 38 : 2-78 | 1 : 1 | 3.80 | 67.4 | (0.254, 0.653) | 156 |
| Example 76 | | 1 : 2 | 3.85 | 66.4 | (0.275, 0.657) | 160 |
| Example 77 | | 1 : 3 | 3.93 | 65.4 | (0.264, 0.642) | 165 |
| Example 78 | 38 : 2-50 | 1 : 1 | 3.80 | 67.4 | (0.254, 0.653) | 302 |
| Example 79 | | 1 : 2 | 3.85 | 66.5 | (0.275, 0.657) | 307 |
| Example 80 | | 1 : 3 | 3.92 | 65.5 | (0.264, 0.642) | 313 |
| Example 81 | 47 : 2-74 | 1 : 1 | 3.84 | 68.2 | (0.256, 0.638) | 157 |
| Example 82 | | 1 : 2 | 3.90 | 67.2 | (0.251, 0.632) | 162 |
| Example 83 | | 1 : 3 | 3.95 | 66.3 | (0.253, 0.684) | 167 |
| Example 84 | 47 : 2-51 | 1 : 1 | 3.83 | 68.3 | (0.256, 0.638) | 305 |
| Example 85 | | 1 : 2 | 3.90 | 67.3 | (0.251, 0.632) | 309 |
| Example 86 | | 1 : 3 | 3.94 | 66.4 | (0.253, 0.684) | 314 |
| Example 87 | 66 : 2-77 | 1 : 1 | 3.76 | 67.6 | (0.235, 0.655) | 158 |
| Example 88 | | 1 : 2 | 3.84 | 66.7 | (0.236, 0.624) | 163 |
| Example 89 | | 1 : 3 | 3.92 | 65.7 | (0.255, 0.692) | 168 |
| Example 90 | 66 : 2-56 | 1 : 1 | 3.76 | 67.6 | (0.235, 0.655) | 307 |
| Example 91 | | 1 : 2 | 3.84 | 66.7 | (0.236, 0.624) | 312 |
| Example 92 | | 1 : 3 | 3.92 | 65.7 | (0.255, 0.692) | 317 |
| Example 93 | 80 : 2-79 | 1 : 1 | 3.83 | 66.9 | (0.253, 0.724) | 159 |
| Example 94 | | 1 : 2 | 3.88 | 65.9 | (0.242, 0.625) | 164 |
| Example 95 | | 1 : 3 | 3.92 | 64.9 | (0.261, 0.623) | 167 |
| Example 96 | 80 : 2-57 | 1 : 1 | 3.83 | 66.9 | (0.253, 0.724) | 308 |
| Example 97 | | 1 : 2 | 3.87 | 65.9 | (0.242, 0.625) | 312 |
| Example 98 | | 1 : 3 | 3.91 | 65.1 | (0.261, 0.623) | 316 |
| Example 99 | 86 : 2-76 | 1 : 1 | 3.82 | 67.3 | (0.253, 0.614) | 163 |
| Example 100 | | 1 : 2 | 3.88 | 66.4 | (0.254, 0.659) | 168 |
| Example 101 | | 1 : 3 | 3.94 | 65.5 | (0.255, 0.635) | 173 |
| Example 102 | 86 : 2-53 | 1 : 1 | 3.81 | 67.4 | (0.253, 0.614) | 317 |
| Example 103 | | 1 : 2 | 3.87 | 66.5 | (0.254, 0.659) | 323 |
| Example 104 | | 1 : 3 | 3.93 | 65.5 | (0.255, 0.635) | 329 |
| Example 105 | 97 : 2-78 | 1 : 1 | 3.79 | 65.9 | (0.257, 0.714) | 165 |
| Example 106 | | 1 : 2 | 3.86 | 65.1 | (0.249, 0.666) | 168 |
| Example 107 | | 1 : 3 | 3.92 | 64.1 | (0.253, 0.635) | 172 |
| Example 108 | 97 : 2-50 | 1 : 1 | 3.78 | 66.0 | (0.257, 0.714) | 315 |
| Example 109 | | 1 : 2 | 3.86 | 65.1 | (0.249, 0.666) | 320 |
| Example 110 | | 1 : 3 | 3.92 | 64.2 | (0.253, 0.635) | 325 |
| Example 111 | 99 : 2-74 | 1 : 1 | 3.83 | 67.3 | (0.268, 0.615) | 160 |
| Example 112 | | 1 : 2 | 3.88 | 66.3 | (0.253, 0.628) | 165 |
| Example 113 | | 1 : 3 | 3.92 | 65.4 | (0.256, 0.713) | 170 |
| Example 114 | 99 : 2-51 | 1 : 1 | 3.82 | 67.2 | (0.268, 0.615) | 312 |
| Example 115 | | 1 : 2 | 3.90 | 66.2 | (0.253, 0.628) | 314 |
| Example 116 | | 1 : 3 | 3.93 | 65.3 | (0.256, 0.713) | 316 |
| Example 117 | 107 : 2-77 | 1 : 1 | 3.82 | 67.8 | (0.243, 0.612) | 154 |
| Example 118 | | 1 : 2 | 3.86 | 66.9 | (0.265, 0.669) | 158 |
| Example 119 | | 1 : 3 | 3.92 | 66.0 | (0.255, 0.627) | 163 |
| Example 120 | 107 : 2-56 | 1 : 1 | 3.82 | 67.7 | (0.243, 0.612) | 298 |
| Example 121 | | 1 : 2 | 3.87 | 66.8 | (0.265, 0.669) | 304 |
| Example 122 | | 1 : 3 | 3.92 | 65.8 | (0.255, 0.627) | 309 |
| Example 123 | 108 : 2-79 | 1 : 1 | 3.82 | 67.0 | (0.243, 0.653) | 156 |
| Example 124 | | 1 : 2 | 3.87 | 65.9 | (0.247, 0.644) | 160 |
| Example 125 | | 1 : 3 | 3.93 | 65.1 | (0.274, 0.658) | 164 |
| Example 126 | 108 : 2-57 | 1 : 1 | 3.81 | 66.9 | (0.243, 0.653) | 307 |
| Example 127 | | 1 : 2 | 3.88 | 65.9 | (0.247, 0.644) | 312 |
| Example 128 | | 1 : 3 | 3.93 | 65.0 | (0.274, 0.658) | 318 |
| Example 129 | 119 : 2-78 | 1 : 1 | 3.80 | 67.4 | (0.263, 0.621) | 154 |
| Example 130 | | 1 : 2 | 3.85 | 66.5 | (0.256, 0.670) | 156 |
| Example 131 | | 1 : 3 | 3.90 | 65.5 | (0.245, 0.637) | 159 |
| Example 132 | 119 : 2-50 | 1 : 1 | 3.80 | 67.5 | (0.263, 0.621) | 302 |
| Example 133 | | 1 : 2 | 3.85 | 66.6 | (0.256, 0.670) | 306 |
| Example 134 | | 1 : 3 | 3.90 | 65.6 | (0.245, 0.637) | 310 |
| Example 135 | 160 : 2-74 | 1 : 1 | 3.82 | 67.3 | (0.254, 0.653) | 154 |
| Example 136 | | 1 : 2 | 3.87 | 66.3 | (0.275, 0.657) | 158 |
| Example 137 | | 1 : 3 | 3.93 | 65.3 | (0.264, 0.642) | 163 |
| Example 138 | 160 : 2-51 | 1 : 1 | 3.80 | 67.3 | (0.254, 0.653) | 300 |
| Example 139 | | 1 : 2 | 3.85 | 66.4 | (0.275, 0.657) | 305 |
| Example 140 | | 1 : 3 | 3.92 | 65.4 | (0.264, 0.642) | 311 |
| Example 141 | 263 : 2-78 | 1 : 1 | 3.85 | 68.1 | (0.256, 0.638) | 155 |
| Example 142 | | 1 : 2 | 3.91 | 67.1 | (0.251, 0.632) | 160 |
| Example 143 | | 1 : 3 | 3.95 | 66.2 | (0.253, 0.684) | 165 |
| Example 144 | 263 : 2-50 | 1 : 1 | 3.84 | 68.2 | (0.256, 0.638) | 303 |
| Example 145 | | 1 : 2 | 3.90 | 67.2 | (0.251, 0.632) | 307 |
| Example 146 | | 1 : 3 | 3.94 | 66.3 | (0.253, 0.684) | 312 |
| Example 147 | 37 : 2-76 (Red host) | 1 : 1 | 4.10 | 65.6 | (0.672, 0.319) | 143 |
| Example 148 | | 1 : 2 | 4.15 | 65.2 | (0.670, 0.321) | 146 |
| Example 149 | | 1 : 3 | 4.19 | 64.9 | (0.671, 0.320) | 149 |
| Comparative Example 9 | A : 2-78 | 1 : 1 | 4.49 | 59.8 | (0.256, 0.723) | 84 |
| Comparative Example 10 | | 1 : 2 | 4.54 | 59.3 | (0.243, 0.629) | 87 |
| Comparative Example 11 | | 1 : 3 | 4.59 | 58.8 | (0.268, 0.734) | 89 |
| Comparative Example 12 | C : 2-76 | 1 : 1 | 4.50 | 59.5 | (0.266, 0.657) | 75 |
| Comparative Example 13 | | 1 : 2 | 4.55 | 59.0 | (0.268, 0.739) | 79 |
| Comparative Example 14 | | 1 : 3 | 4.60 | 58.7 | (0.257, 0.624) | 84 |
| Comparative Example 15 | D : 2-79 | 1 : 1 | 4.52 | 59.3 | (0.687, 0.643) | 76 |
| Comparative Example 16 | | 1 : 2 | 4.57 | 58.9 | (0.267, 0.628) | 80 |
| Comparative Example 17 | | 1 : 3 | 4.62 | 58.5 | (0.265, 0.624) | 85 |
| Comparative Example 18 | E: Change in line width was 20% or more, and short-circuit occurred in pattern crossing part 2-77 | 1 : 1 | 4.60 | 58.0 | (0.276, 0.613) | 72 |
| Comparative Example 19 | | 1 : 2 | 4.64 | 57.7 | (0.259, 0.628) | 76 |
| Comparative Example 20 | | 1 : 3 | 4.69 | 57.4 | (0.244, 0.628) | 79 |
| Comparative Example 21 | H : 2-74 | 1 : 1 | 4.65 | 57.3 | (0.256, 0.723) | 70 |
| Comparative Example 22 | | 1 : 2 | 4.70 | 57.0 | (0.243, 0.629) | 74 |
| Comparative Example 23 | | 1 : 3 | 4.75 | 56.5 | (0.268, 0.734) | 78 |

When comparing the results in Table 10 with the results in Table 11, it can be confirmed that when both the heterocyclic compound of Chemical Formula 1 and the compound of Chemical Formula 2 are used as hosts of the light emitting layer, all of the driving voltage, the light emitting efficiency and the service life are improved.

It shows that better efficiency and service life effect are achieved when both the compound of Chemical Formula 1 and the compound of Chemical Formula 2 are included. From this result, it can be expected that an exciplex phenomenon will occur when both two compounds are included.

The exciplex phenomenon is a phenomenon in which energy with a magnitude of the HOMO level of a donor (p-host) and the LUMO level of an acceptor (n-host) is released due to an electron exchange between two molecules. When the exciplex phenomenon between two molecules occurs, a reverse intersystem crossing (RISC) occurs, and the internal quantum efficiency of fluorescence can be increased to 100% due to the RISC. When a donor with a good hole transport capacity (p-host) and an acceptor with a good electron transport capacity (n-host) are used as hosts for the light emitting layer, holes are injected into the p-host and electrons are injected into the n-host, so that the driving voltage can be lowered, which can help to improve the service life. In the present invention, it could be confirmed that the compound of Chemical Formula 2 serving as a donor and the compound of Chemical Formula 1 serving as an acceptor exhibit excellent device characteristics when the compounds are used as hosts of the light emitting device.

In particular, it can be confirmed that the service life characteristics are excellent when the compound is substituted with deuterium. This results from substitution with deuterium as described in the results in Table 10, indicating that even though the compound has a similar structure, the characteristics of the compound may vary depending on the substitution with deuterium.

In contrast, it can be seen that when the compounds (Comparative Examples 9 to 23) out of the scope of the present invention are used in combination with the compound of Chemical Formula 2, the driving voltage, light emitting efficiency and service life deteriorate compared to the present invention.

That is, it can be confirmed that when both the heterocyclic compound of Chemical Formula 1 and the compound of Chemical Formula 2 of the present invention are used as hosts of a light emitting layer, the driving voltage, light emitting efficiency and service life are remarkably excellent.

### [Explanation of Reference Numerals and Symbols]

- 100:: Substrate
- 200:: Positive electrode
- 300:: Organic material layer
- 301:: Hole injection layer
- 302:: Hole transport layer
- 303:: Light emitting layer
- 304:: Hole blocking layer
- 305:: Electron transport layer
- 306:: Electron injection layer
- 400:: Negative electrode

## Claims

1. A heterocyclic compound represented by the following Chemical Formula 1: 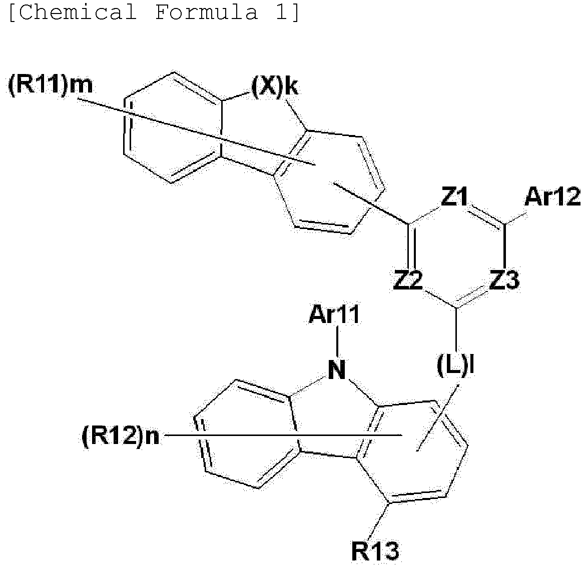
wherein, in Chemical Formula 1,
Z1 to Z3 are the same as or different from each other, and are each independently N; or CR, and at least one is N,
R is hydrogen; deuterium; a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group,
L is a direct bond; or a substituted or unsubstituted C6 to C60 arylene group,
1 is an integer from 0 to 3, and when 1 is 2 or higher, L's are the same as or different from each other,
X is 0; or S,
R11 to R13 are the same as or different from each other, and are each independently hydrogen; deuterium; a halogen group; a cyano group; a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group,
k is 0 or 1,
when k is 0, it means that there is no bond rather than a direct bond,
when k is 0, m is an integer from 0 to 9, and when k is 1, m is an integer from 0 to 7, and when m is 2 or higher, R11's are the same as or different from each other,
n is an integer from 0 to 6, and when n is 2 or higher, R12's are the same as or different from each other,
Ar11 and Ar12 are the same as or different from each other, and are each independently a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group,
a deuterium content of 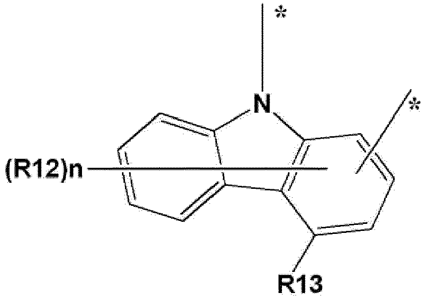 in the structure of Chemical Formula 1 is more than 0% and 100% or less, and
the 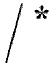 is a position bonded to Chemical Formula 1.

2. The heterocyclic compound of claim 1, wherein a deuterium content of the heterocyclic compound represented by Chemical Formula 1 is more than 0% and 100% or less.

3. The heterocyclic compound of claim 1, wherein Chemical Formula 1 is dividedly represented by the following Structures 1-A to 1-D, and a deuterium content of the following Structure 1-C is 30% to 100%: 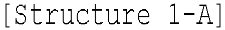 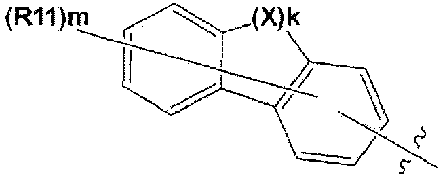 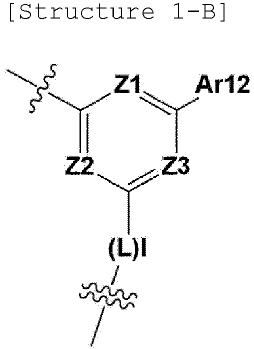 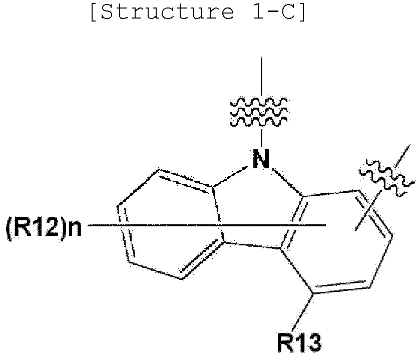 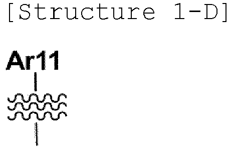 in Structures 1-A to 1-D,
the definitions of Z1 to Z3, L, l, X, R11 to R13, k, m, n, Ar11 and Ar12 are the same as the definitions in Chemical Formula 1, and 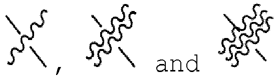 are each a binding position where those which are the same are linked to each other.

4. The heterocyclic compound of claim 1, wherein Chemical Formula 1 is represented by any one of the following Chemical Formulae 1-1-1 to 1-1-3: 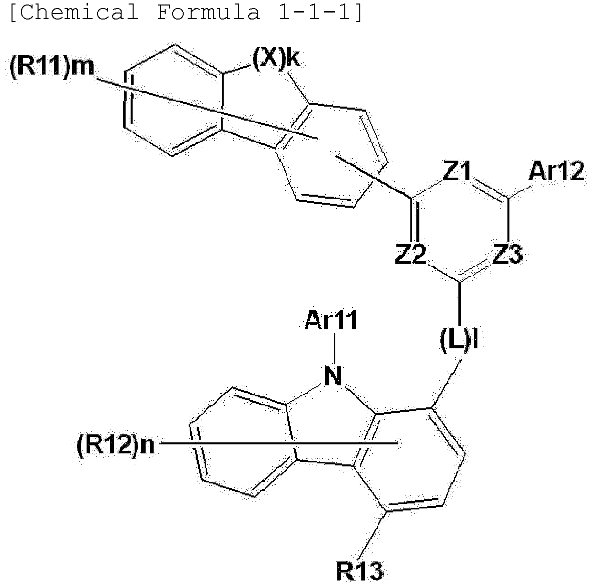 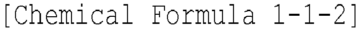 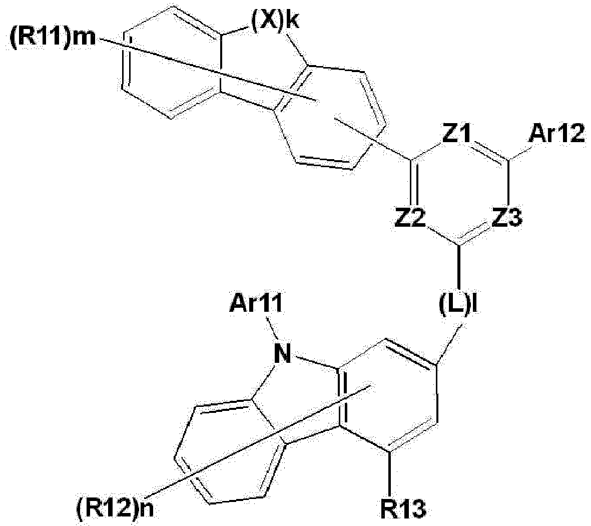 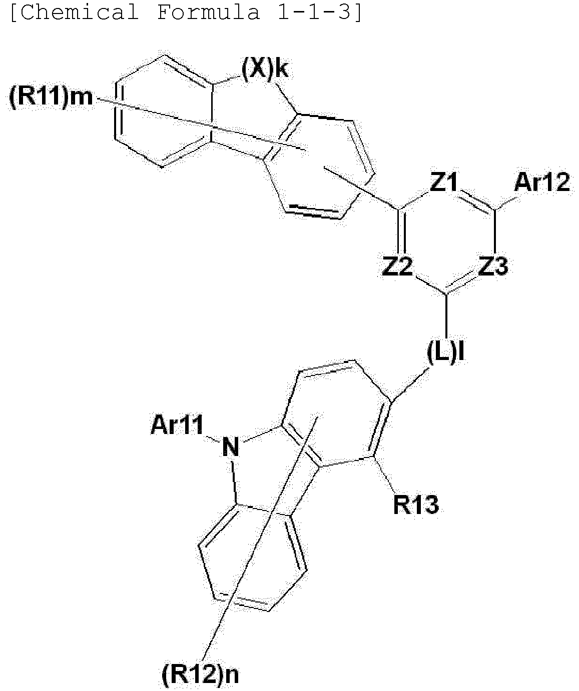 in Chemical Formulae 1-1-1 to 1-1-3,
the definitions of Z1 to Z3, L, l, X, R11 to R13, k, m, n, Ar11 and Ar12 are the same as the definitions in Chemical Formula 1.

5. The heterocyclic compound of claim 1, wherein Chemical Formula 1 is represented by any one of the following Chemical Formulae 1-1 to 1-3: 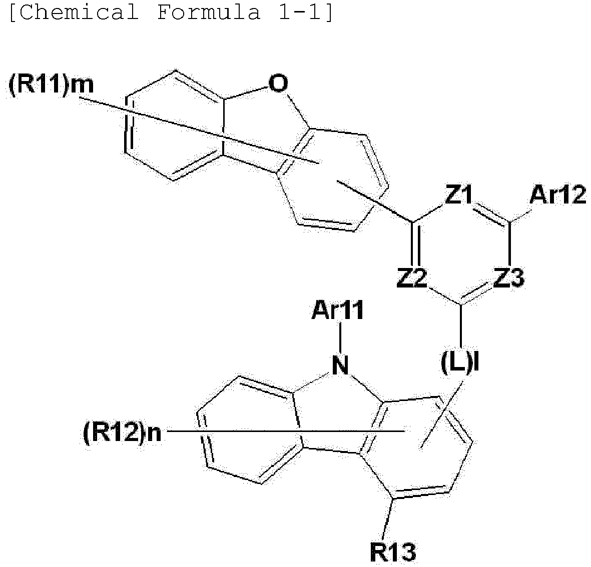 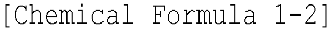 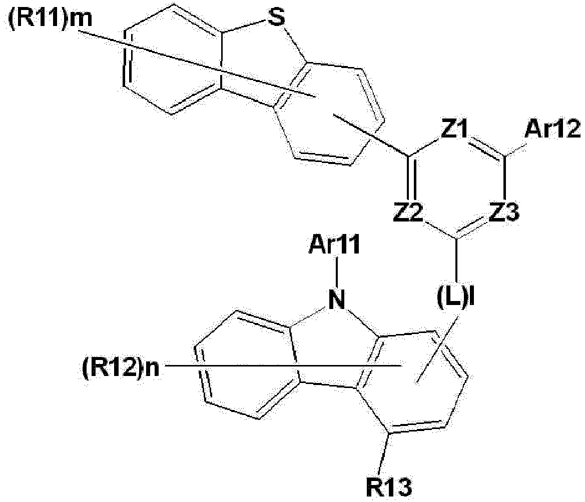 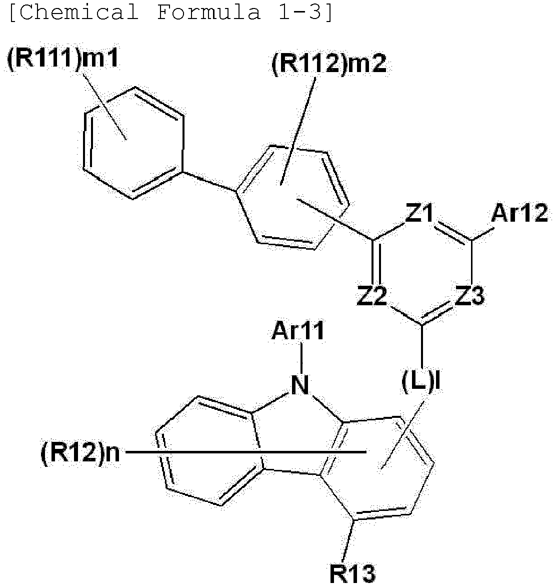 in Chemical Formulae 1-1 to 1-3,
the definitions of Z1 to Z3, L, l, R11 to R13, m, n, Ar11 and Ar12 are the same as the definitions in Chemical Formula 1,
R111 and R112 are the same as or different from each other, and are each independently hydrogen; deuterium; a halogen group; a cyano group; a substituted or unsubstituted C1 to C60 alkyl group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group, and
m1 is an integer from 0 to 5, m2 is an integer from 0 to 4, and when m1 and m2 are each 2 or higher, substituents in the parenthesis are the same as or different from each other.

6. The heterocyclic compound of claim 1, wherein Ar11 and Ar12 are the same as or different from each other, and are each independently a substituted or unsubstituted C6 to C20 aryl group; or a substituted or unsubstituted C2 to C20 heteroaryl group.

7. The heterocyclic compound of claim 1, wherein Chemical Formula 1 is represented by any one of the following compounds:

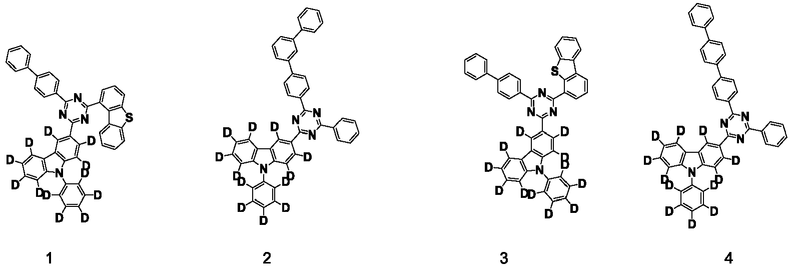

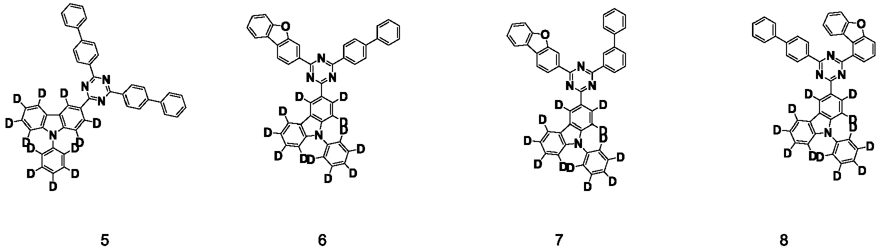

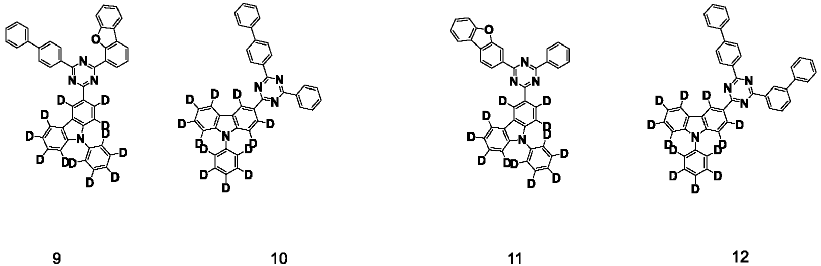

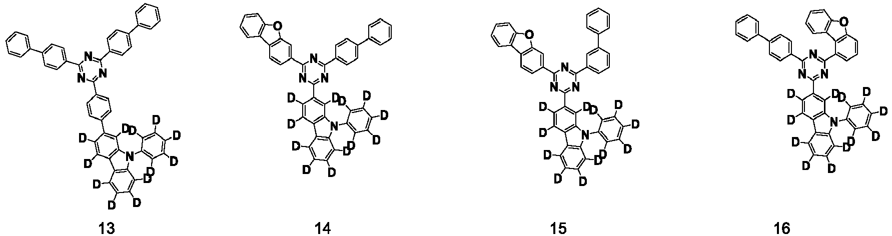

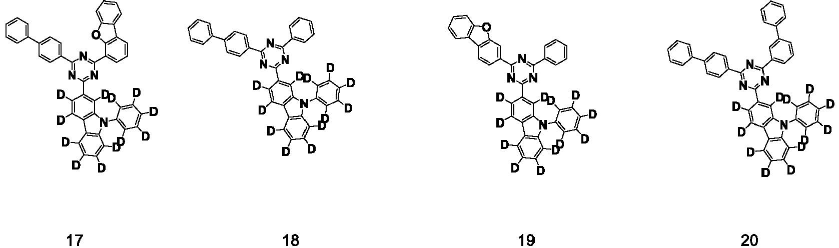

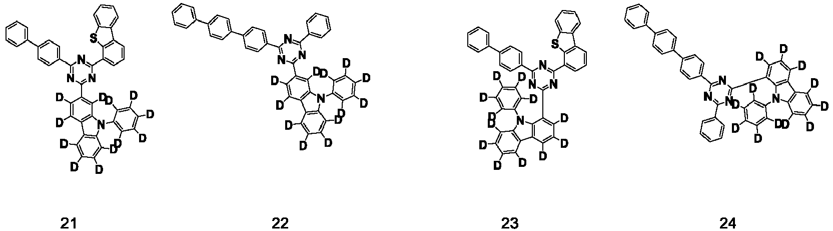

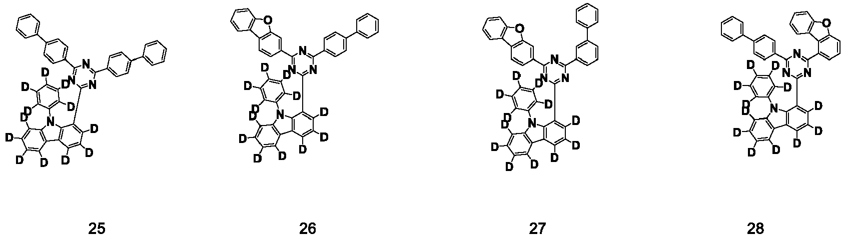

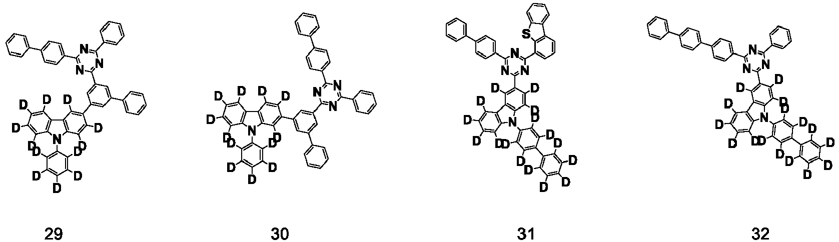

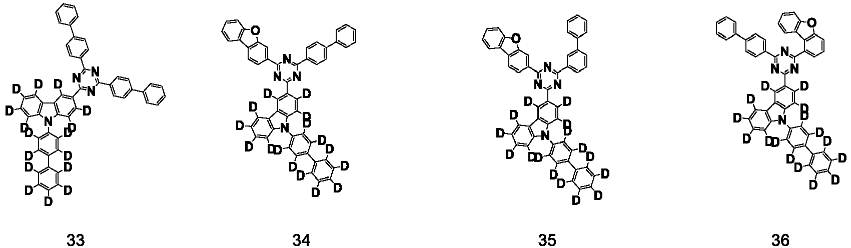

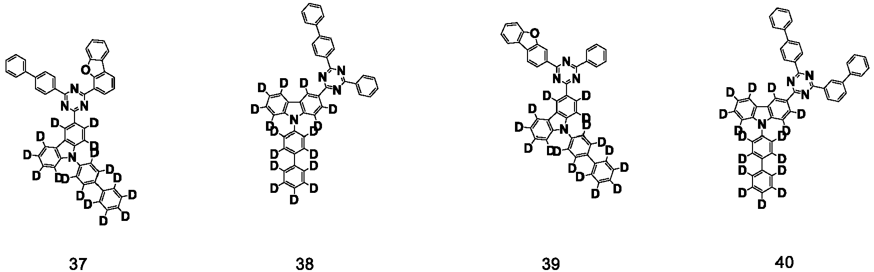

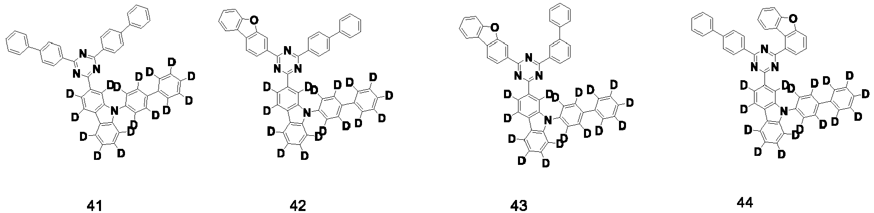

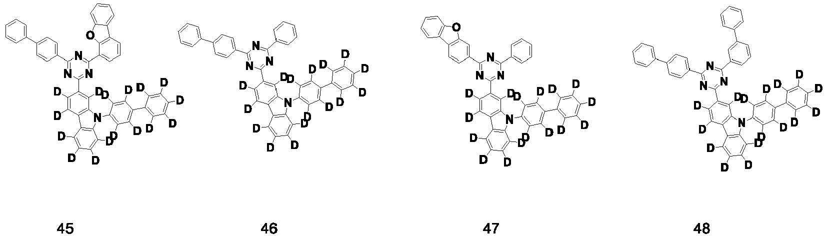

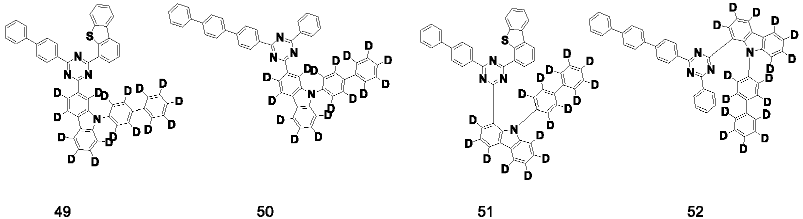

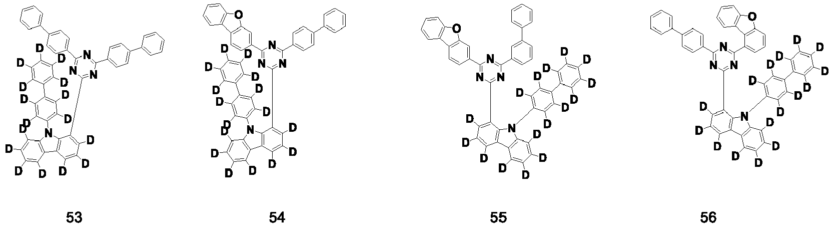

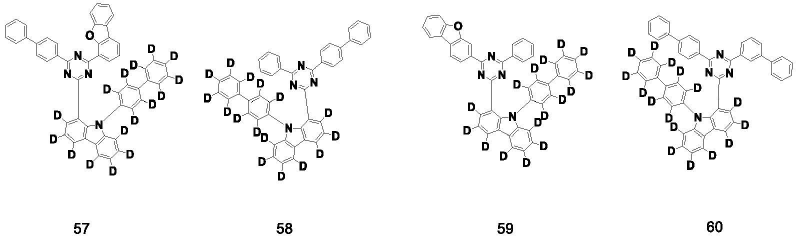

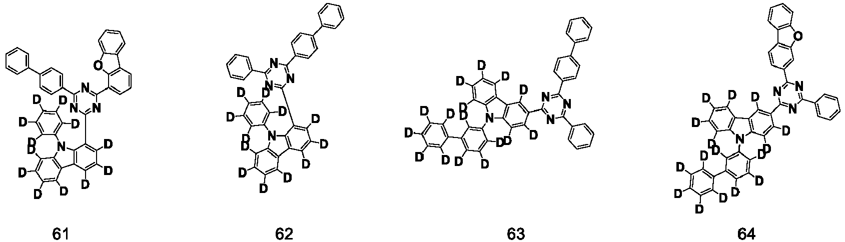

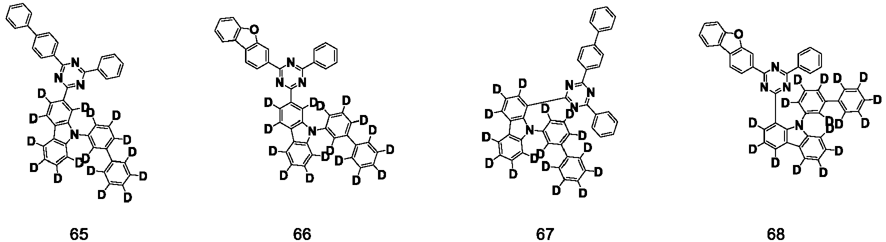

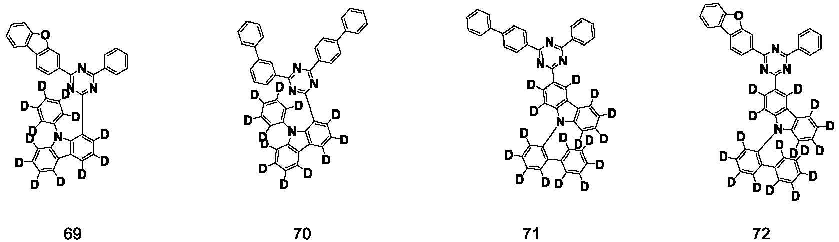

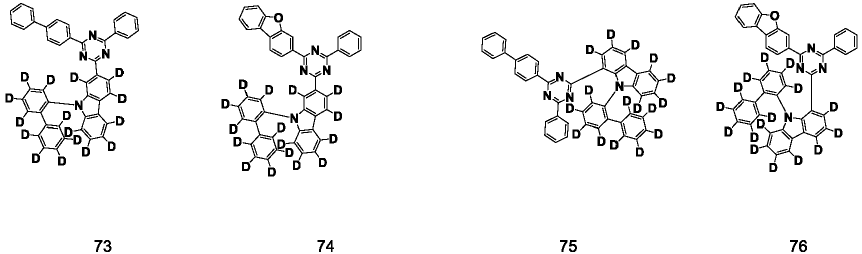

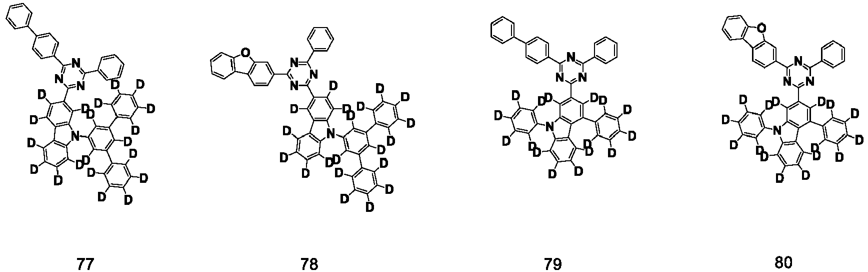

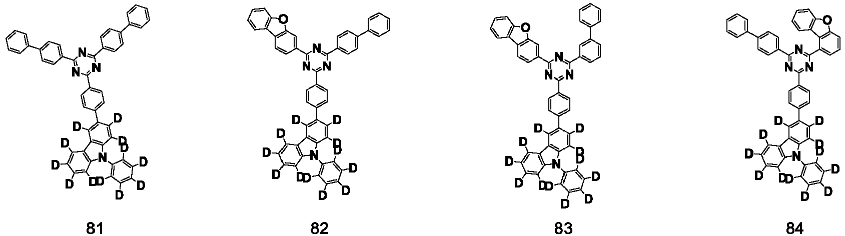

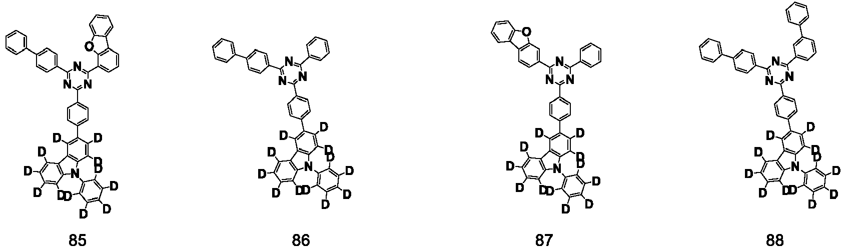

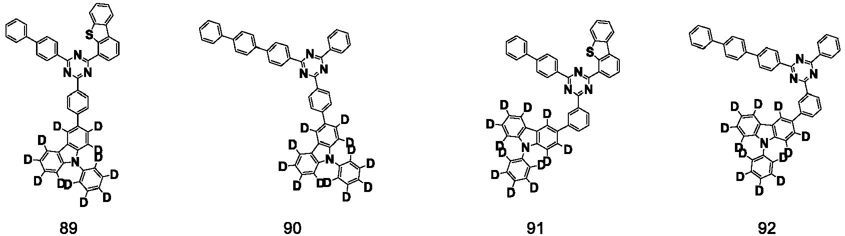

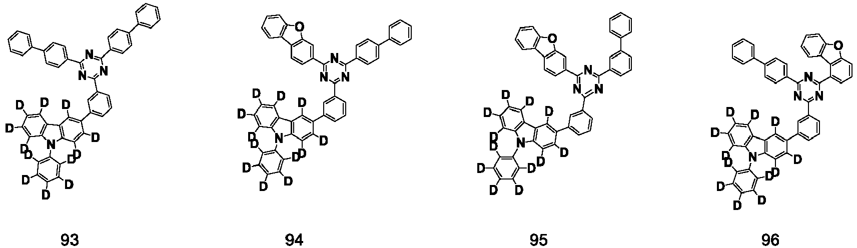

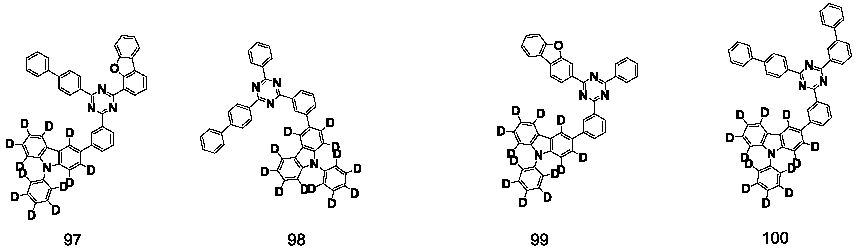

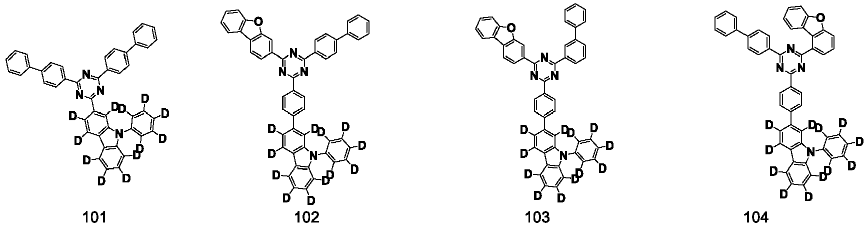

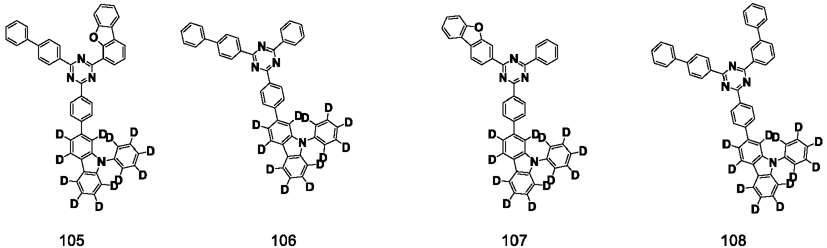

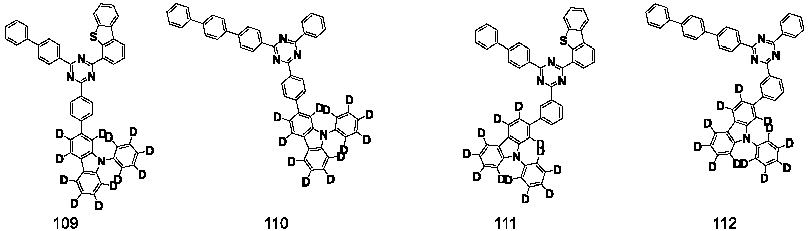

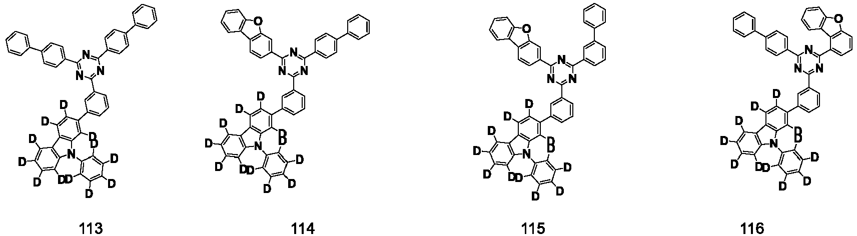

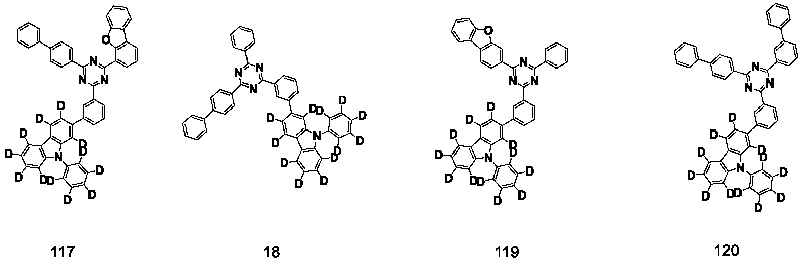

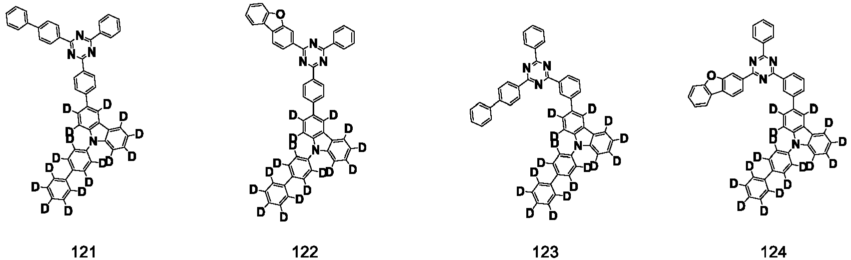

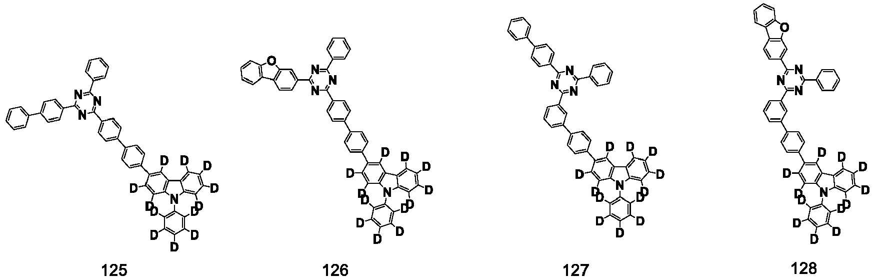

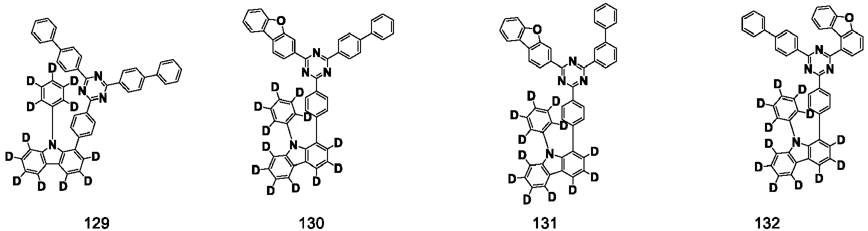

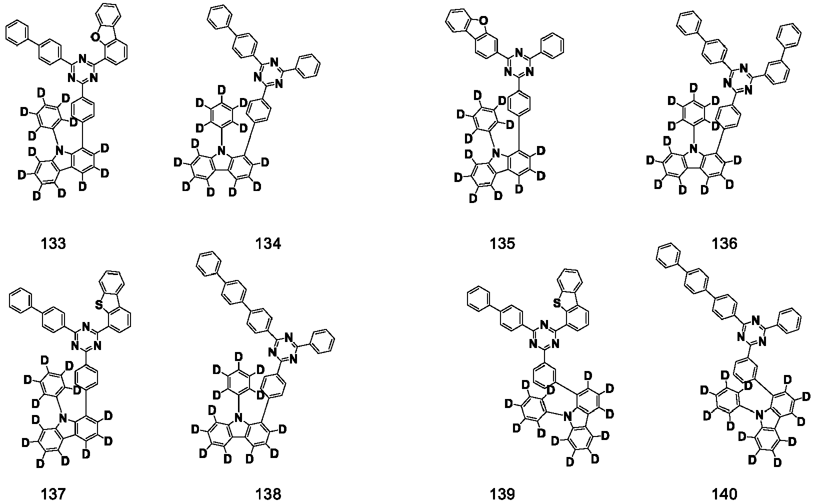

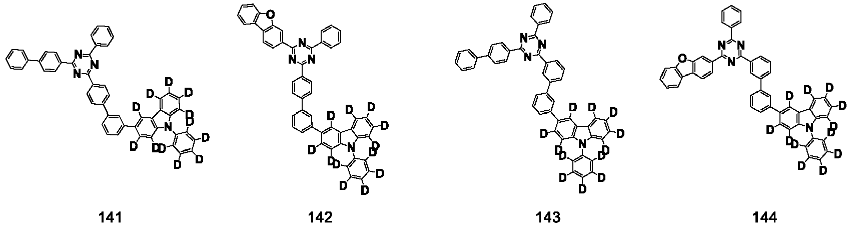

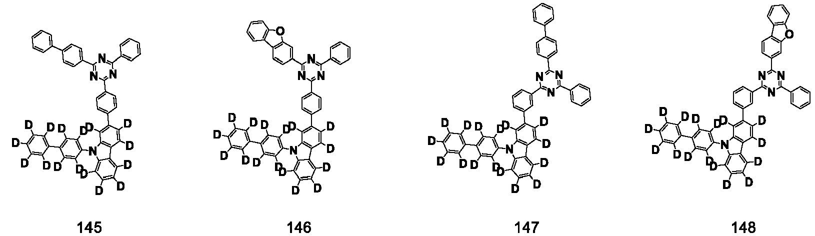

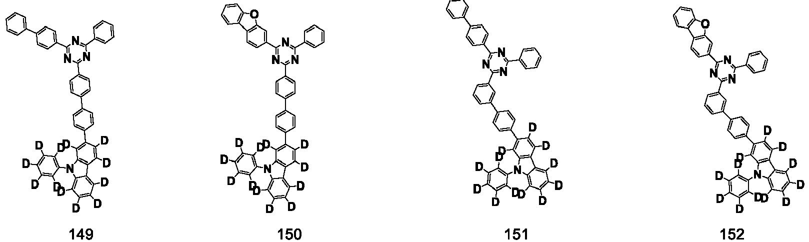

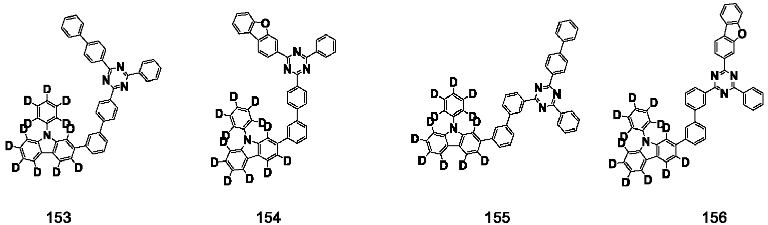

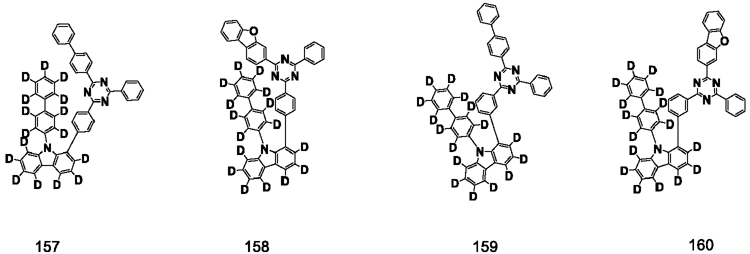

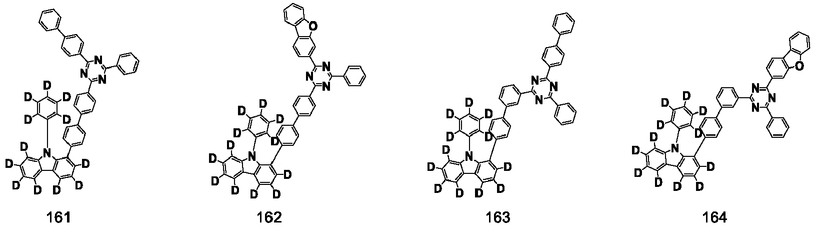

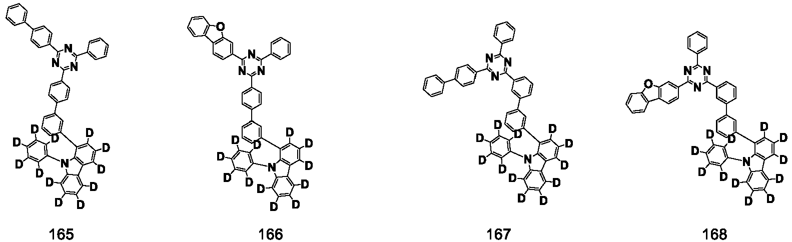

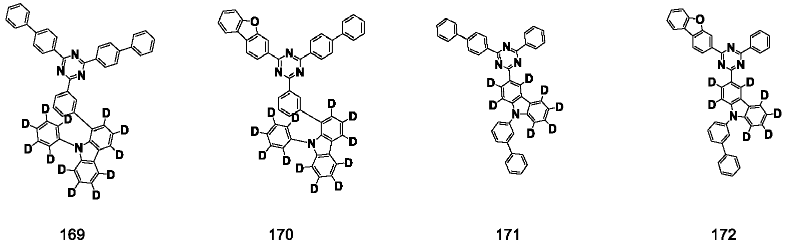

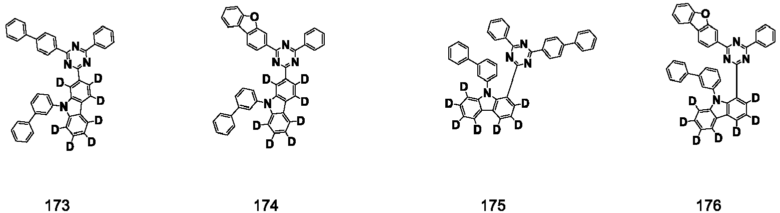

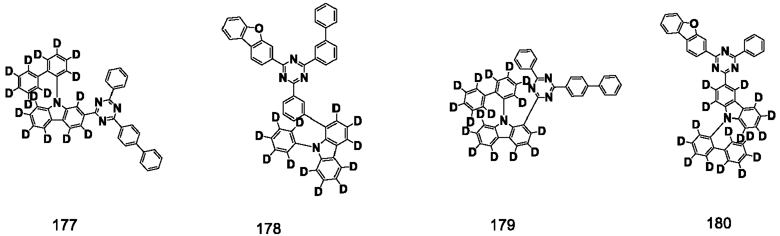

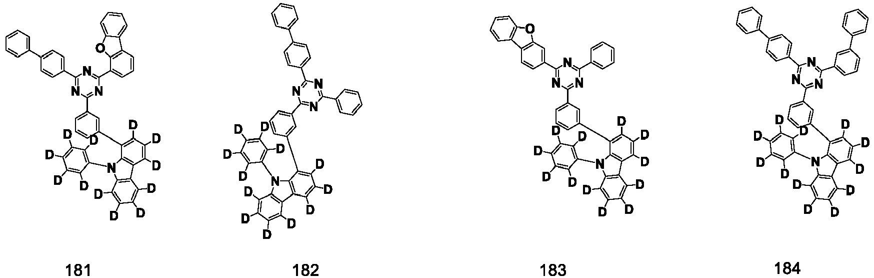

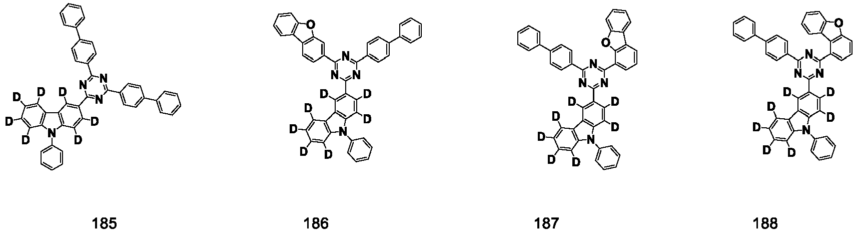

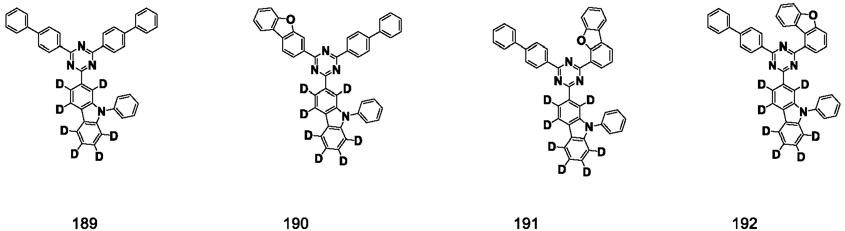

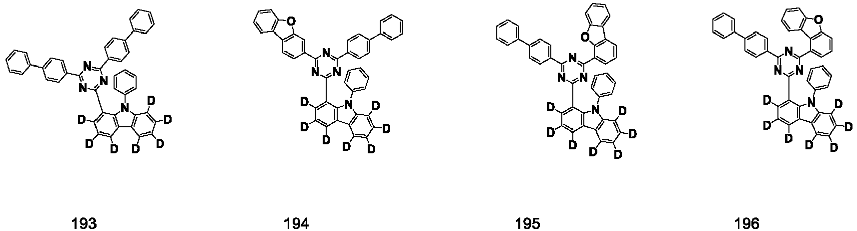

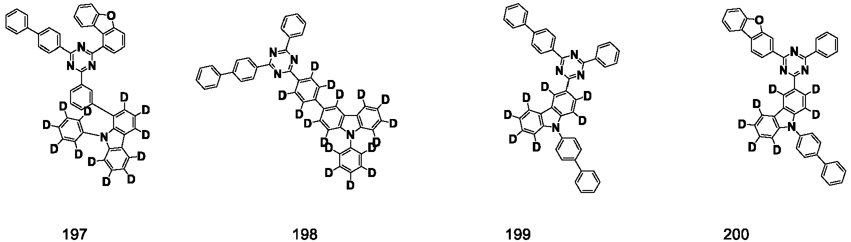

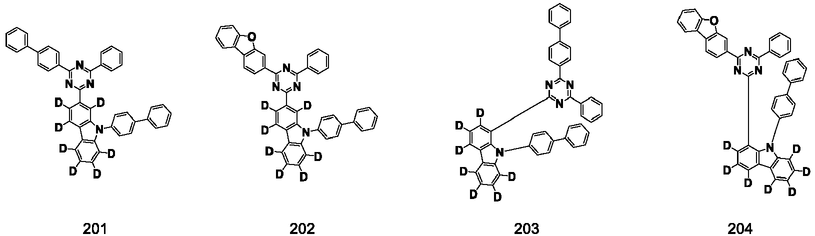

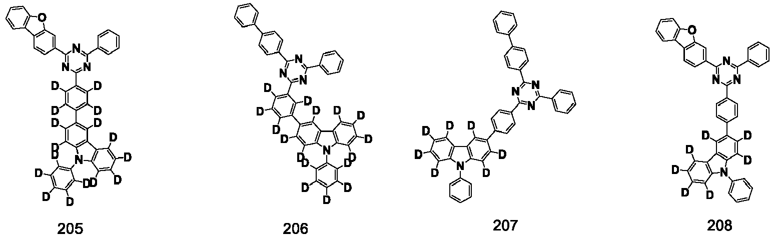

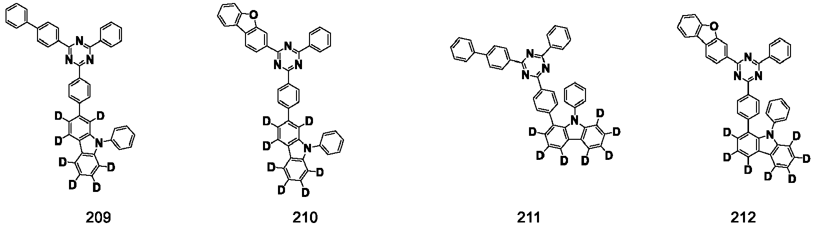

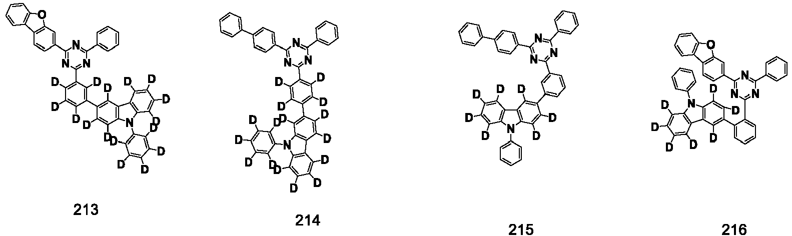

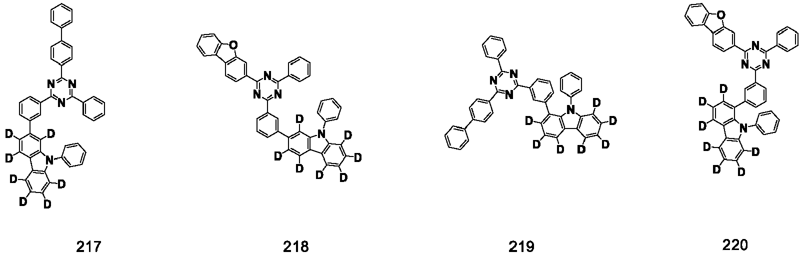

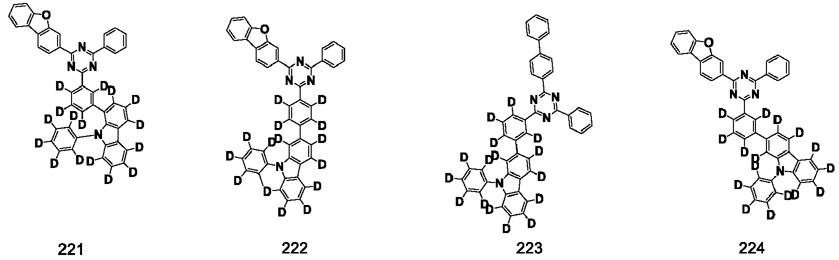

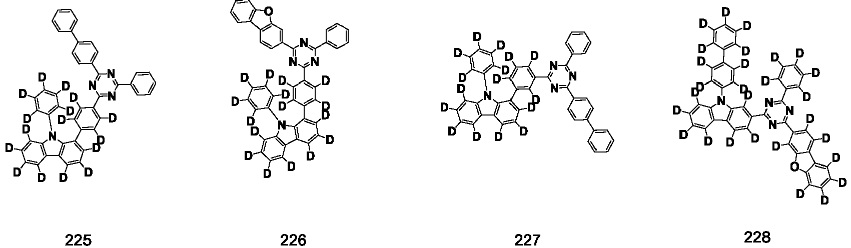

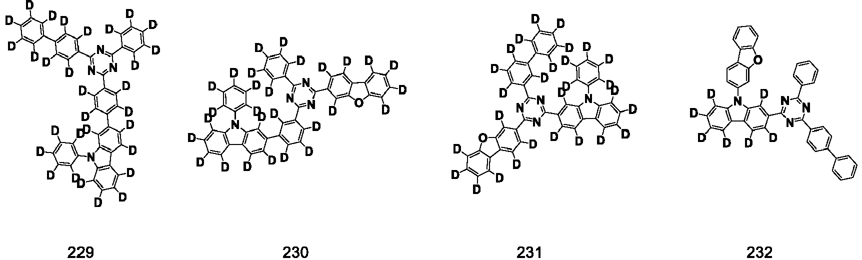

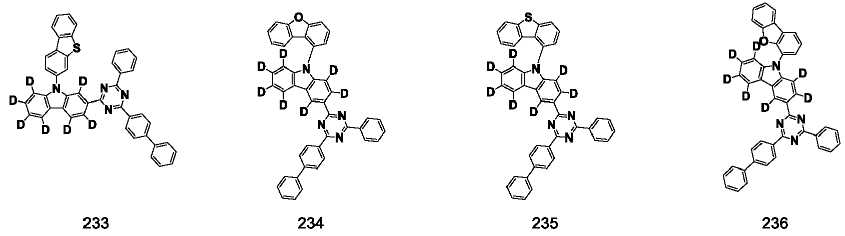

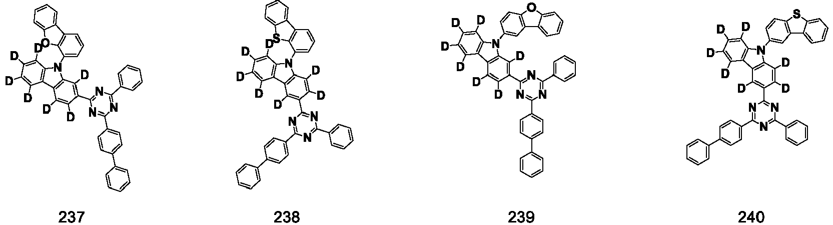

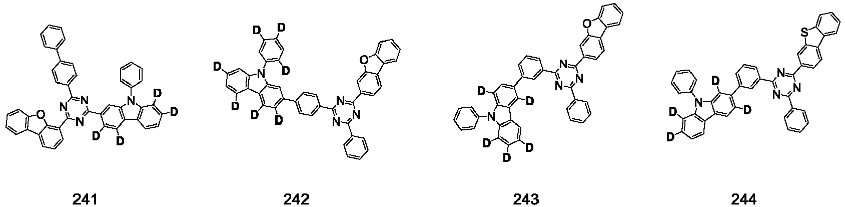

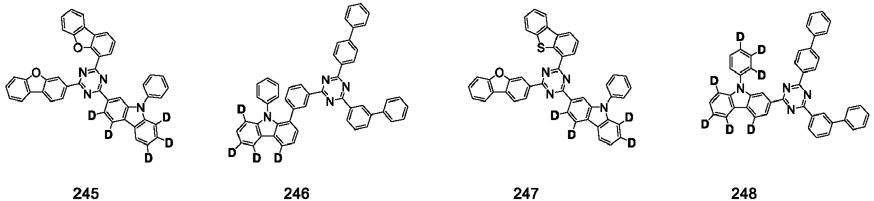

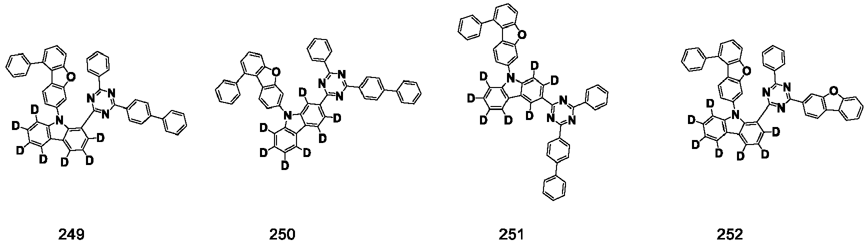

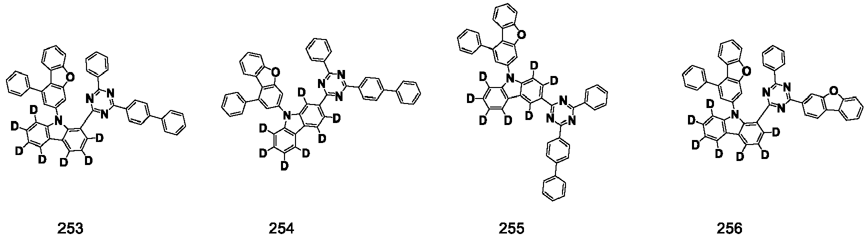

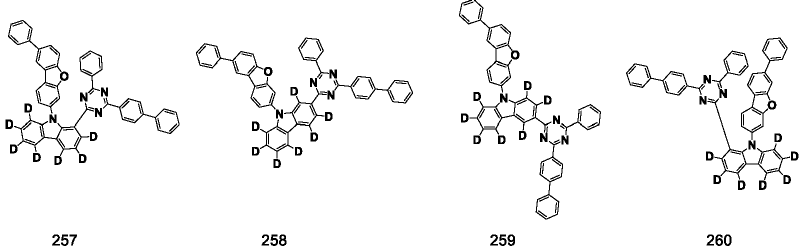

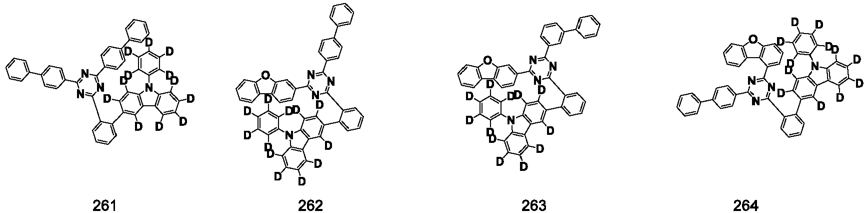

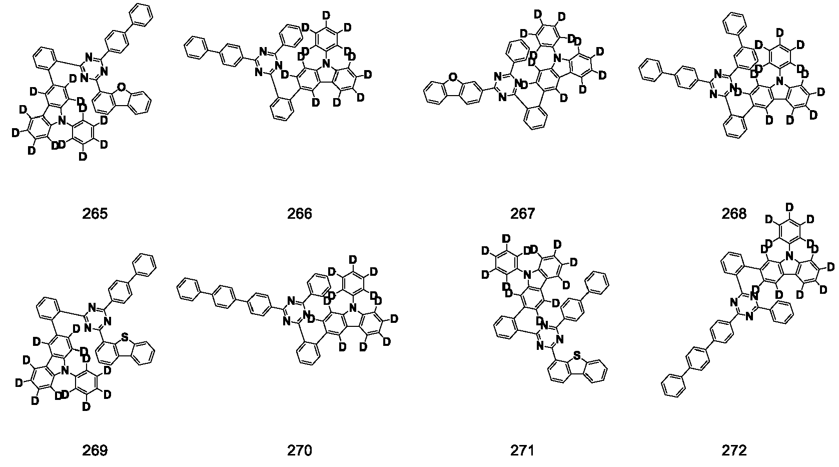

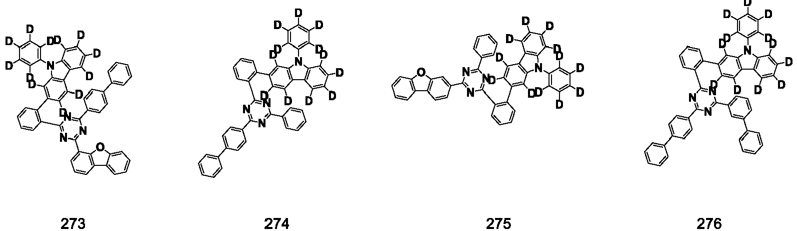

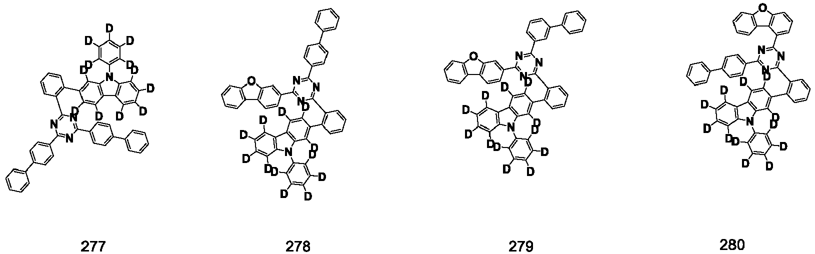

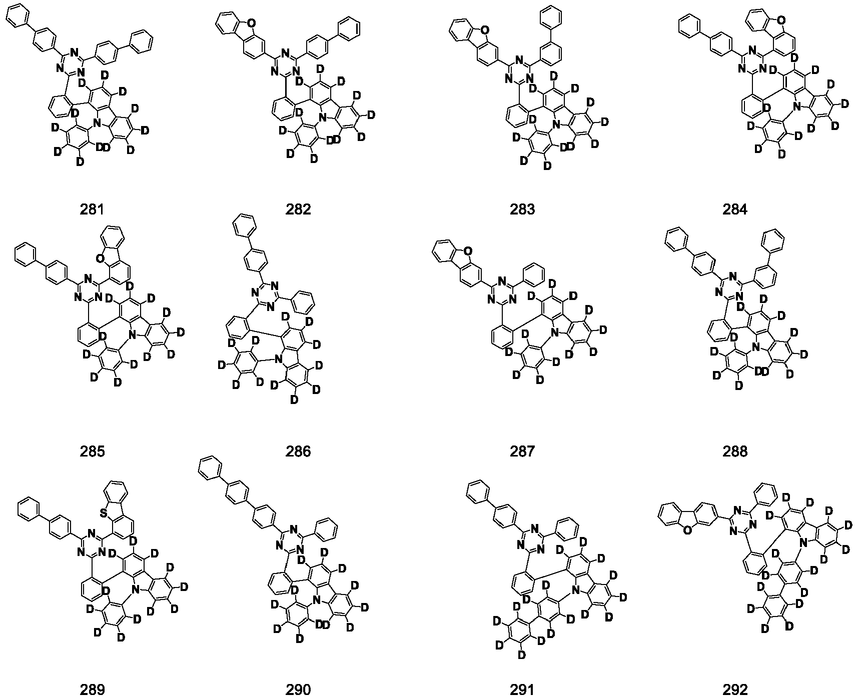

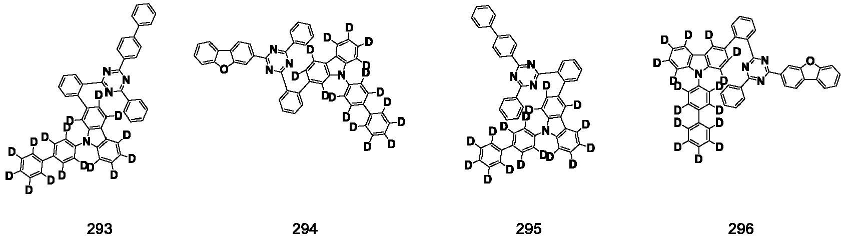

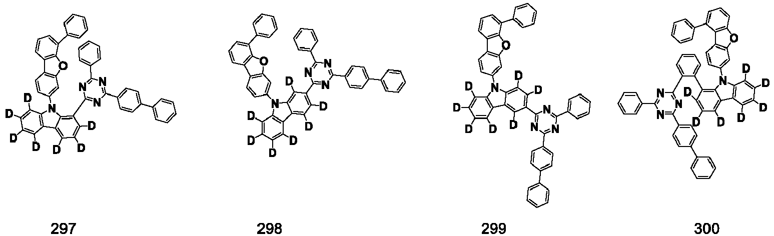

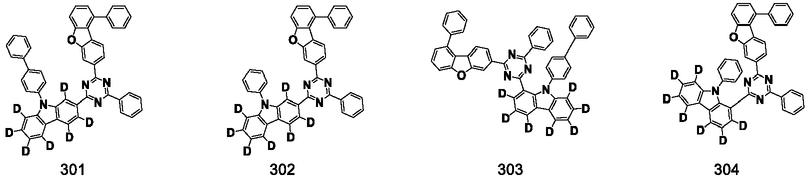

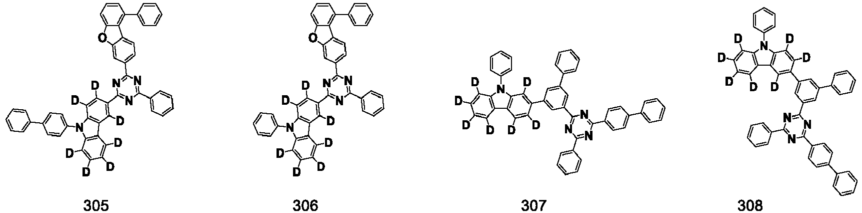

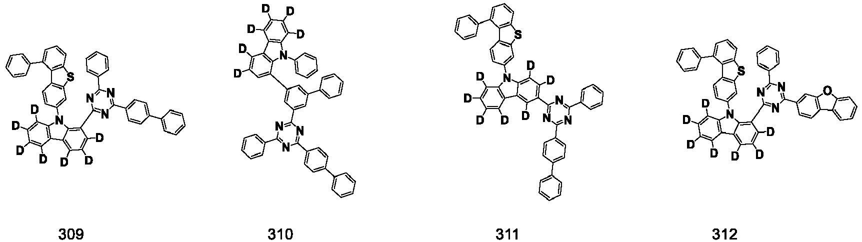

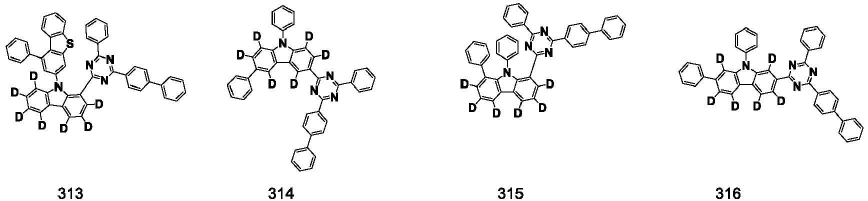

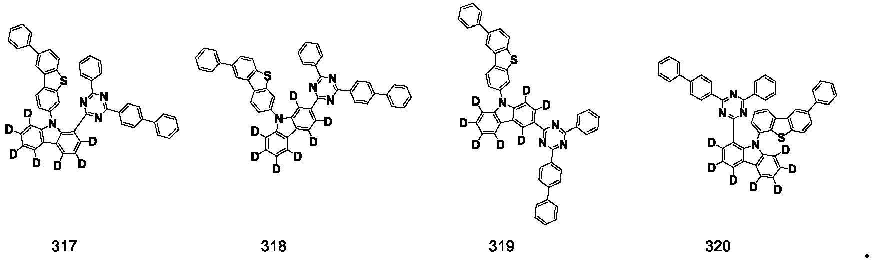

8. An organic light emitting device comprising:
a first electrode;
a second electrode provided to face the first electrode; and
an organic material layer having one or more layers provided between the first electrode and the second electrode,
wherein one or more layers of the organic material layer comprise the heterocyclic compound of any one of claims 1 to 7.

9. The organic light emitting device of claim 8, wherein the organic material layer further comprises a heterocyclic compound represented by the following Chemical Formula 2: 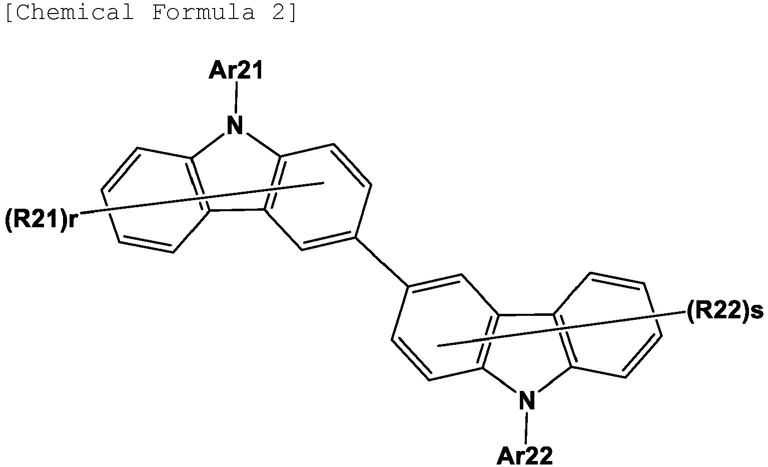 in Chemical Formula 2,
R21 and R22 are the same as or different from each other, and are each independently selected from the group consisting of hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiR'R"R"'; -P(=O)R'R"; and an amine group which is unsubstituted or substituted with a substituted or unsubstituted C1 to C60 alkyl group, a substituted or unsubstituted C6 to C60 aryl group or a substituted or unsubstituted C2 to C60 heteroaryl group, or two or more adjacent groups are bonded to each other to form a substituted or unsubstituted C6 to C60 aliphatic or aromatic hydrocarbon ring or a substituted or unsubstituted C2 to C60 aliphatic or aromatic hetero ring,
R', R" and R‴ are the same as or different from each other, and are each independently hydrogen; deuterium; a cyano group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
r and s are an integer from 0 to 7, and when r and s are 2 or higher, substituents in the parenthesis are the same as or different from each other, and
Ar21 and Ar22 are the same as or different from each other, and are each independently a substituted or unsubstituted C6 to C60 aryl group; or a substituted or unsubstituted C2 to C60 heteroaryl group.

10. The organic light emitting device of claim 9, wherein R21 and R22 are the same as or different from each other, and are each independently hydrogen; or deuterium, and
Ar21 and Ar22 are the same as or different from each other, and are each independently a substituted or unsubstituted C6 to C20 aryl group; or a substituted or unsubstituted C2 to C20 heteroaryl group.

11. The organic light emitting device of claim 9, wherein Chemical Formula 2 is represented by any one of the following compounds:

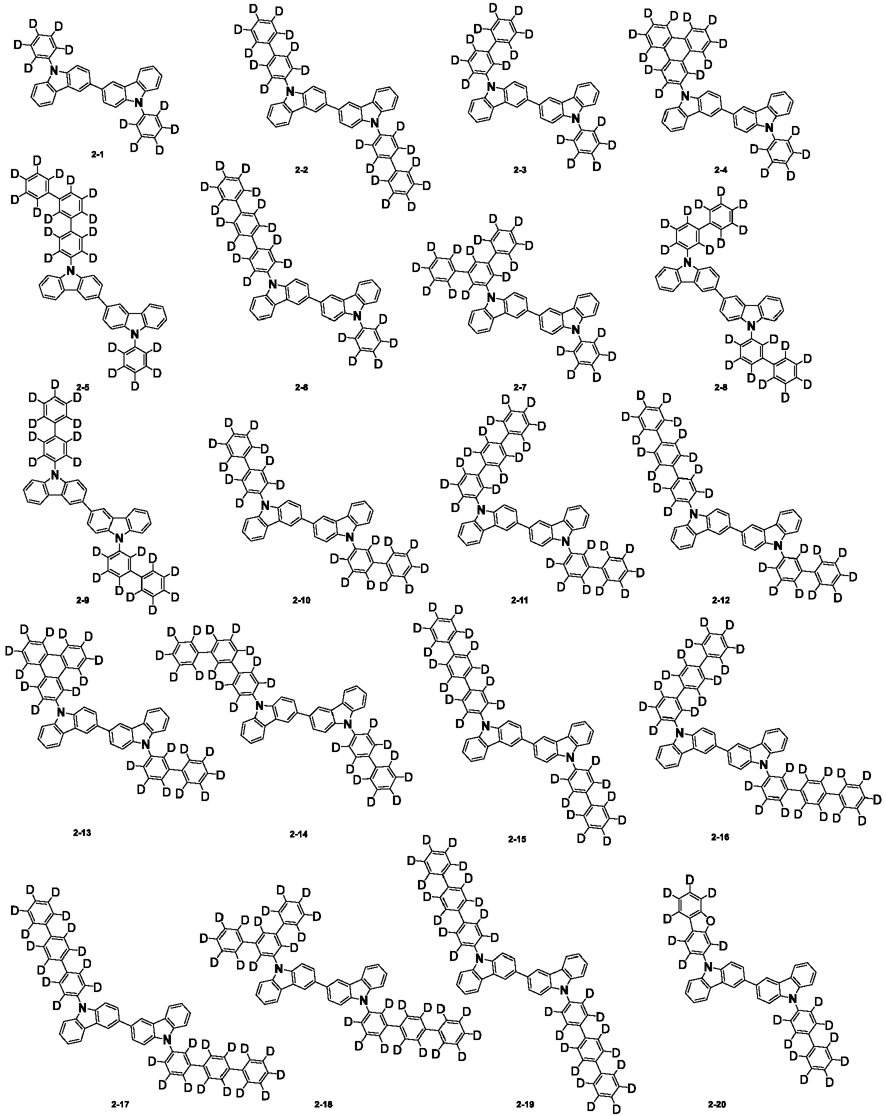

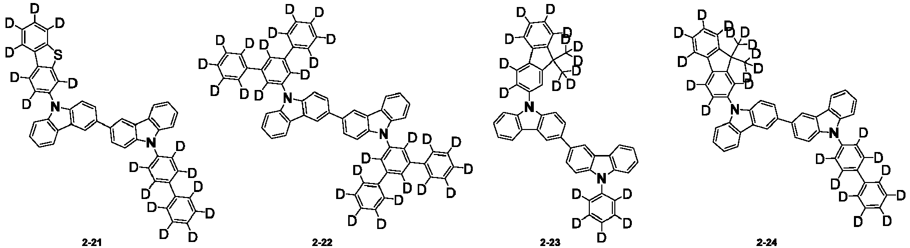

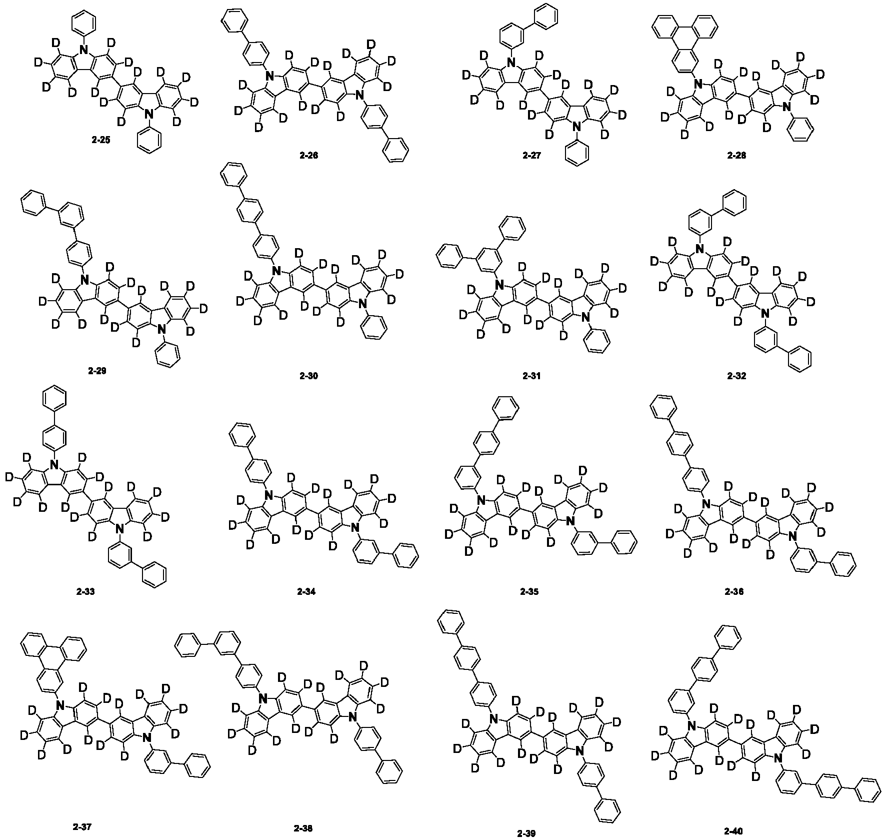

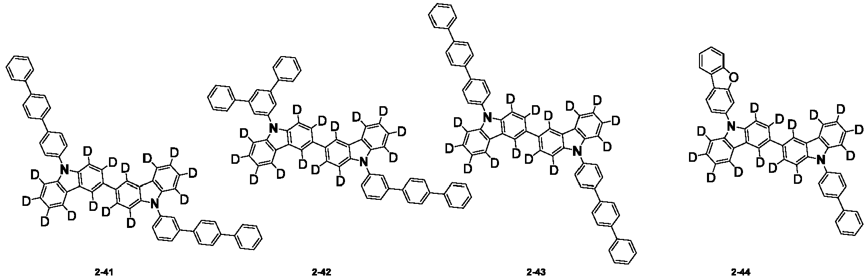

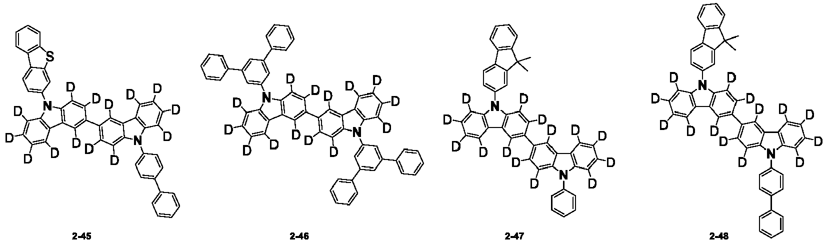

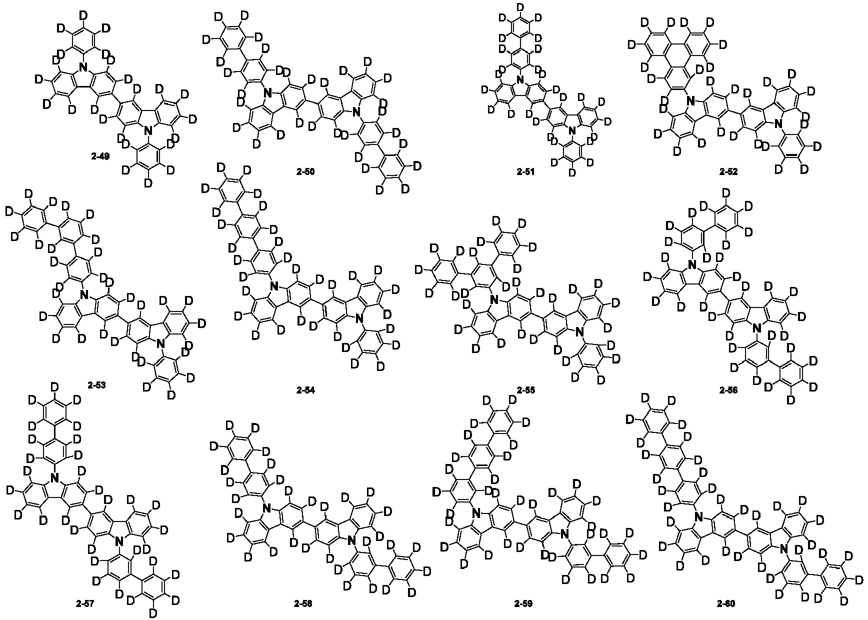

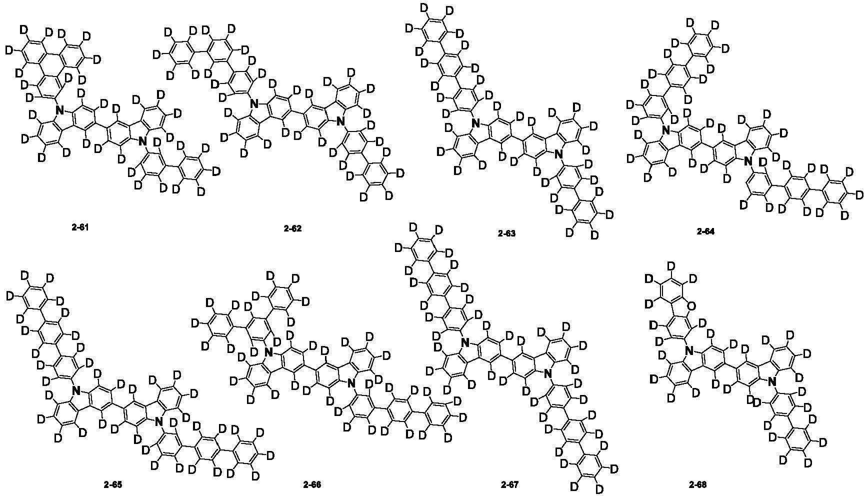

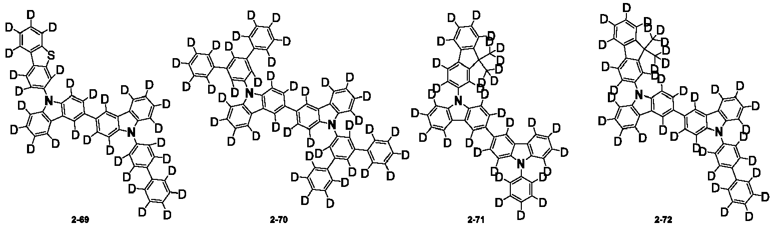

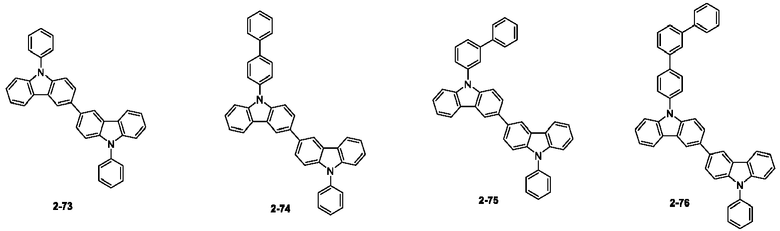

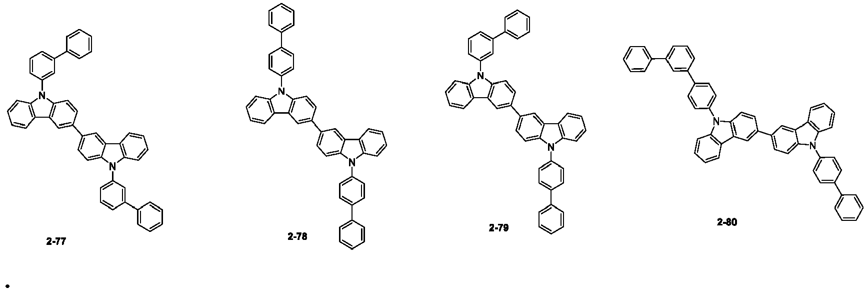

12. The organic light emitting device of claim 9, wherein the organic material layer comprises a light emitting layer, and the light emitting layer comprises the heterocyclic compound represented by Chemical Formula 1 and the heterocyclic compound represented by Chemical Formula 2.

13. The organic light emitting device of claim 9, wherein the organic material layer comprises a light emitting layer, and the light emitting layer comprises a host material, and the host material comprises the heterocyclic compound represented by Chemical Formula 1 and the heterocyclic compound represented by Chemical Formula 2.

14. The organic light emitting device of claim 8, further comprising one or two or more layers selected from the group consisting of a light emitting layer, a hole injection layer, a hole transport layer, an electron blocking layer, a hole blocking layer, an electron transport layer and an electron injection layer.

15. A composition for an organic material layer of an organic light emitting device, the composition comprising the heterocyclic compound according to any one of claims 1 to 7; and a heterocyclic compound represented by the following Chemical Formula 2: 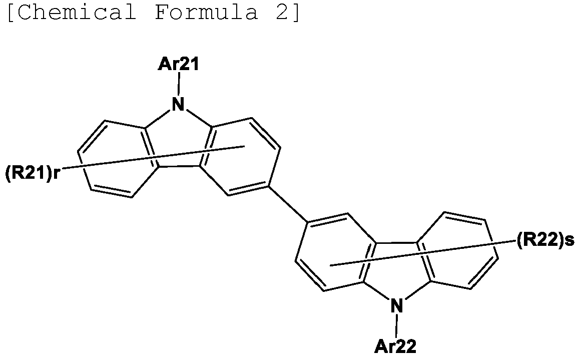 wherein, in Chemical Formula 2,
R21 and R22 are the same as or different from each other, and are each independently selected from the group consisting of hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiR'R"R"'; -P(=O)R'R"; and an amine group which is unsubstituted or substituted with a substituted or unsubstituted C1 to C60 alkyl group, a substituted or unsubstituted C6 to C60 aryl group or a substituted or unsubstituted C2 to C60 heteroaryl group, or two or more adjacent groups are bonded to each other to form a substituted or unsubstituted C6 to C60 aliphatic or aromatic hydrocarbon ring or a substituted or unsubstituted C2 to C60 aliphatic or aromatic hetero ring,
R', R" and R"' are the same as or different from each other, and are each independently hydrogen; deuterium; a cyano group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
r and s are an integer from 0 to 7, and when r and s are 2 or higher, substituents in the parenthesis are the same as or different from each other, and
Ar21 and Ar22 are the same as or different from each other, and are each independently a substituted or unsubstituted C6 to C60 aryl group; or a substituted or unsubstituted C2 to C60 heteroaryl group.

16. The composition of claim 15, wherein a weight ratio of the heterocyclic compound : the heterocyclic compound represented by Chemical Formula 2 in the composition is 1 : 10 to 10 : 1.
